# EUROPEAN PATENT SPECIFICATION

(11) **EP 1 156 060 B1**
(45) Date of publication and mention of the grant of the patent: **27.06.2007**
(21) Application number: 00110065.0
(22) Date of filing: 12.05.2000
(51) Int. Cl.: C07K 16/00, C07K 16/30, C07K 16/28, C12N 15/13, C12N 15/70, C12N 15/85, C12N 1/21, C12N 5/10, A61K 39/395, G01N 33/577, G01N 33/574, G01N 33/68, A61P 35/00, A61P 37/00

(54) **Human peptides/proteins causing or leading to the killing of cells including lymphoid tumor cells**
Humane Peptide/Proteine, die das Töten von Zellen, einschliesslich lymphoide Tumorzellen, herbeiführen oder bewirken
Peptides/protéines humaines causant ou menant à la mort de cellules y compris de cellules lymphoides tumorales

(43) Date of publication of application: 21.11.2001
(73) Proprietor: GPC Biotech AG, 82152 Martinsried (DE); MorphoSys AG, 82152 Martinsried/Planegg (DE)
(72) Inventor: Nagy, Zoltan, 82515 - Wolftshausen (DE); Brunner, Christoph, 83670 Bad Heilbrunn (DE); Tesar, Michael, 82326 Weilheim (DE); Thomassen-Wolf, Elizabeth, 81369 Munich (DE)
(74) Representative: Wachenfeld, Joachim

(56) References cited:
- WO-A-99/45031
- M. DYER: "The role of CAMPATH-1 antibodies in the treatment of lymphoid malignancies." SEMINARS IN ONCOLOGY, vol. 26, no. 5 suppl. 14, October 1999 (1999-10), pages 52-57, XP000939139 New York, NY, USA
- A. WÖLPL ET AL.: "Human monoclonal antibody with T-cell-like specificity recognizes MHC class I self-peptide presented by HLA-DR1 on activated cells." TISSUE ANTIGENS, vol. 51, no. 3, March 1998 (1998-03), pages 258-269, XP000939106 Copenhagen, Denmark
- N. IKEWAKI ET AL.: "Development and characterization of a human monoclonal antibody probably detecting the leukocyte differentiation antigen CD39." TISSUE ANTIGENS, vol. 39, no. 4, April 1992 (1992-04), pages 174-181, XP000939129 Copenhagen, Denmark
- M. BUNCE ET AL.: "The production of a human monoclonal antibody defining a split of HLA-DRw13 (DRw13b)." TISSUE ANTIGENS, vol. 36, no. 3, September 1990 (1990-09), pages 100-102, XP000939132 Copenhagen, Denmark
- H. VIKEN ET AL.: "Serologic subtyping of HLA-DR8 bymeans of the cytotoxic human monoclonal antibody 5643." HUMAN IMMUNOLOGY, vol. 43, no. 3, July 1995 (1995-07), pages 200-206, XP000939101 New York, NY, USA
- M. RHEINNECKER ET AL.: "Multivalent antibody fragments with high functional affinity for a tumor-associated carbohydrate antigen." THE JOURNAL OF IMMUNOLOGY, vol. 157, no. 7, 1 October 1996 (1996-10-01), pages 2989-2997, XP002147465 Baltimore, MD, USA
- A. HOESS ET AL.: "Generation of human antibodies that selectively recognize diseased cells overexpressing surface bound antigens." PROCEEDINGS OF THE AMERICAN ASSOCIATION FOR CANCER RESEARCH, vol. 38, March 1997 (1997-03), page 30 XP002147466 USA
- A. KNAPPIK ET AL.: "Fully synthetic human combinatorial antibody libraries (HuCAL) based on modular consensus frameworks and CDRs randomized with trinucleotides." JOURNAL OF MOLECULAR BIOLOGY, vol. 296, no. 1, 11 February 2000 (2000-02-11), pages 57-86, XP000939143 London, GB

## Description

The present invention relates to human peptides/proteins as specified in the appended claims causing or leading to the killing of cells including lymphoid tumor cells. The invention further relates to nucleic acids encoding the peptides/proteins, methods for the production of the peptides/proteins, pharmaceutical, diagnostic and multivalent compositions and kits comprising the peptides/proteins and uses of the peptides/proteins. Furthermore, methods for killing cells are described.

Every mammalian species, which has been studied to date, carries a cluster of genes coding for the so-called major histocompatibility complex (MHC). This tightly linked cluster of genes code for surface antigens, which play a central role in the development of both humoral and cell-mediated immune responses. In humans the products coded for by the MHC are referred to as Human Leukocyte Antigens or HLA. The MHC-genes are organized into regions encoding three classes of molecules, class I to III.

Class I MHC molecules are 45 kD transmembrane glycoproteins, noncovalently associated with another glycoprotein, the 12 kD beta-2 microglobulin (Brown et al., 1993). The latter is not inserted into the cell membrane, and is encoded outside the MHC. Human class I molecules are of three different isotypes, termed HLA-A, -B, and -C, encoded in separate loci. The tissue expression of class I molecules is ubiquitous and codominant. MHC class I molecules present peptide antigens necessary for the activation of cytotoxic T-cells.

Class II MHC molecules are noncovalently associated heterodimers of two transmembrane glycoproteins, the 35 kD α chain and the 28 kD β chain (Brown et al., 1993). In humans, class II molecules occur as three different isotypes, termed human leukocyte antigen DR (HLA-DR), HLA-DP and HLA-DQ. Polymorphism in DR is restricted to the β chain, whereas both chains are polymorphic in the DP and DQ isotypes. Class II molecules are expressed codominantly, but in contrast to class I, exhibit a restricted tissue distribution: they are present only on the surface of cells of the immune system, for example dendritic cells, macrophages, B lymphocytes, and activated T lymphocytes. They are also expressed on human adrenocortical cells in the zona reticularis of normal adrenal glands and on granulosa-lutein cells in corpora lutea of normal ovaries (Kahoury et al., 1990). Their major biological role is to bind antigenic peptides and present them on the surface of antigen presenting cells (APC) for recognition by CD4 helper T (Th) lymphocytes (Babbitt et al., 1985). MHC class II molecules can also be expressed on the surface of non-immune system cells, for example, cells that express MHC class II molecules during a pathological inflammatory response. These cells may include synovial cells, endothelial cells, thyroid stromal cells and glial cells.

Class III MHC molecules are also associated with immune responses, but encode somewhat different products. These include a number of soluble serum proteins, enzymes and proteins like tumor necrosis factor or steroid 21-hydroxylase enzymes. In humans, class III molecules occur as three different isotypes, termed Ca, C2 and Bf (Kuby, 1994).

Since Th cell activation is a crucial event of the initiation of virtually all immune responses and is mediated through class II molecules, class II MHC offers itself as a target for immunomodulation (Baxevanis et al., 1980; Rosenbaum et al., 1981; Adorini et al., 1988). Besides peptide presentation, class II molecules can transduce various signals that influence the physiology of APC. Such signals arise by the interaction of multiple class II molecules with an antibody or with the antigen receptor of Th cells (Vidovic et al., 1995a; Vidovic et al., 1995b), and can induce B cell activation and immunoglobulin secretion (Cambier et al., 1991; Palacios et al., 1983), cytokine production by monocytes (Palacios, 1985) as well as the up-regulation of co-stimulatory (Nabavi et al., 1992) and cell adhesion molecules (Mourad et al., 1990).

There is also a set of observations suggesting that class II ligation, under certain conditions, can lead to cell growth arrest or be cytotoxic. Ligation under these conditions is the interaction of a peptide/protein with a class II MHC molecule. There is substantial contradiction about the latter effects and their possible mechanisms. Certain authors claim that formation of a complex of class II molecules on B cells leads to growth inhibition (Vaickus et al., 1989; Kabelitz et al., 1989), whereas according to others class II complex formation results in cell death (Vidovic et al., 1995a; Newell et al., 1993; Truman et al., 1994; Truman et al., 1997; Drenou et al., 1999). In certain experimental systems, the phenomenon was observed with resting B cells only (Newell et al., 1993), or in other systems with activated B cells only (Vidovic et al., 1995a; Truman et al., 1994).

Based on these observations, anti-class II monoclonal antibodies (mAbs) have been envisaged for a number of years as therapeutic candidates. Indeed, this proposal has been supported by the beneficial effect of mouse-derived anti-class II mAbs in a series of animal disease models (Waldor et al., 1983; Jonker et al., 1988; Stevens et al., 1990; Smith et al., 1994).

Despite these early supporting data, to date no anti-MHC class II mAb of human composition has been described that displays the desired cytotoxic and other biological properties. Indeed, despite the relative ease by which mouse-derived mAbs may be derived, work using mouse-derived mAbs has demonstrated the difficulty of obtaining an antibody with the desired biological properties. For example, significant and not fully understood differences were observed in the T cell inhibitory capacity of different anti-class 11 mAbs (Naquet et al., 1983). Furthermore, the application of certain mouse-derived mAbs *in vivo* was associated with unexpected side effects, sometimes resulting in death of laboratory primates (Billing et al., 1983; Jonker et al., 1991).

It is generally accepted that mouse-derived mAbs (including chimeric and so-called 'humanized' mAbs) carry an increased risk of generating an adverse immune response in patients compared to treatment with a human mAb. This risk is potentially increased when treating chronic diseases such as rheumatoid arthritis or multiple sclerosis with any mouse-derived mAb; prolonged exposure of the human immune system to a non-human molecule often leads to the development of an adverse immune reaction. Furthermore, it is significantly difficult to obtain mouse-derived antibodies with the desired specificity or affinity to the desired antigen. Such observation may have a significant influence on the overall therapeutic effect or advantage provided by mouse-derived mAbs. Finally, even if a mouse mAb could be identified that displayed the desired specificity or affinity, often these desired features are detrimentally affected during the 'humanization' or 'chimerization' procedures necessary to reduce immunogenic potential. Once a mouse-derived mAb has been 'humanized' or chimerized, then it is significantly difficult to optimize its specificity or affinity.

The art has sought over a number of years for anti-MHC class II mAbs of human composition that show biological properties suitable for use in a pharmaceutical composition for the treatment of humans. Workers in the field have practiced the process steps of first identifying a mouse-derived mAb, and then modifying the structure of this mAb with the aim of improving immunotolerance of this non-human molecule for human patients (for further details, see Jones et al., 1986; Riechmann et al., 1988; Presta, 1992). This modification is typically made using so-called 'humanization' procedures or by fabricating a human-mouse chimeric mAb. Other workers have attempted to identify human antibodies that bind to human antigens having desired properties within natural repertoires of human antibody diversity. For example, by exploring the foetal-tolerance mechanism in pregnant women (Bonagura et al., 1987) or by panning libraries of natural diversities of antibodies (Stausbøl-Grøn et al., 1996; Winter et al., 1994). However, to date no anti-MHC class II mAb of human composition has been described that displays the desired biological properties of cytotoxicity, tumor selectivity, specificity, low immunogenicity and affinity.

Furthermore, in the prior art the human monoclonal antibody UL-5A1 which interacts with DR1 (Dra/DRB1*0101) molecules on lymphoblastoid cell lines thereby inducing lysis of the cells is described, see Wölpl et al., Tissue Antigens, 1998, pages 258 to 269. Said antibody reacts specifically with cells that had been activated (abstract). The reactivity of said antibody is modulated by activation of cells, i.e. non-activated cells are not stained by the labeled antibody whereas activated cells are stained (page 262 Table 3 and page 267 right-hand col. 2^{nd} paragraph). The F-value is the measure for binding to activated cells in comparison to non-activated cells (see page 259 right-hand col. 4^{th} paragraph - page 260 left-hand col. 1^{st} paragraph). Said F-value is considered to give an indication for the IC50 value of the antibody. It is suggested to use UL-5A1 for therapy of EBV-induced polyclonal lymphoproliferative disease (page 267 right-hand col. 4^{th} paragraph).

In addition, in Bunce et al., Tissue Antigens, 1990, pages 100 to 102, a human monoclonal antibody NDS40 which recognizes HLA-DRw13b on B cells from chronic lymphatic leukemia patients is described (page 100 left-hand col. 2^{nd} paragraph). Said antibody is cytotoxic and is of the lambda IgM class (abstract). Furthermore, said antibody is used as a diagnostic tool to differentiate HLA-DRw12 and HLA-DRw13b.

Finally, Viken et al., Human Immunology, 1995, pages 200 to 206, describes the cytotoxic human IgM(kappa) mAb 5643 which interacts with HLA DR (α, β 1*0801) subtype of DR8. The epitope interacting with said antibody is localized within the α-helix of the DR β chain including the amino acid residues 57 and 67 (abstract). Said antibody is used to discriminate the subtypes of DR8 (page 202 right-hand col. 4^{th} paragraph).

For therapeutic purposes a peptide/protein reacting with most or all allelic forms of a human class II MHC molecule would be desirable. Moreover, the candidate peptide/protein should trigger a cytotoxic mechanism shared by a wide range of lymphoid tumors, or should be able to activate different cytotoxic mechanisms. To allow for a wide range of possible applications, the peptide/protein desired should mediate its cytotoxic effect without the dependence on further components of the immune system. For therapeutic purposes most patients receive for the treatment of e.g. cancer standard chemo- or radiotherapy. Most of these treatments leave the patient immunocompromised. Any additional treatment that relies on an intact immune system is therefore likely to fail. The underlying problem is further demonstrated in humans who suffer from a disease that destroys the immune system, e.g. HIV. Opportunistic infections and malignant transformations are able to escape the immune-surveillance and cause further complications.

Thus, the technical problem underlying the present invention is to provide means causing or leading to the killing of cells, preferably lymphoid tumor cells, for therapeutic purposes with a minimum of non-desired side effects.

The solution to the above technical problems is achieved by the embodiments characterized in the claims. In particular, the above problem is solved by a peptide/protein as specified in the appended claims comprising at least one antibody-based antigen-binding domain of human composition with binding specificity for an antigen expressed on the surface of a human cell, wherein binding of a multivalent composition of one or more of said peptides/proteins to said antigen expressed on the surface of a cell causes or leads to killing of said cell.

As used herein, the term "peptide" relates to molecules consisting of one or more chains of multiple, i. e. two or more, amino acids linked via peptide bonds. The term "protein" refers to peptides where at least part of the peptide has or is able to acquire a defined three-dimensional arrangement by forming secondary, tertiary, or quaternary structures within and/or between its peptide chain(s). This definition comprises proteins such as naturally occurring or at least partially artificial proteins, as well as fragments or domains of whole proteins, as long as these fragments or domains are able to acquire a defined three-dimensional arrangement as described above.

In this context, "peptide/protein comprising at least one antibody-based antigen-binding domain" refers to an immunoglobulin (or antibody) or to a functional fragment thereof. The term "functional fragment" refers to a fragment of an immunoglobulin which retains the antigen-binding moiety of an immunoglobulin. Functional immunoglobulin fragments according to the present invention may be Fv (Skerra and Plückthun, 1988), scFv (Bird et al., 1988; Huston et al., 1988), disulftde-linked Fv (Glockshuber et al., 1992; Brinkmann et al., 1993), Fab, F(ab')₂ fragments or other fragments well-known to the practitioner skilled in the art, which comprise the variable domains of an immunoglobulin or functional immunoglobulin fragment.

Examples of peptides/proteins consisting of one chain are single-chain Fv antibody fragments, and examples for peptides/proteins consisting of more chains are Fab antibody fragments.

As used herein, an "antibody-based antigen-binding domain of human composition" means a peptide/protein comprising at least an antibody VH domain and an antibody VL domain, wherein a homology search in a database of protein sequences comprising immunoglobulin sequences results for both the VH and the VL domain in an immunoglobulin domain of human origin as hit with the highest degree of sequence identity. Such a homology search may be a BLAST search, e.g. by accessing http://www.ncbi.nlm.nih.gov/BLAST and performing a "BasicBLAST" search using the "blastp" routine. Preferably, such a composition does not result in an adverse immune response thereto when administered to a human recipient

As used herein, a "multivalent composition" means a composition comprising at least two of said antigen-binding domains. Preferably, said at least two antigen-binding domains are in close proximity so as to mimic the structural arrangement relative to each other of binding sites comprised in a full immunoglobulin molecule. Examples for multivalent compositions are full immunoglobulin molecules (e.g. IgG, IgA or IgM molecules) or multivalent fragments thereof (e.g. F(ab')₂). Additionally, multivalent compositions of higher valencies may be formed from two or more multivalent compositions (e.g. two or more full immunoglobulin molecules), e.g. by cross-linking. Multivalent compositions, however, may be formed as well from two or more monovalent immunoglobulin fragments, e.g. by self-association as in mini-antibodies, or by cross-linking.

As used herein, the term "mini-antibody fragment" means a multivalent antibody fragment comprising at least two antigen-binding domains multimerized by self-associating domains fused to each of said domains (Pack, 1994), e.g. dimers comprising two scFv fragments, each fused to a self-associating dimerization domain. Dimerization domains, which are particularly preferred, include those derived from a leucine zipper (Pack and Plückthun, 1992) or helix-tum-helix motif (Pack et al., 1993).

As used herein, "activated cells" means cells of a certain population of interest, which are not resting. Activation might be caused by mitogens (e.g., lipopoysaccharide, phytohemagglutinine) or cytokines (e.g., interferon gamma). Preferably, said activation occurs during tumor transformation (e.g., by Epstein-Barr virus, or "spontaneously"). Preferably, activated cells are characterized by the features of MHC class II molecules expressed on the cell surface and one or more additional features including increased cell size, cell division, DNA replication, expression of CD45 or CD11 and production/secretion of immunoglobulin.

As used herein, "non-activated cells" means cells of a population of interest, which are resting and non-dividing. Said non-activated cells may include resting B cells as purified from healthy human blood. Such cells can, preferably, be characterized by lack or reduced level of MHC class II molecules expressed on the cell surface and lack or reduced level of one or more additional features including increased cell size, cell division, DNA replication, expression of CD45 or CD11 and production/secretion of immunoglobulin.

"At least 5-fold lower IC50" means that the concentration of a multivalent composition comprising at least one peptide/protein of the present invention that is required to kill 50% of activated cells is at least five times less than the concentration of the multivalent composition required to kill non-activated cells. Preferably, the concentration required to kill 50% of non-activated cells cannot be achieved with therapeutically appropriate concentrations of the multivalent composition. Most preferably, the IC50 value is determined in the test described below in example G.

"Lymphoid cells" when used in reference to a cell line or a cell, means that the cell line or cell is derived from the lymphoid lineage. "Lymphoid cells" include cells of the B and the T lymphocyte lineages, and of the macrophage lineage.

Cells, which are "non lymphoid cells and express MHC class II", are cells other than lymphoid cells that express MHC class II molecules, e.g. during a pathological inflammatory response. For example, said cells may include synovial cells, endothelial cells, thyroid stromal cells and glial cells, and it may also comprise genetically altered cells capable of expressing MHC class II molecules.

The terms "apoptosis" and "apoptotic activity" refer to the orderly or controlled form of cell death in mammals that is accompanied by one or more characteristic cell changes, including condensation of cytoplasm, loss of plasma membrane microvilli, segmentation of the nucleus, degradation of chromosomal DNA or loss of mitochondrial function. Apoptosis follows a very stringent time course and is executed by caspases, a specific group of proteases. Apoptotic activity can be determined and measured, for instance, by cell viability assays, Annexin V staining or caspase inhibition assays. Apoptosis can be induced using a cross-linking antibody such as anti-CD95 as described in Example H.

As used herein, the term "first domain of the α-chain of HLA-DR" means the N-terminal domain of the alpha-chain of the MHC class II DR molecule.

As used herein, the term "first domain of the β-chain of HLA-DR" means the N-terminal domain of the beta-chain of the MHC class II DR molecule.

The term "innate pre-programmed process" refers to a process that, once it is started, follows an autonomous cascade of mechanisms within a cell, which does not require any further auxillary support from the environment of said cell in order to complete the process.

As used herein, the term "HuCAL" refers to a fully synthetic human combinatorial antibody library as described in Knappik et al. (2000).

As used herein, the term "CDR3" refers to the third complementarity-determining region of the VH and VL domains of antibodies or fragments thereof, wherein the VH CDR3 covers positions 95 to 102 (possible insertions after positions 100 listed as 100a to 100z), and VL CDR3 positions 89 to 96 (possible insertions in Vλ after position 95 listed as 95a to 95c) (see Knappik et al., 2000).

As used herein, the term "hybridizes under stringent conditions" is intended to describe conditions for hybridization and washing under which nucleotide sequences at least 60% homologous to each other typically remain hybridized to each other. Preferably, the conditions are such that sequences at least 65%, more preferably at least 70%, and even more preferably at least 75% homologous to each other typically remain hybridized to each other. Such stringent conditions are known to those skilled in the art and can be found in Current Protocols in Molecular Biology, John Wiley & Sons, New York. (1989), 6.3.1-6.3.6. A preferred, non-limiting example of stringent hybridization conditions is hybridization in 6 x sodium chloride/sodium citrate (SSC) at about 45°C, followed by one or more washes in 0.2 x SSC, 0.1 % SDS at 50°-65°C.

A multivalent composition of at least one peptide/protein according to the invention and as specified in the appended claims is capable of causing cell death of activated cells, preferably lymphoid tumor cells without requiring any further additional measures such as chemotherapy and with limited immunogenic side effects on the treated patient. Further, the multivalent composition comprising a peptide/protein according to the invention has the capability of binding to at least one epitope on the target antigen, however, several epitope binding sites might be combined in one molecule. Preferably, the multivalent composition comprising a peptide/protein according to the invention shows at least 5-fold, or more preferably 10-fold higher killing activity against activated cells compared to non-activated cells. This higher activity on activated cells can be expressed as the at least 5-fold lower IC50 value on activated versus non-activated cells or as the higher percentage of killing of activated cells versus non-activated cells when using the same concentration of protein. Under the latter alternative, the multivalent composition comprising a peptide/protein according to the invention and as specified in the appended claims at a given peptide/protein concentration kills at least 50%, preferably at least 80%, of activated cells, whereas the same concentration of a multivalent composition comprising a peptide/protein according to the invention under the same incubation conditions kills less than 15%, preferably less than 10% of the non-activated cells. The assay conditions for determining the IC50 value and the percentage killing activity are described below.

According to a preferred embodiment, the peptide/protein as specified in the appended claims is directed to a lymphoid cell or a non-lymphoid cell that expresses MHC class II molecules. The latter type of cells occurs for example at pathological sites of inflammation and/or autoimmune diseases, e.g. synovial cells, endothelial cells, thyroid stromal cells and glial cells, or it may also comprise genetically altered cells capable of expressing MHC class II molecules.

Preferably, the peptide/protein as specified in the appended claims is directed to lymphoid tumor cells. More preferred are lymphoid tumor cells that represent a disease selected from B cell non-Hodgkin lymphoma, B cell lymphoma, B cell acute lymphoid leukemia, Burkitt lymphoma, Hodgkin lymphoma, hairy cell leukemia, acute myeloid leukemia and B cell precursor leukemia. Most preferred are lymphoid tumor cells from a cell line taken from the list of GRANTA-519, KARPAS-422, DOHH-2, MHH-CALL-4, MN-60, BJAB, L-428, BONNA-12, EOL-1, MHH-PREB-1 and MHH-CALL-2 cell lines.

In a further preferred embodiment the peptide/protein as specified in the appended claims binds to at least one antigen comprising epitopes selected from HLA-DR molecules.

In a further preferred embodiment the peptide/protein as specified in the appended claims binds to at least one epitope in the alpha-chain of an HLA-DR molecule.

Preferably, the peptide/protein as specified in the appended claims binds to at least one epitope in the first domain of the alpha-chain of HLA-DR, the first domain being the N-terminal domain of the chain.

In a preferred embodiment the peptide/protein as specified in the appended claims binds to at least one epitope within the alpha-helix ranging from Glu⁵⁵ to Tyr⁷⁹ of the alpha-chain of HLA-DR.

Alternatively, the peptide/protein as specified in the appended claims binds to at least one epitope in the beta-chain of an HLA-DR molecule. Preferably, the peptide/protein as specified in the appended claims binds to at least one epitope in the first domain of the beta-chain of HLA-DR, the first domain being the N-terminal domain of the chain.

In an embodiment the mechanism of killing a target cell induced by the peptide/protein involves an innate pre-programmed process of said cell.

Preferably, the peptide/protein as specified in the appended claims induces a killing mechanism, which is not an apoptotic cell death process.

In a preferred embodiment the peptide protein as specified in the appended claims induces a killing mechanism which is dependet on the action of proteases other than caspases.

In a further preferred embodiment, the peptide/protein as specified in the appended claims as a multivalent composition shows an IC50 value for activated cells, preferably lymphoid tumor cells, in the assay described below which is lower than 100 nM, preferably lower than 40 nM, most preferably lower than 20 nM.

In a further embodiment the multivalent composition comprises at least one full antibody which is selected from classes IgG1, 2a, 2b, 3, 4, IgA, and IgM.

In a further embodiment the multivalent composition comprises at least one of a F(ab')₂ antibody fragment or mini-antibody fragment

In a preferred embodiment the multivalent composition comprises at least two monovalent antibody fragments selected from Fv, scFv, dsFv and Fab fragments, and further comprises a cross-linking moiety or moieties.

According to a further embodiment the multivalent composition is formed prior to binding to the cell. This can be achieved, e.g. by cross-linking multiple peptide/protein of the present invention.

In an alternative embodiment a multivalent composition is formed after binding to said cell. This can be achieved for example by binding the peptide/protein onto epitopes, which are in close proximity on the cell, followed by cross-linking of said peptide/protein.

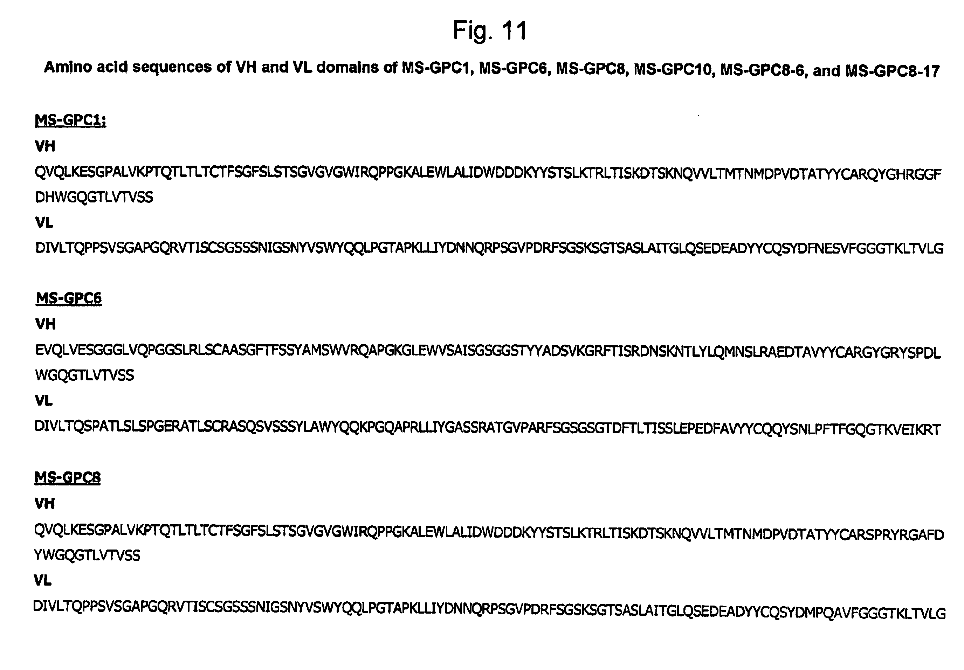

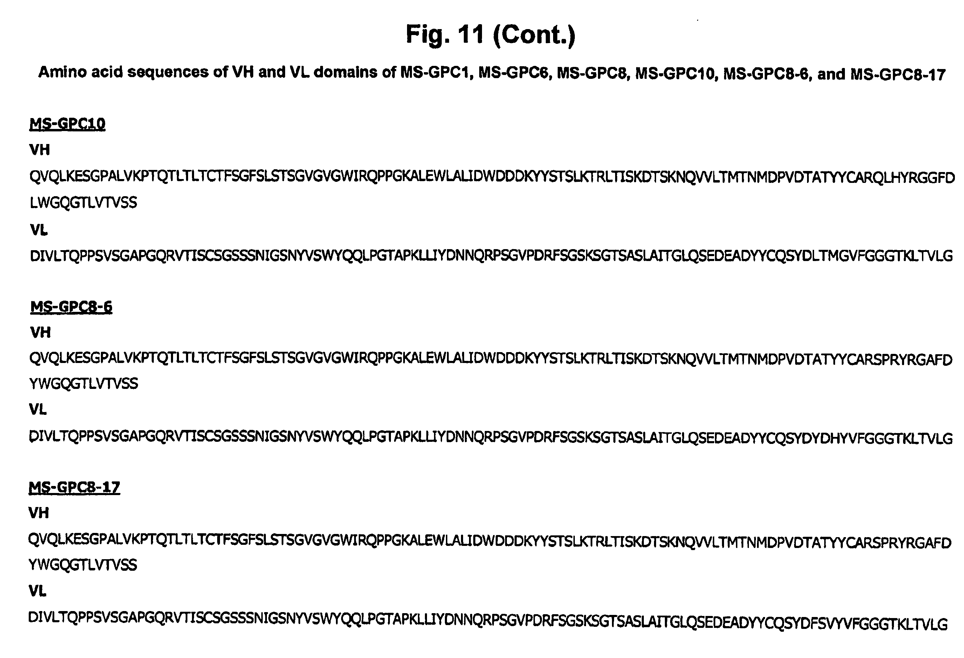

in a preferred embodiment the peptide/protein comprises an antigen-binding domain which consists of a combination of a VH domain and a VL domain, wherein said combination is found in one of the clones selected from the list of MS-GPC-1, MS-GPC-6, MS-GPC-8, MS-GPC-10, MS-GPC-8-6, and MS-GPC-8-17 as shown in Table 1 and Figure 11.

In a further preferred embodiment the peptide/protein as specified in the appended claims comprises an antigen-binding domain which consists of a combination of HuCAL VH2 and HuCAL Vλ1, wherein the VH CDR3 sequence is taken from the consensus CDR3 sequence
nnnnRGnFDn
wherein each n independently represents any amino acid residue; and
wherein the VL CDR3 sequence is taken from the consensus CDR3 sequence
QSYDnnnn
wherein each n independently represents any amino acid residue.

The present invention also relates to a nucleic acid which encodes a peptide/protein as specified in the appended claims according to the invention. Also encompassed are variants of this nucleic acid which hybridize under stringent conditions with a nucleic acid as described above, wherein said variants encode a peptide/protein according to the invention A vector comprising at least one nucleic acid according to the invention or a variant therof is also contemplated.

The invention further relates to a host cell harboring nucleic acids or vectors as described above.

According to the invention, the peptide/protein is prepared by a method comprising the step of expressing at least one nucleic acid or a variant thereof as described above in a suitable expression system, which can be a cell free expression system, but is preferably a cell containing expression system, and isolating the protein therefrom. The nucleic acid for preparing the proteins according to the invention can be obtained by standard laboratory procedures well known to one of ordinary skill in the art (see, e.g. Ausubel et al., 1998)

The present invention further relates to a pharmaceutical composition containing at least one peptide/protein according to the invention, optionally together with a pharmaceutical acceptable carrier and/or diluent

The peptide/protein according to the invention is preferably used for preparing a pharmaceutical composition for treating animals, preferably humans.

The peptide/protein according to the invention is particularly useful in the treatment of cancers, preferably cancer comprising cells that express MHC class II antigen. Such forms of cancer include Hodgkin, non-Hodgkin and Burkitt lymphoma, B and certain T cell lymphoma, B cell acute lymphoid leukemia, acute myeloid leukemias and hairy cell leukemia.

In a further embodiment the protein/peptide according to the invention is used for preparing a pharmaceutical composition for treating diseases of the immune system including conditions such as rheumatoid arthritis, juvenile arthritis, multiple sclerosis, Grave's disease, insulin-dependent diabetes, narcolepsy, psoriasis, systemic lupus erythematosus, ankylosing spondylitis, transplant rejection, graft vs. host disease, Hashimoto's disease, myasthenia gravis, pemphigus vulgaris, glomerulonephritis, thyroiditis, pancreatitis, insulitis, primary biliary cirrhosis, irritable bowel disease and Sjogren syndrome.

The invention further relates to a diagnostic composition containing at least one peptide/protein and/or nucleic acid according to the invention, optionally together with further reagents, such as buffers, for performing the diagnosis.

In a preferred embodiment the diagnostic composition contains the peptide/protein according to the invention cross-linked by at least one moiety. Such moieties can be for example antibodies recognizing an epitope present on the peptide/protein such as the FLAG peptide epitope (Hopp et al., 1988; Knappik and Plückthun, 1994) or bifunctional chemical compounds reacting with a nucleophilic amino acid side chain as present in cysteine or lysine (King et al., 1994). Methods for cross-linking peptides/proteins are well known to the practitioner of ordinary skill in the art.

A diagnostic composition containing at least one nucleic acid and/or variant thereof according to the invention is also contemplated.

Also described is a method for selectively killing activated cells comprising the step of bringing the target cells in contact with a multivalent composition comprising at least one peptide/protein according to the invention.

Furthermore, the present invention relates to a kit comprising at least one peptide/protein according to the present invention, and a cross-linking moiety.

Additionally, the present invention relates to a kit comprising (i) a peptide/protein according to the present invention, (ii) a detectable moiety or moieties, and (iii) reagents and/or solutions to effect and/or detect binding of (i) to an antigen.

The present invention further relates to a multivalent composition comprising at least one peptide/protein and comprising at least two antigen binding domains.

### Figure Captions

### Figure 1

Specificity of the anti-HLA DR antibody fragments MS-GPC-1, 6, 8 & 10 isolated from the HuCAL library to HLA-DR protein, a mouse-human chimeric HLA protein and negative control proteins (lysozyme, transferrin, BSA and human β-globulin). Specificity was assessed using standard ELISA procedures. A non-related antibody fragment (irr. scFv) was used as control.

### Figure 2

Reactivity of the anti-HLA-DR antibody fragments (MS-GPC-1, 6, 8 and 10) to various cells lines expressing MHC class II molecules. "+" represents strong reactivity as detected using standard immunofluorescence procedure. "+/-" represents weak reactivity and "-" represents no detected reactivity between an anti-HLA-DR antibody fragment and a particular cell line.

### Figure 3

Viability of tumor cells in the presence of monovalent and cross-linked anti-HLA DR antibody fragments as accessed by trypan blue staining. Viability of GRANTA-519 cells was assessed 4 h after incubation with anti-HLA DR antibody fragments (MS-GPC-1, 6, 8 and 10) with and without anti-FLAG M2 mAb as cross-linking agent.

### Figure 4

Killing of activated versus non-activated cells. MHH-PREB-1 cells are activated with Lipopolysaccharide and Interferon-gamma, and subsequently incubated for 4 h with 200 nM of anti-HLA DR antibody fragment MS-GPC-8 cross-linked using 100 nM of anti-FLAG M2 mAb. Little killing of control non-activated MHH-PREB-1 cells is seen.

### Figure 5

IC50 values for activated and non-activated cells. Dilution series of the four anti-HLA DR antibody fragments (MS-GPC-1, 6, 8 and 10) cross-linked using the anti-FLAG M2 mAb shows that 50% killing of activated GRANTA-519 cells is achieved at less than 100 nM bivalent equivalent concentration of anti-HLA DR antibody fragment 50% killing is not measurable for non-activated resting B cells over the range of 2 to 200 nM bivalent equivalent concentration of anti-HLA DR antibody fragment.

### Figure 6

a. Incubation of Priess cells with the anti-HLA DR antibody fragment MS-GPC-8, cross-linked using the anti-FLAG M2 mAb, shows more rapid killing than a culture of Priess cells induced into apoptosis using anti-CD95 mAb. An Annexin V/PI staining technique identifies dead cells.
b. Incubation of Priess cells with the anti-HLA DR antibody fragment MS-GPC-8, cross-linked using the anti-FLAG M2 mAb, shows little evidence of an apoptotic mechanism compared to an apoptotic culture of Priess cells induced using anti-CD95 mAb. An Annexin V/PI staining technique identifies apoptotic cells.

### Figure 7

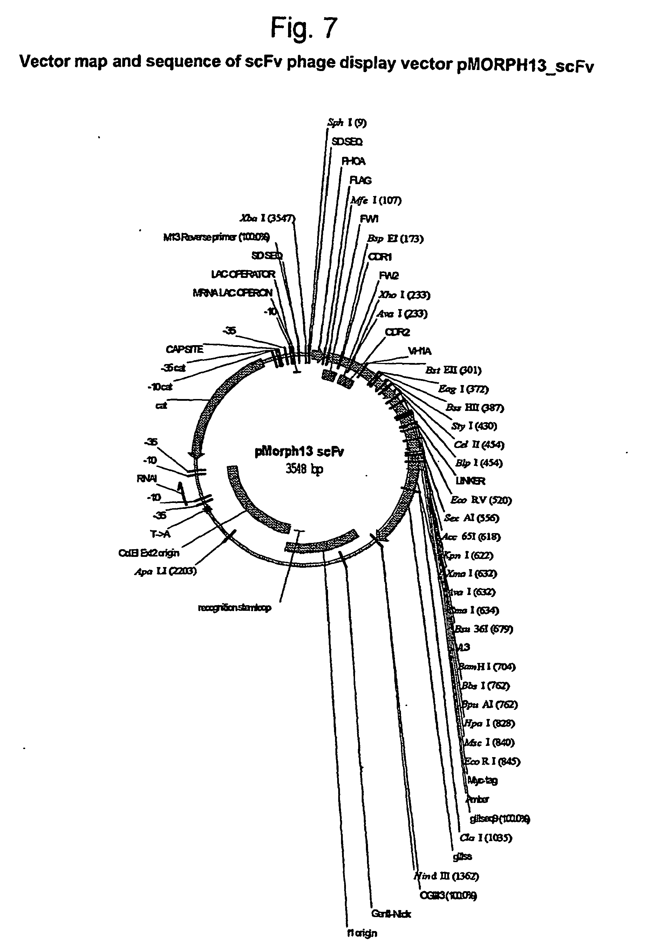

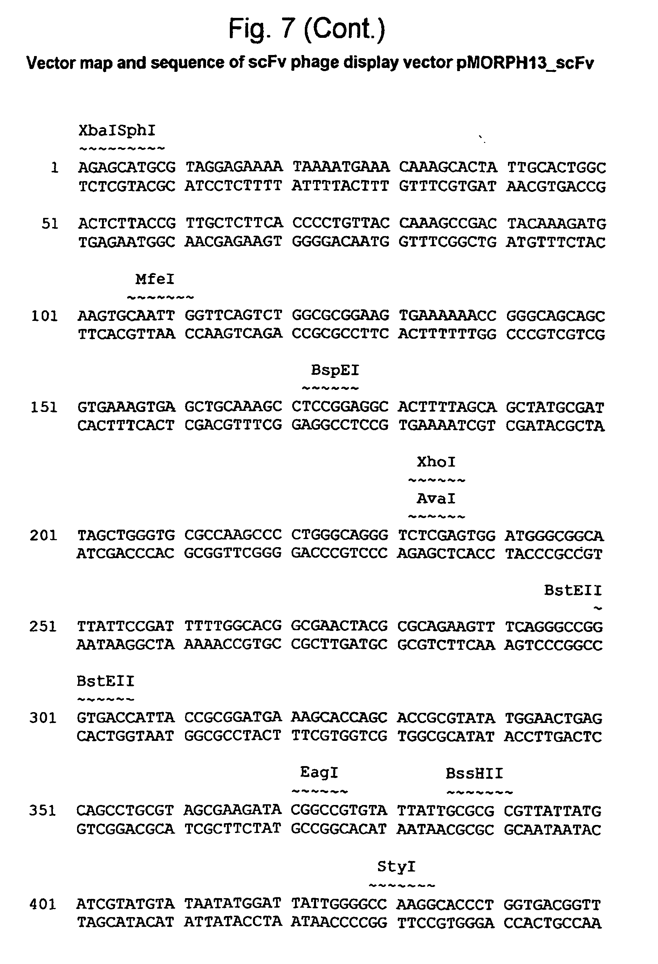

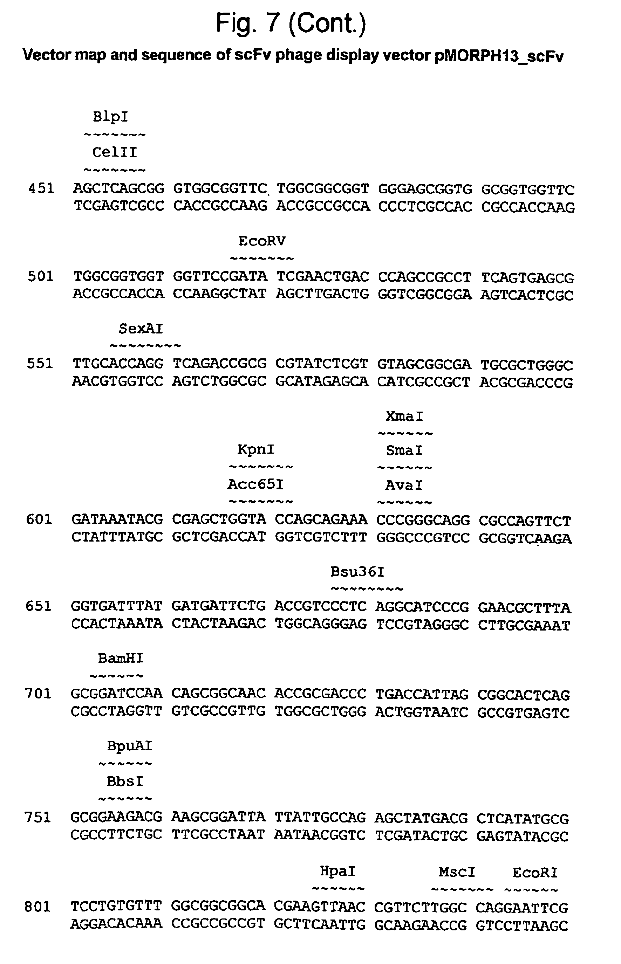

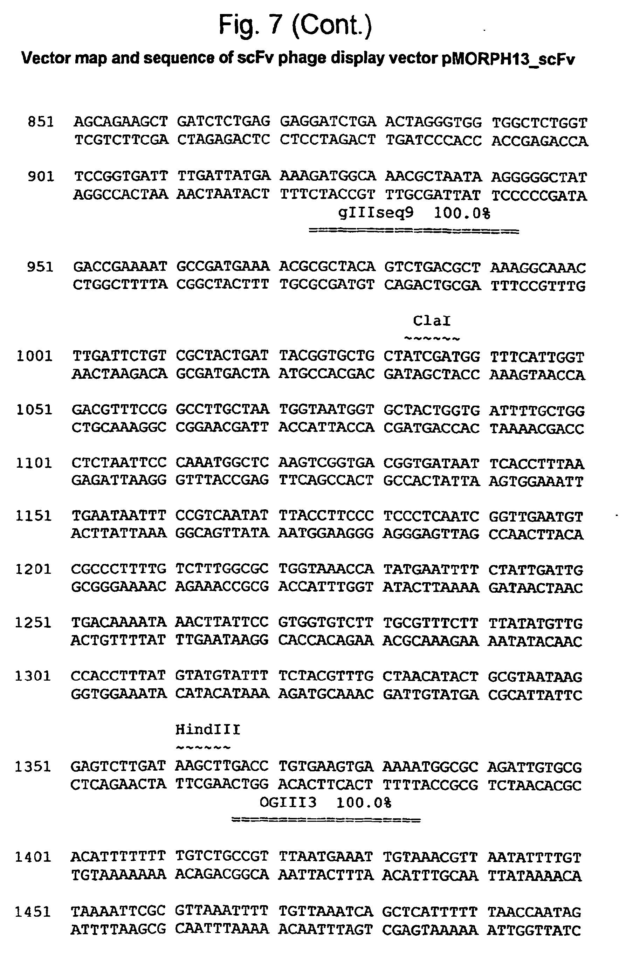

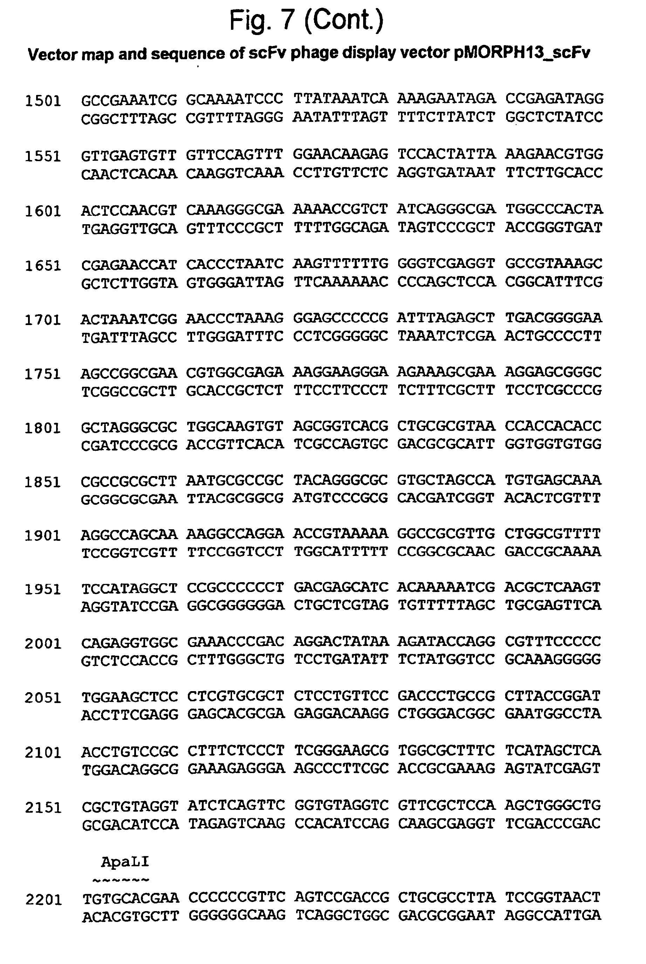

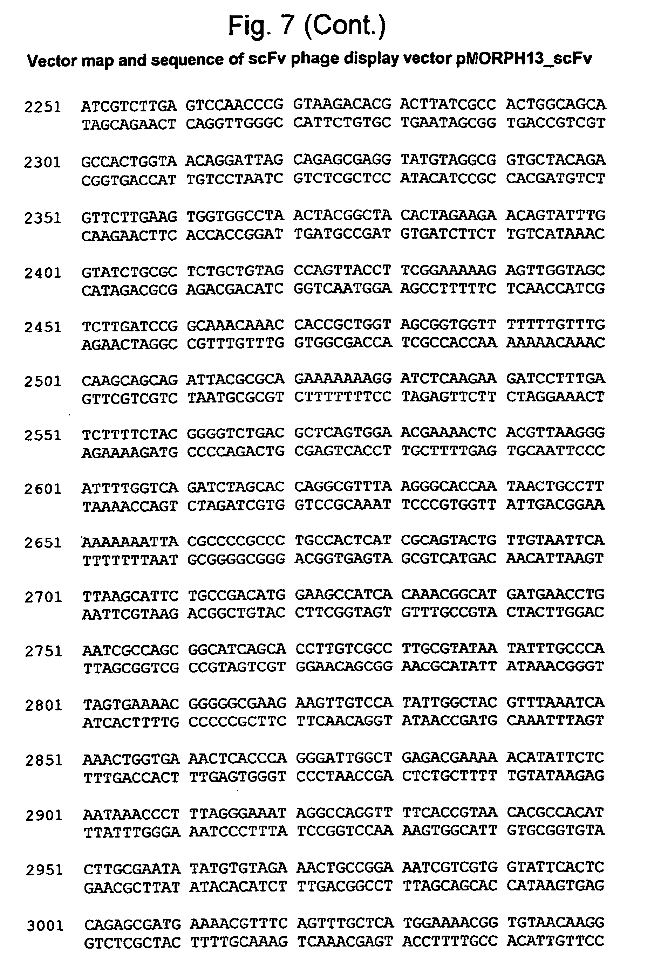

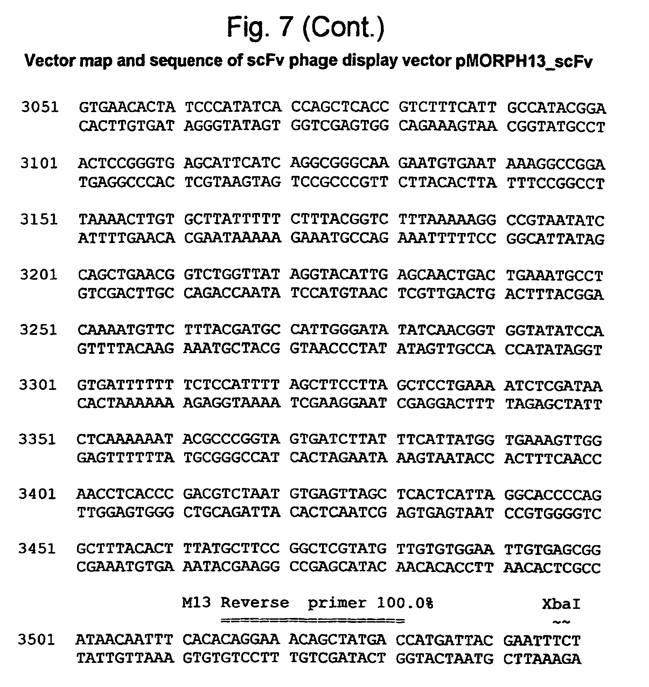

Vector map and sequence of scFv phage display vector pMORPH13_scFv.
The vector pMORPH13_scFv is a phagemid vector comprising a gene encoding a fusion between the C-terminal domain of the gene III protein of filamentous phage and a HuCAL scFv. In Fig. 7, a vector comprising a model scFv gene (combination of VH1A and Vλ3 (Knappik et al., 2000) is shown.

### Figure 8

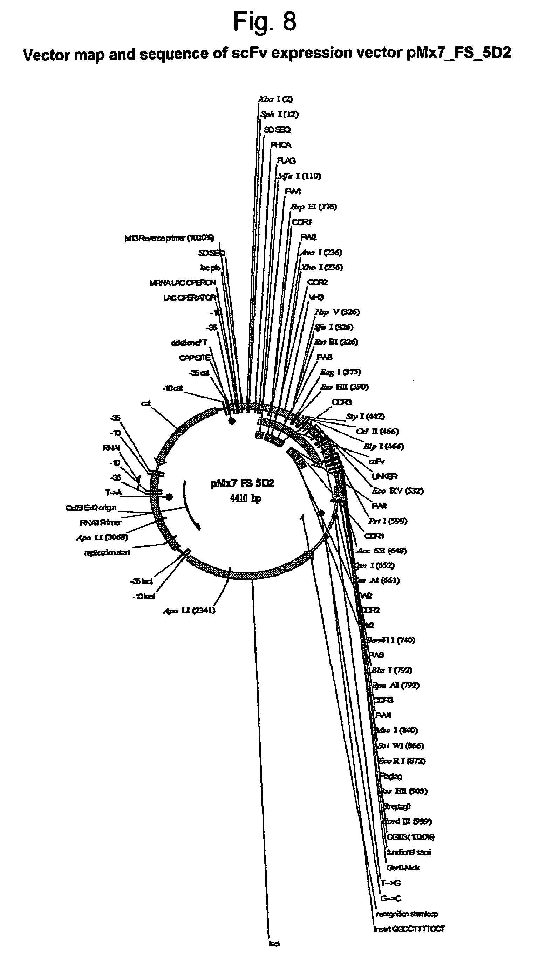

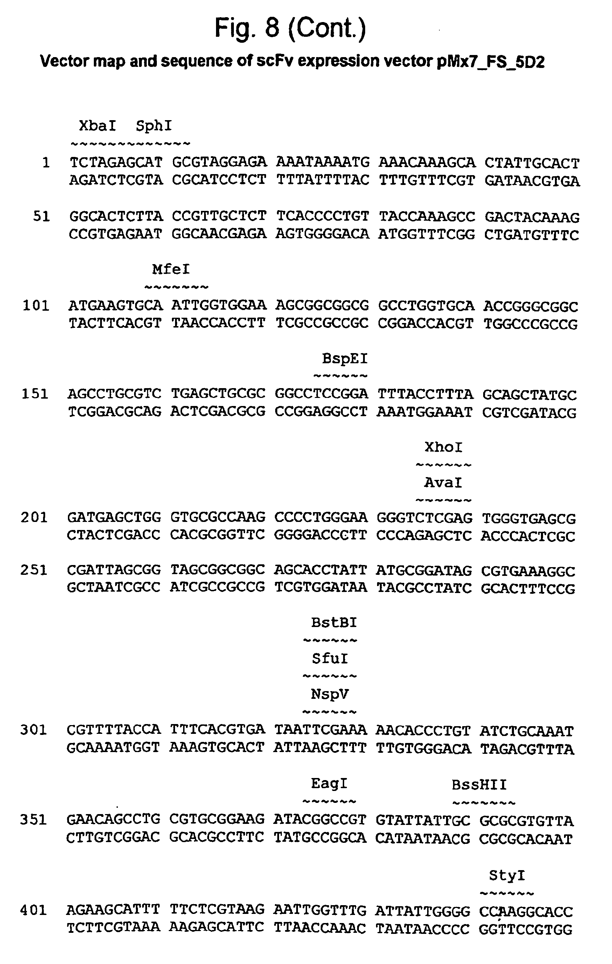

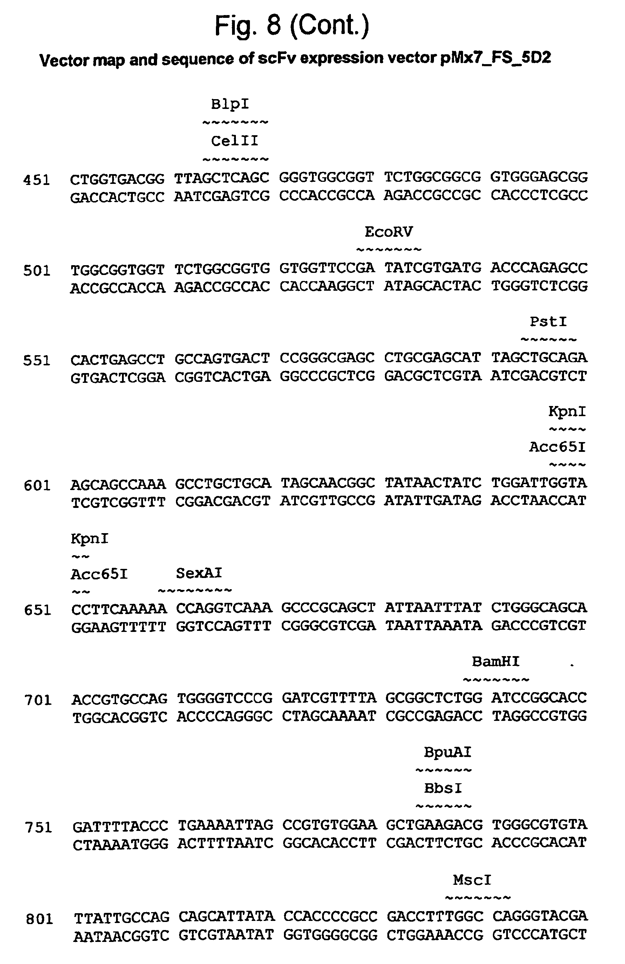

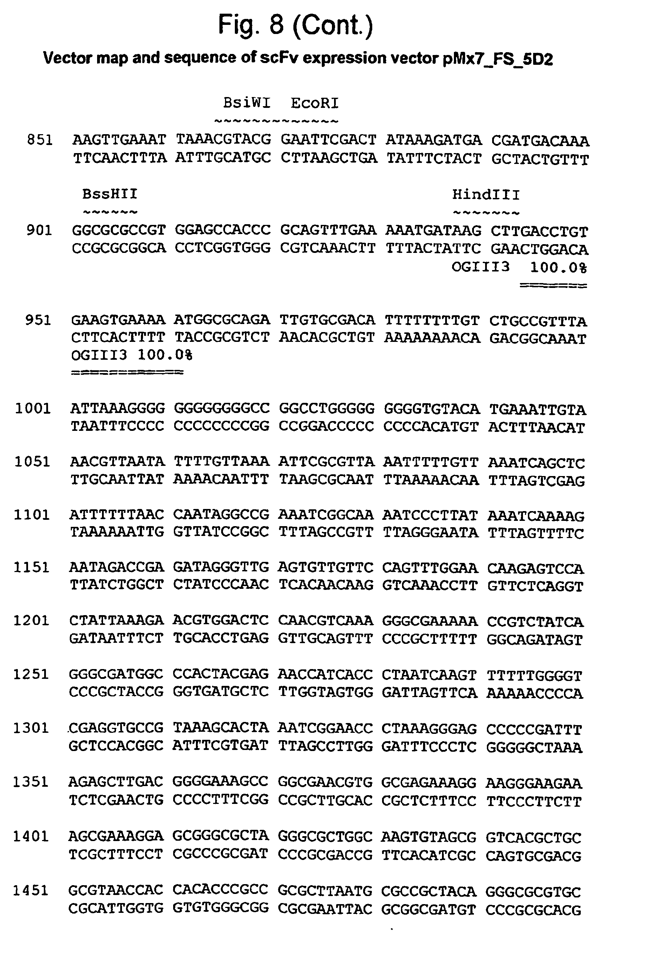

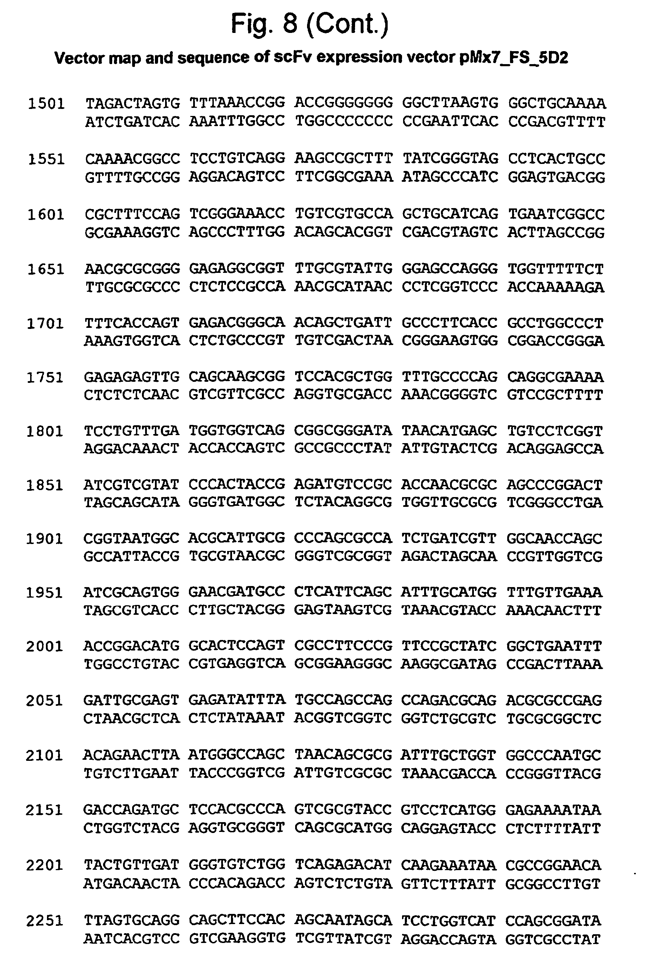

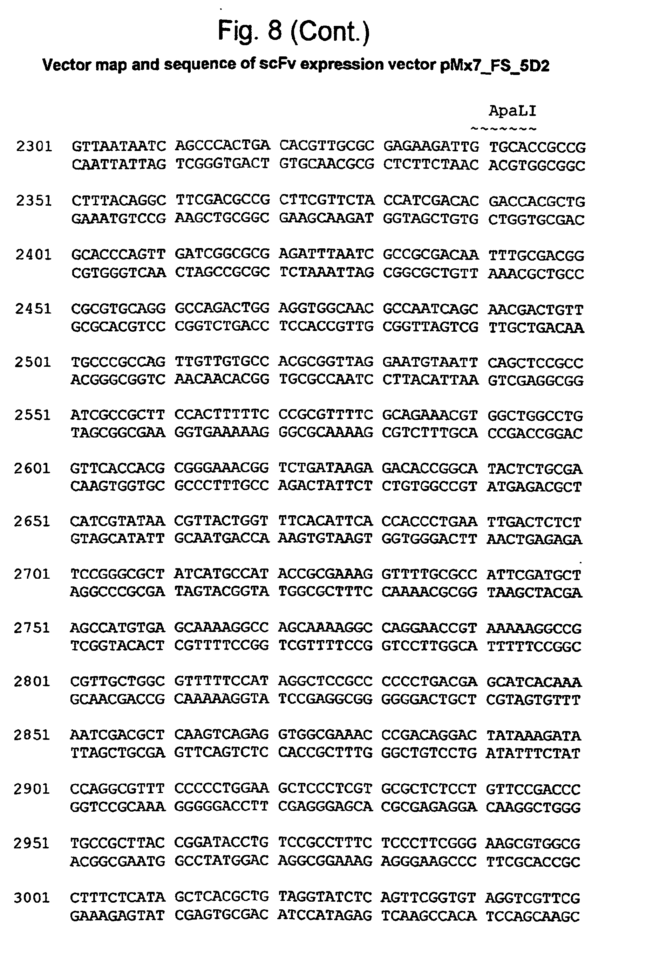

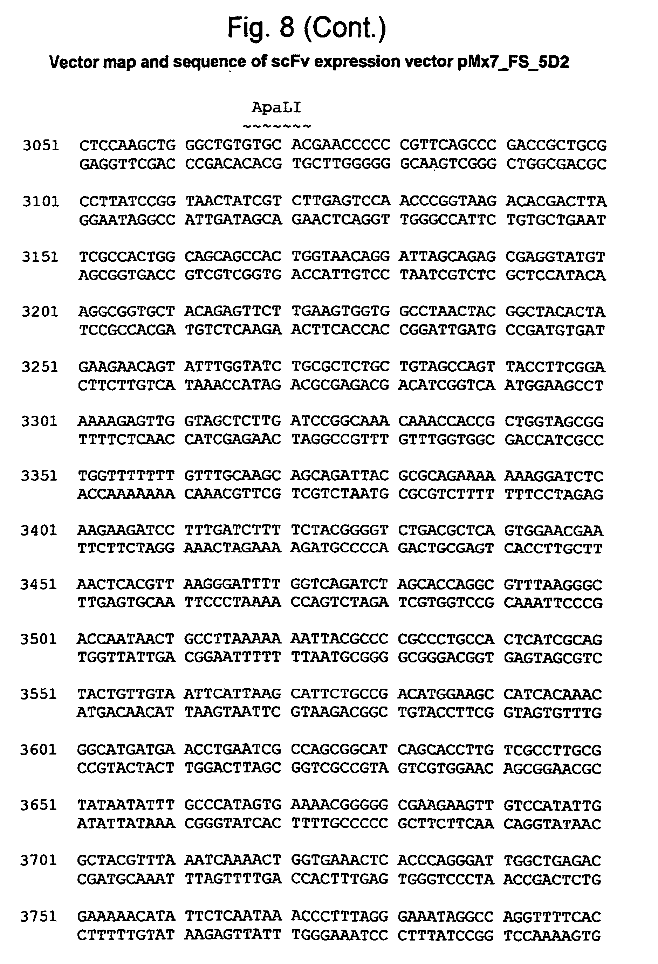

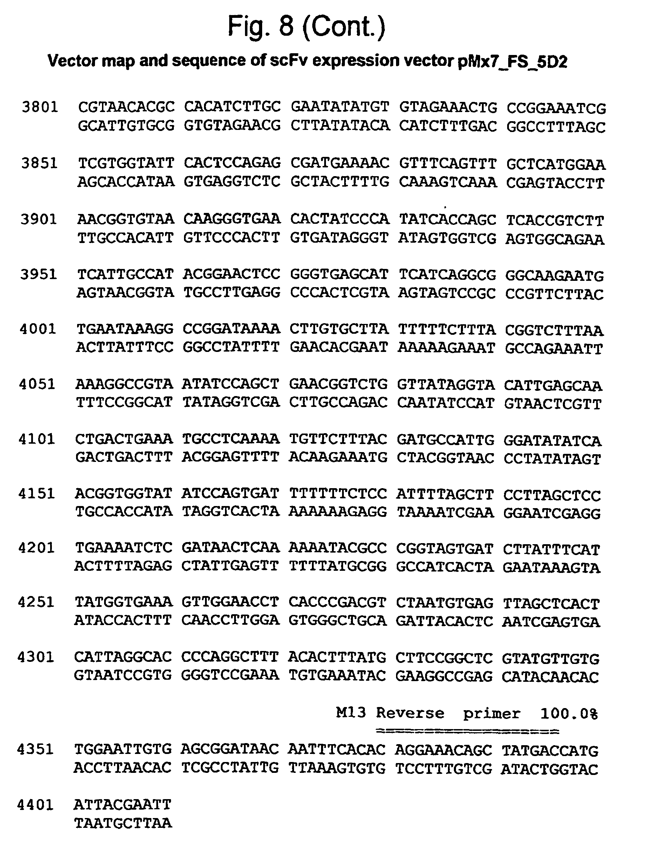

Vector map and sequence of scFv expression vector pMx7_FS_5D2.
The expression vector pMx7_FS_5D2 leads to the expression of HuCAL scFv fragments (in Fig. 8, the vector comprises a gene encoding a "dummy" antibody fragment called "5D2") when VH-CH1 is fused to a combination of a FLAG tag (Hopp et al., 1988; Knappik and PlUckthun, 1994) and a STREP tag II (WSHPQFEK) (IBA GmbH, G6tfingen, Germany; see: htttp://vww.iba-go.de and Schmidt and Skerra, 1993; Schmidt and Skerra. 1994; Schmidt et al., 1996; Voss and Skerra, 1997).

### Figure 9

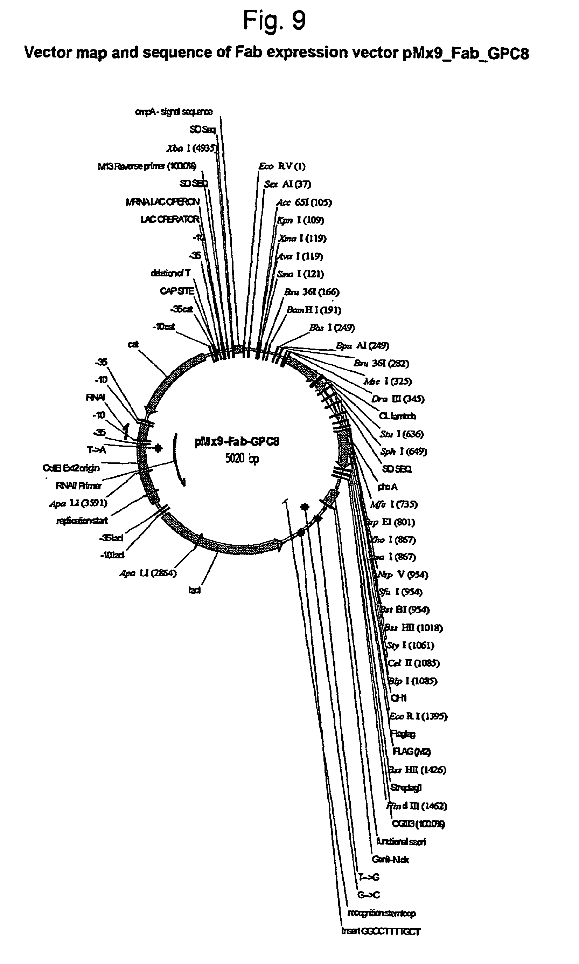

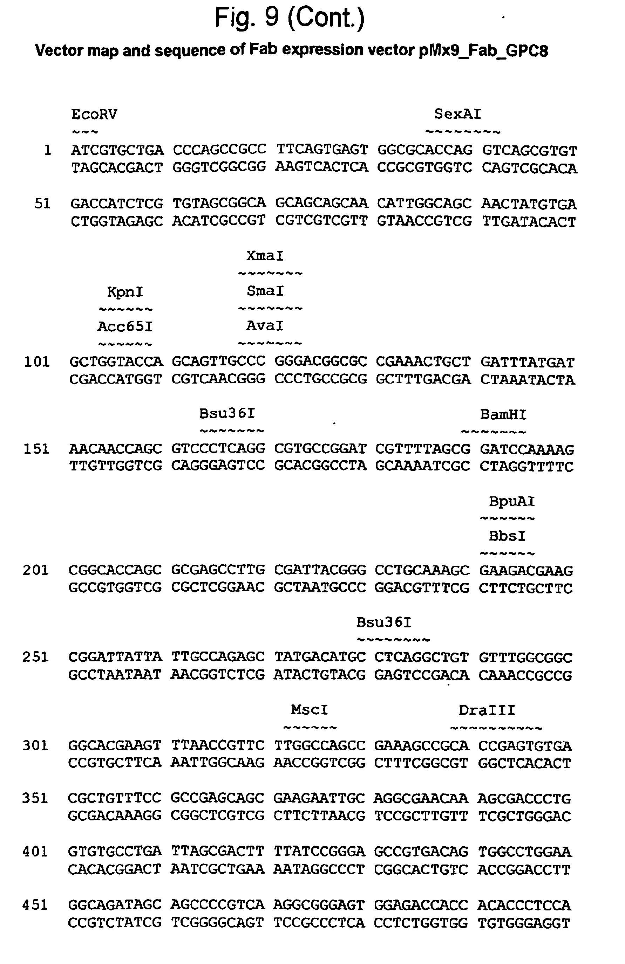

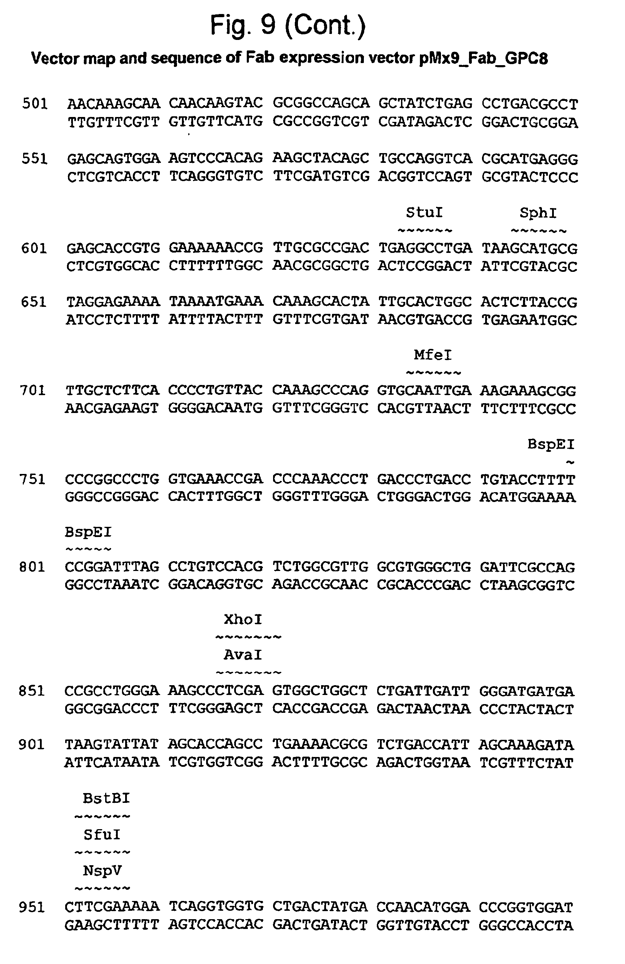

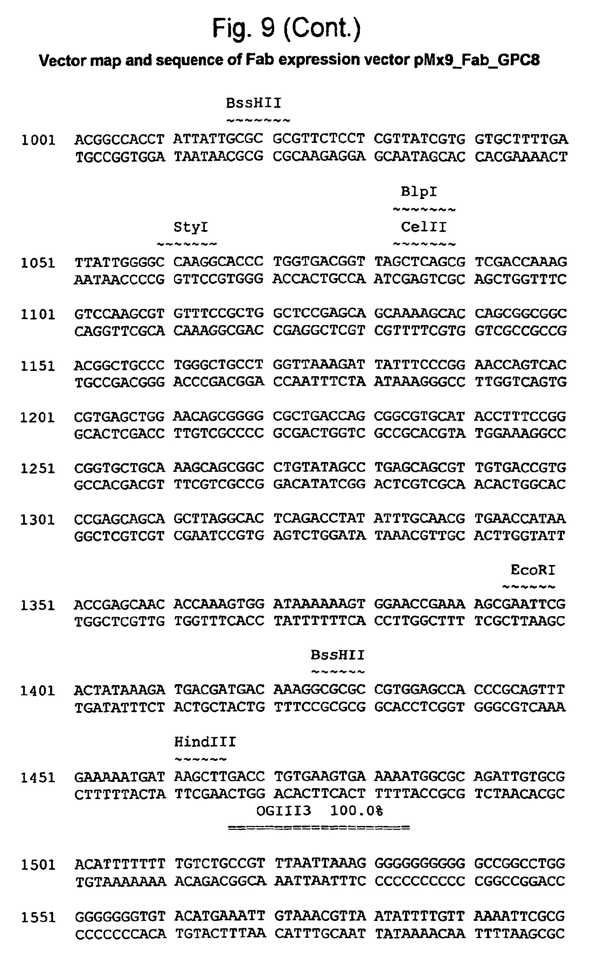

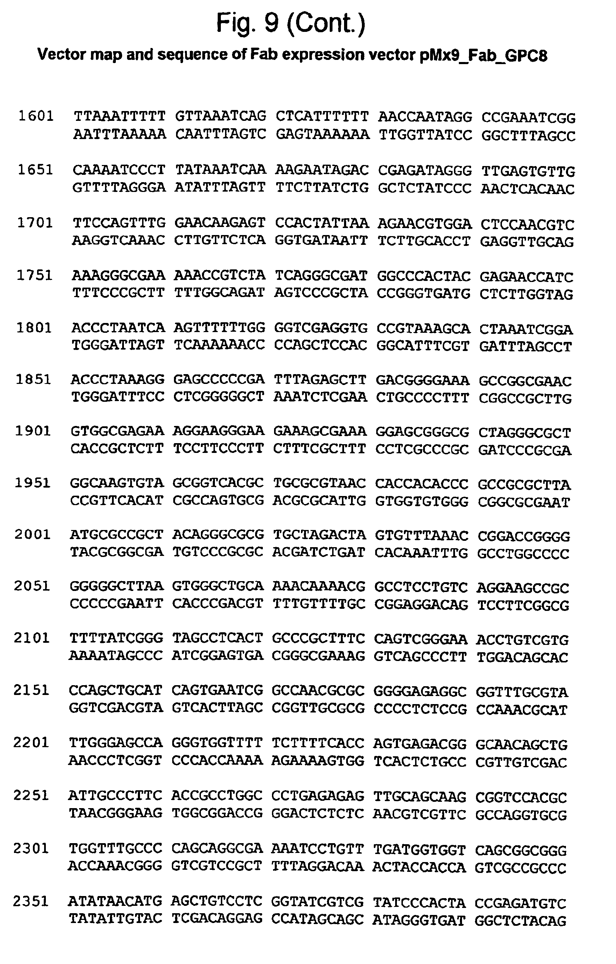

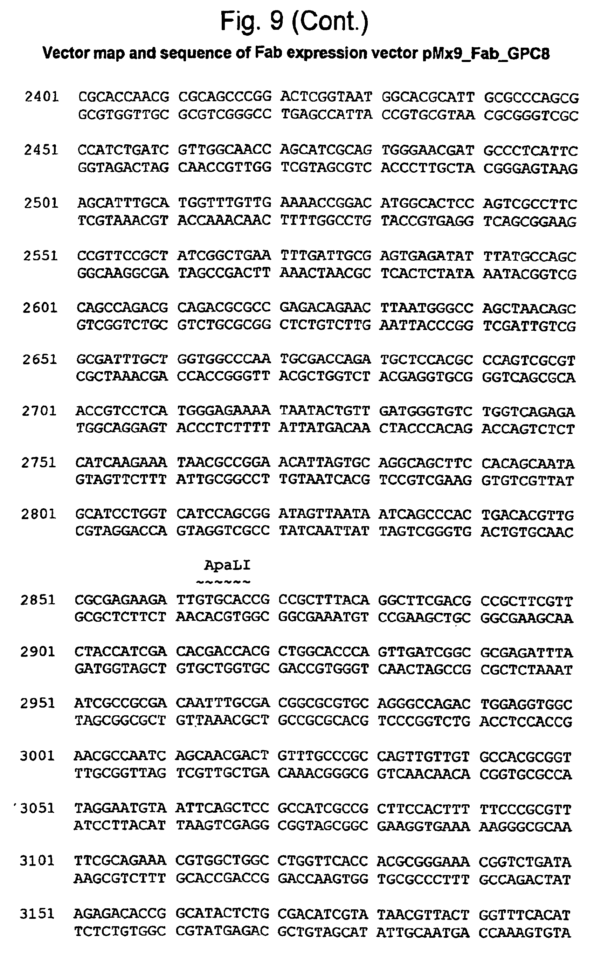

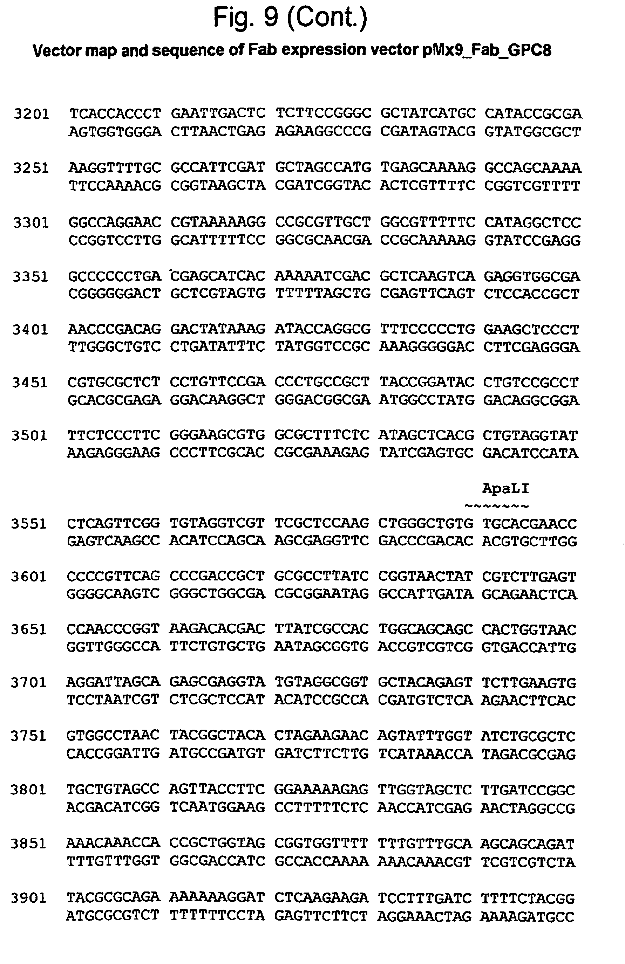

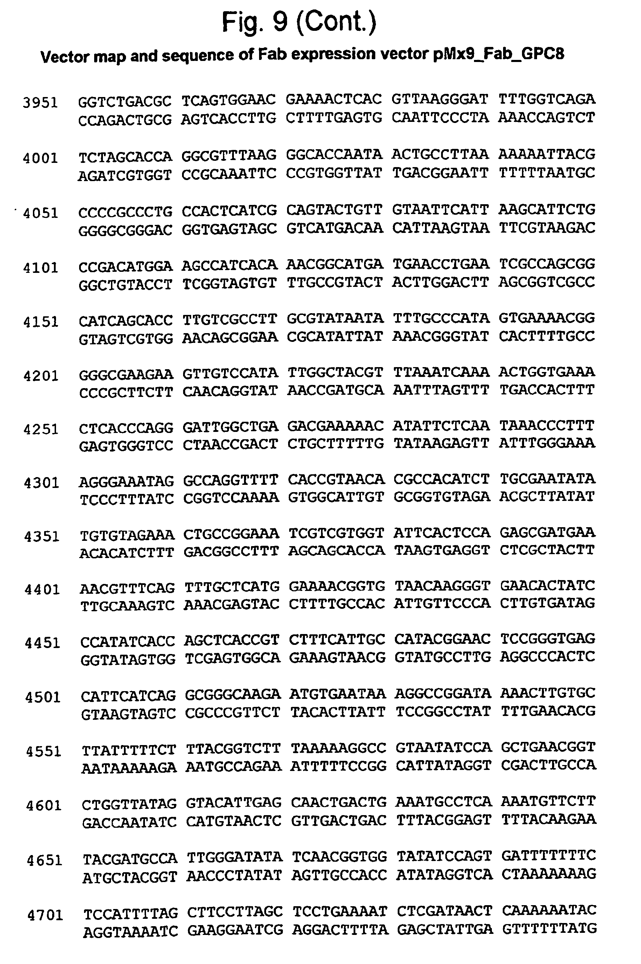

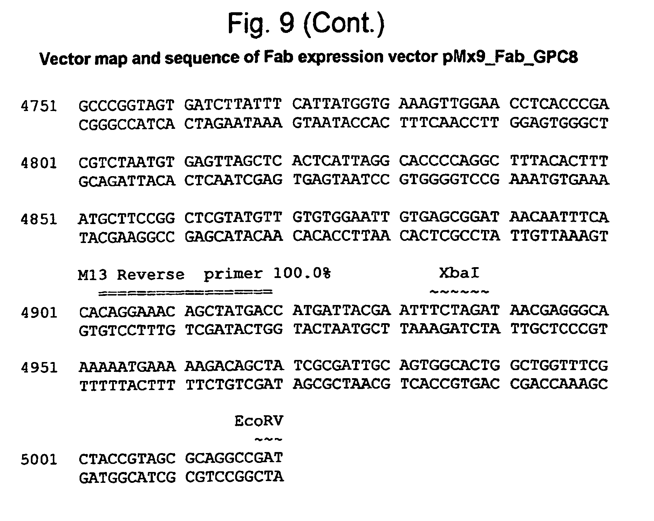

Vector map and sequence of Fab expression vector pMx9_Fab_GPC8.
The expression vector pMx9_Pab_GPC8 leads to the expression of HuCAL Fab fragments (in Fig. 9, the vector comprises the Fab fragment MS-GPC8) when VH-Ch1 is fused to a combination of a FLAG tag (Hopp et al., 1988; Knappik and Plückthun, 1994) and a STREP tag II (WSHPQFEK) (IBA GmbH, Göttingen, Germany; see: htttp://www.iba-go.de and Schmidt and Skerra, 1993; Schmidt and Skerra, 1994; Schmidt et al., 1996; Voss and Skerra, 1997).

### Figure 10

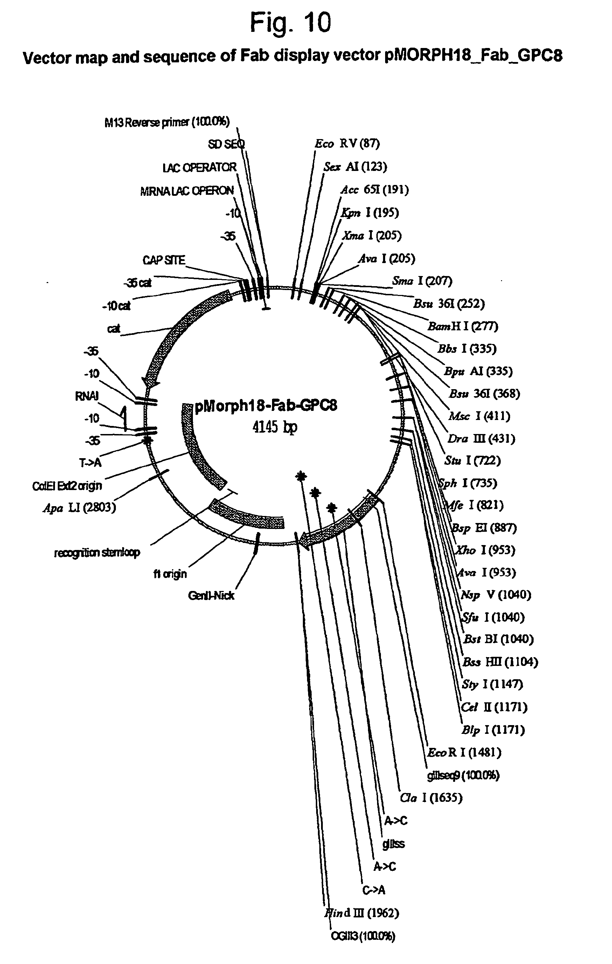

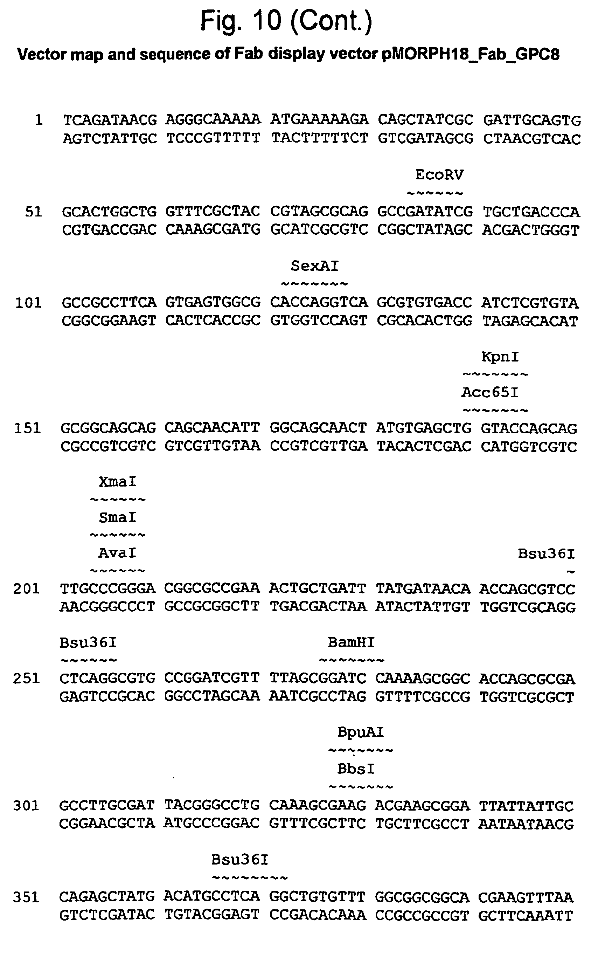

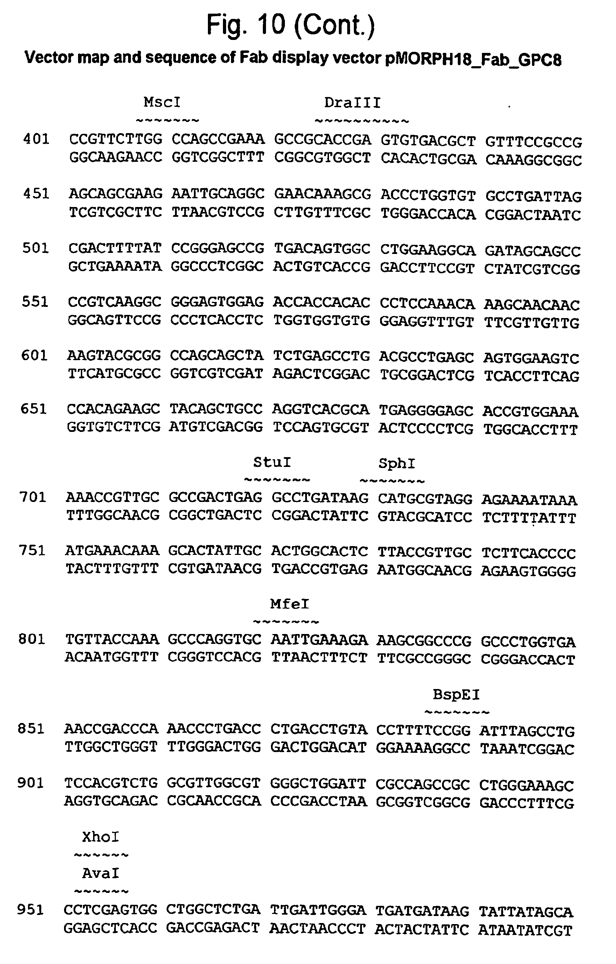

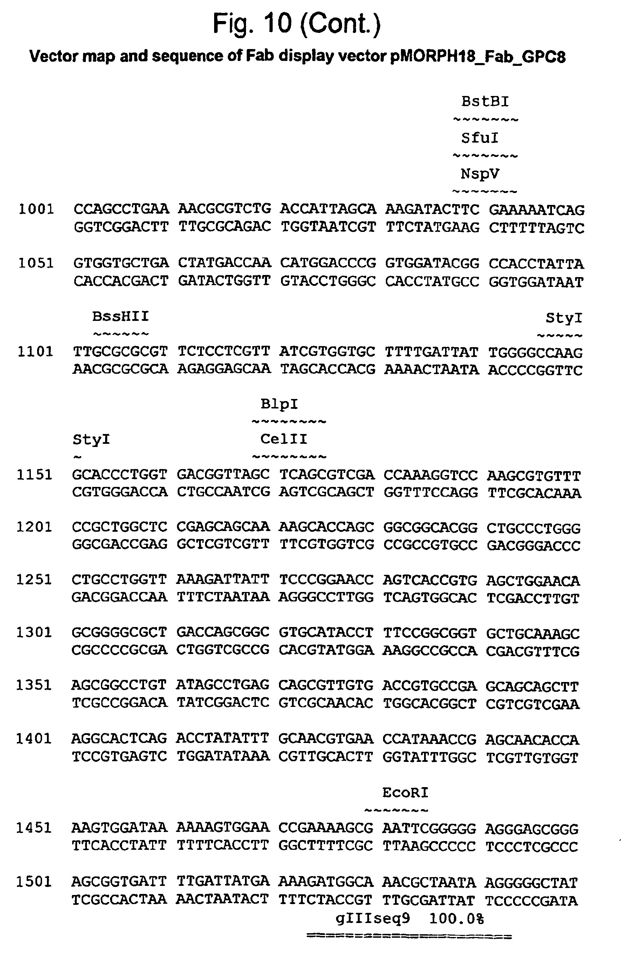

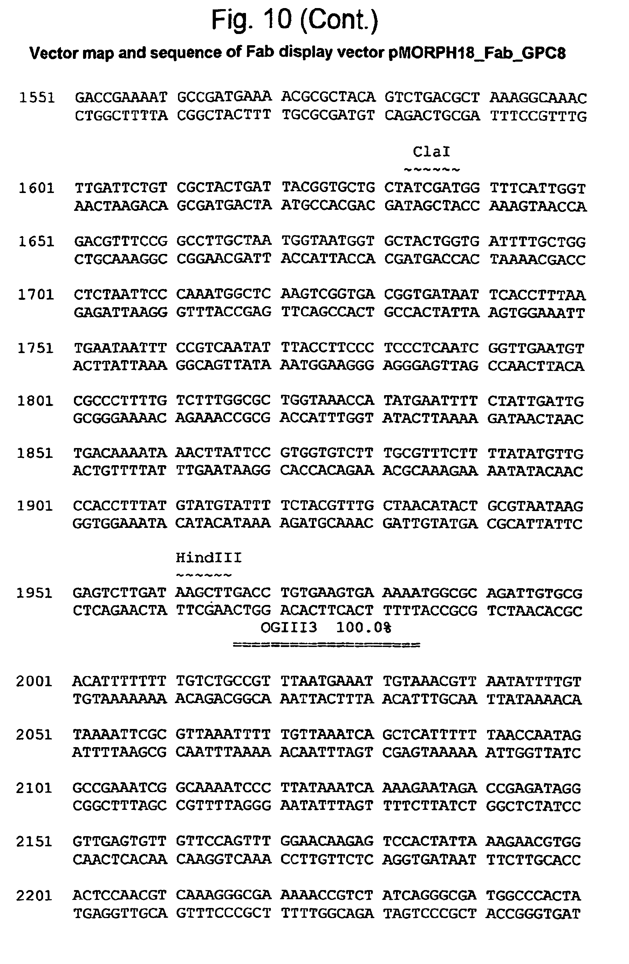

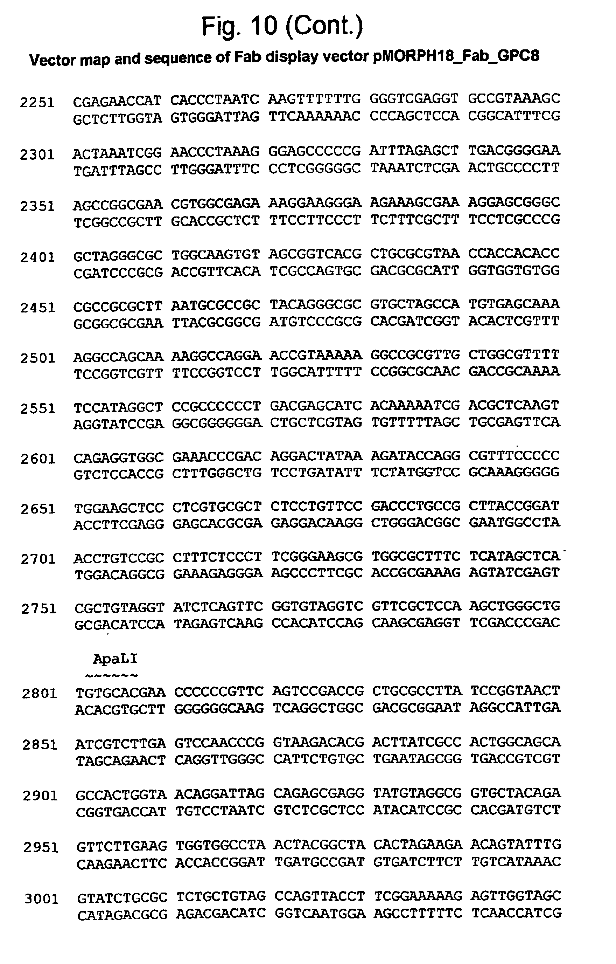

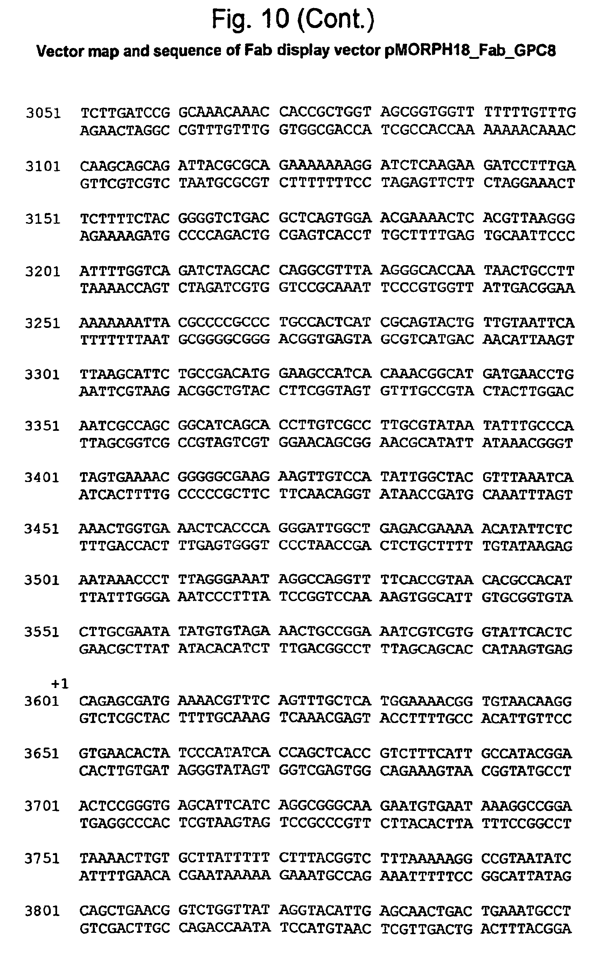

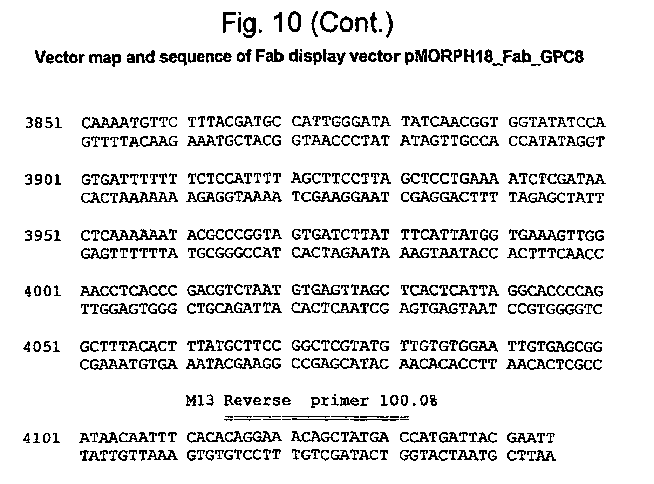

Vector map and sequence of Fab phage display vector pMORPH18_Fab_GPC8.
The derivatives of vector pMORPH18 are phagemid vectors comprising a gene encoding a fusion between the C-terminal domain of the gene III protein of filamentous phage and the VH-CH1 chain of a HuCAL antibody. Additionally, the vector comprises the separately encoded VL-CL chain. In Fig. 10, a vector comprising the Fab fragment MS-GPC-8 is shown.

### Figure 11

Amino acid sequences of VH and VL domains of MS-GPC-1, MS-GPC-6, MS-GPC-8, MS-GPC-10, MS-GPC-8-6, and MS-GPC-8-17.

The following examples illustrate the invention.

### Examples

(All buffers, solutions or procedures without explicit reference can be found in standard textbooks, for example Current Protocols of Immunology (1997 and 1999) or Sambrook et al., 1989.)

### A. Preparation of a human antigen

To demonstrate that we could identify cytotoxic antigen-binding domains of human composition, we first prepared a purified form of a human antigen, the human MHC class II DR protein (DRA*0101/DRB1*0401) from PRIESS cells (Gorga et al., 1984; Gorga et al., 1986; Gorga et al., 1987; Stem et al., 1992) as follows.

First, PRIESS cells (ECACC, Salisbury UK) were cultured in RPMI and 10% FCS using standard conditions, and 10¹⁰ cells were lysed in 200 ml PBS (pH 7.5) containing 1% NP-40, 25 mM iodoacetamide, 1 mM PMSF and 10 mg/l each of the protease inhibitors chymostatin, antipain, pepstatin A, soybean trypsin inhibitor and leupeptin. The lysate was centrifuged at 10.000 g (30 minutes, 4°C) and the resulting supernatant was supplemented with 40 ml of an aqueous solution containing 5% sodium deoxycholate, 5 mM iodoacetamide and 10 mg/l each of the above protease inhibitors and centrifuged at 100.000 g for two hours (4°C). To remove material that bound non-specifically and endogenous antibodies, the resulting supernatant was made 0.2 mM with PMSF and passed overnight (4°C) through a rabbit serum affigel-10 column (5 ml) followed by a Protein G Sepharose Fast Flow column (2 ml; Pharmacia) using a flow rate of 0.2 ml/min.

Second, the pre-treated lysate was batch incubated with 5 ml LB3.1-Protein G Sepharose Fast Flow beads (Stem et al., 1993) overnight at 4°C using gentle mixing, and then transferred into a small column which was then washed extensively with three solutions: (1) 100 ml of a solution consisting of 50 mM Tris/HCl (pH 8.0), 150 mM NaCl, 0.5% NP-40, 0.5% sodium deoxycholate, 10% glycerol and 0.03% sodium azide at a flow rate of 0.6 ml/min). (2) 25 ml of a solution consisting 50 mM Tris/HCl (pH 9.0), 0.5 M NaCl, 0.5 % NP-40, 0.5% sodium deoxycholate, 10% glycerol and 0.03% sodium azide at a flow rate of 0.9 ml/min; (3) 25 ml of a solution consisting of 2 mM Tris/HCl (pH 8.0), 1% octyl-β-D-glucopyranoside, 10% glycerol and 0.03% sodium azide at a flow rate of 0.9 ml/min.

Third, MHC class II DR protein (DRA*0101/DRB1*0401) was eluted using 15 ml of a solution consisting of 50 mM diethylamine/HCl (pH 11.5), 150 mM NaCl, 1 mM EDTA, 1 mM EGTA, 1% octyl-β-D-glucopyranoside, 10% glycerol, 10 mM iodoacetamide and 0.03% sodium azide at a flow rate of 0.4 ml/min. 800 µl fractions were immediately neutralised with 100 µl 1M Tris/HCl (pH 6.8), 150 mM NaCl and 1% octyl-β-D-glucopyranoside. The incubation of the lysate with LB3.1-Protein G Sepharose Fast Flow beads was repeated until the lysate was exhausted of MHC protein. Pure eluted fractions of the MHC class II DR protein (as analyzed by SDS-PAGE) were pooled and concentrated to 1.0-1.3 g/l using Vivaspin concentrators with a 30 kDa molecular weight cut-off. Approximately 1 mg of the MHC class II DR preparation was re-buffered with PBS containing 1% octyl-β-D-glucopyranoside using the same Vivaspin concentrator to enable direct coupling of the protein to BIAcore CM5 chips.

### B. Screening of HuCAL

### B.1. Introduction

We identified antigen binding antibody fragments of human composition against the human antigen (DRA*0101/DRB1*0401) from a human antibody library based on a novel concept that has been recently developed (Knappik et al., 2000). A consensus framework resulting in a total of 49 different frameworks here represents each of the VH- and VL-subfamilies frequently used in human immune responses. These master genes were designed to take into account and eliminate unfavorable residues promoting protein aggregation as well as to create unique restriction sites leading to modular composition of the genes. In HuCAL-scFv, both the VH- and VL-CDR3 encoding regions of the 49 master genes were randomized.

### B.2. Phagemid rescue, phage amplification and purification

The HuCAL-scFv (Knappik et al., 2000) library, cloned into a phagemid-based phage display vector pMORPH13_scFv (see Fig. 7), in *E.coli* TG-1 was amplified in 2 x TY medium containing 34 µg/ml chloramphenicol and 1% glucose (2 x TY-CG). After helper phage infection (VCSM13) at 37°C at an OD₆₀₀ of about 0.5, centrifugation and resuspension in 2 x TY / 34 µg/ml chloramphenicol / 50 µg/ml kanamycin / 0.1 mM IPTG, cells were grown overnight at 30°C. Phage were PEG-precipitated from the supernatant (Ausubel et al., 1998), resuspended in PBS/20% glycerol and stored at -80°C. Phage amplification between two panning rounds was conducted as follows: mid-log phase TG1-cells were infected with eluted phage and plated onto LB-agar supplemented with 1% of glucose and 34 µg/ml of chloramphenicol. After overnight incubation at 30°C colonies were scraped off, adjusted to an OD₆₀₀ of 0.5 and helper phage added as described above.

### B.3. Manual solid phase panning

Wells of MaxiSorp^{™} microtiterplates (Nunc) were coated with MHC- class II DRA*0101/DRB1*0401 (prepared as above) dissolved in PBS (2 µg/well). After blocking with 5% non-fat dried milk in PBS, 1-5 x 10¹² HuCAL-scFv phage purified as above were added for 1h at 20°C. After several washing steps, bound phages were eluted by pH-elution with 100 mM triethylamine and subsequent neutralization with 1 M TRIS-Cl pH 7.0. Three rounds of panning were performed with phage amplification conducted between each round as described above.

### B.4. Mixed solid phase/whole cell panning

Three rounds of panning and phage amplification were performed as described in B.3. and B.2. with the exception that in the second round between 1x10⁷ and 5x10⁷ PRIESS cells in 1 ml PBS/10% FCS were used in 10 ml Falcon tubes for whole cell panning. After incubation for 1h at 20°C with the phage preparation, the cell suspension was centrifuged (2000 rpm for 3 min) to remove non-binding phage, the cells were washed three times with 10 ml PBS, each time followed by centrifugation as described. Phage that specifically bound to the cells were eluted off by pH-elution using 100 mM HCI. Alternatively, binding phage could be amplified by directly adding E.coli to the suspension after triethlyamine treatment (100 mM) and subsequent neutralization.

### B.5 Identification of HLA-DR binding scFv fragments

Clones obtained after three rounds of solid phase panning (B.3) or mixed solid phase/whole cell panning (B.4) were screened by FACS analysis on PRIESS cells for binding to HLA-DR on the cell surface. For expression, the scFv fragments were cloned via XbaI/EcoRI into pMx7_FS as expression vector (see Fig. 8). Expression conditions are shown below in Example C.2.
Aliquots of 10⁶ Priess cells were transferred at 4°C into wells of a 96-well microtiterplate. ScFv in blocking buffer (PBS/5% FCS) were added for 60 min and detected with the using an anti-FLAG M2 antibody (Kodak) (1:5000 dilution) followed by a polyclonal goat anti-mouse IgG antibody-R-Phycoerythrin-conjugate (Jackson ImmunoResearch, 115-116-146, F(ab')₂ fragment) (1:200 dilution). Cells were fixed in 4% paraformaldehyde for storage at 4°C. 10⁴ events were collected for each assay on the FACS-Calibur (Becton Dickinson).
Only fifteen out of over 500 putative binders were identified which specifically bound to Priess cells. These clones were further analyzed for their killing activity as described below. Table 1 contains the sequence characteristics of clones MS-GPC-1, MS-GPC-6, MS-GPC-8 and MS-GPC-10 identified thereby. The VH and VL families and the CDR3s listed refer to the HuCAL consensus-based antibody genes as described (Knappik et al., 2000) the full sequences of the VH and VL domains are shown in Figure 11.

### C. Generation of Fab-fragments

### C.1. Conversion of scFv to Fab

Both heavy and light chain variable domains of scFv fragments were cloned into pMx9_Fab (Fig. 9), the heavy chain variable domains as MfeI / StyI-fragments, the variable domains of the kappa light chains as EcoRV/ BsiWI-fragments. The lambda chains were first amplified from the corresponding pMORPH13_scFv vector as template with PCR-primers CRT5 (5' primer) and CRT6 (3' primer), wherein CRT6 introduces a unique DraIII restriction endonuclease site.
CRT5: 5' GTGGTGGTTCCGATATC 3'
CRT6: 5' AGCGTCACACTCGGTGCGGCTTTCGGCTGGCCAAGAACGGGTTA 3'

The PCR product is cut with EcoRV / DraIII and cloned into pMx9_Fab (see Fig. 9). The Fab light chains could be detected with a polyclonal goat anti-human IgG antibody-R-Phycoerythrin-conjugate (Jackson ImmunoResearch, 109-116-088, F(ab')₂ fragment) (1:200 dilution).

### C.2. Expression and purification of HuCAL-antibody fragments in E.coli

Expression in E.coli cells (JM83) of scFv and Fab fragments from pMx7 _FS or pMx9_Fab, respectively, were carried out in one litre of 2 x TY-medium supplemented with 34 µg/ml chloramphenicol. After induction with 0.5 mM IPTG (scFv) or 0.1 mM IPTG (Fab), cells were grown at 22°C for 12 hours. Cell pellets were lysed in a French Press (Aminco) in 20 mM sodium phosphate, 0.5 M NaCl, and 10 mM imidazole (pH 7.4). Cell debris was removed by centrifugation and the clear supernatant filtered through 0.2 µm pores before subjecting it to STREP tag purification using a Streptactin matrix and purification conditions according to the supplier (IBA GmbH, Göttingen, Germany). Purification by size exclusion chromatography (SEC) was performed as described by Rheinnecker et al. (1996). The apparent molecular weights were determined by SEC with calibration standards and confirmed in some instances by coupled liquid chromatography-mass spectrometry (TopLab GmbH, Munich, Germany).

### D. HLA-DR specificity assay and epitope mapping

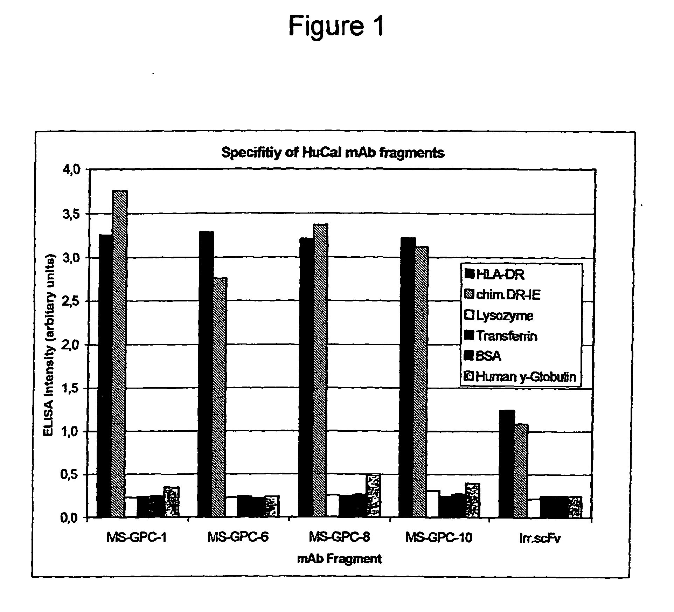

We conducted a binding-speaficity test to demonstrate that antigen-binding domains selected from the HuCAL library bound specifically to the human antigen. Using a standard ELISA procedure, scFv and Fab fragments selected from the HuCAL library were tested for reactivity with the following antigens: HLA-DR (DRA*0101/DRB1*0401), chimeric DR-IE (consisting of the N-terminal domains of DRA*0101 and DRB1*0401 with the remaining molecule derived from a murine class II homologue lEd. (Ito et al., 1996) and a set of negative control proteins comprising lysozyme, transferrin, BSA and human γ globulin. Figure 1 shows that all four anti-HLA DR antibody fragments (MS-GPC-1, 6, 8 & 10) demonstrated better specificity for HLA-DR proteins than for control proteins. A non-related antibody fragment (Irr.scFv) showed very little specific reactivity to the HLA DR proteins. Indeed, any reactivity observed was shown to result from contaminating IgG within the preparation of MHC class II molecules.

We reasoned that the specific anti-HLA DR antibody fragments that were selected from the HuCAL library were recognizing at least one epitope on the N-terminal extracellular protein of the human antigen. We conducted a series of experiments to confirm this hypothesis and to further define the epitope of binding for these anti-HLA DR antibody fragments.

First, the four anti-HLA DR antibody fragments (MS-GPC-1, 6, 8 & 10) were tested for reactivity against a panel of Epstein-Barr virus transformed B cell lines obtained from ECACC (Salisbury UK), each homozygous for one of the most frequent DR alleles in human populations, and to a series of L cells transfected to express human class II isotypes other than DRB1: L105.1. L257.6, L25.4, L256.12 & L21.3 that express the molecules DRB3*0101, DRB4*0101, DP0103/0402, DP 0202/0201, and DQ0201/0602 respectively (Klohe et al., 1988).

Reactivity of the antigen-binding fragment to the panel of cell-lines expressing various MHC- class II molecules was demonstrated using an immunofluorescence procedure as for example, described by Otten et al (1997). Staining was performed on 2x10⁵ cells using an anti-FLAG M2 antibody (Sigma) as the second reagent against the M2 tag carried by each anti-HLA DR antibody fragment and a fluorescein labeled goat anti-mouse lg (Pharmingen) as a staining reagent Cells were incubated at 4°C for 60 min with a predetermined dilution of the anti-HLA DR antibody fragment, followed by the second and third antibody at concentrations determined by the manufacturers. Cells were washed between incubation steps. Finally the cells were washed and subjected to analysis by a FACS Calibur (Becton-Dickinson).

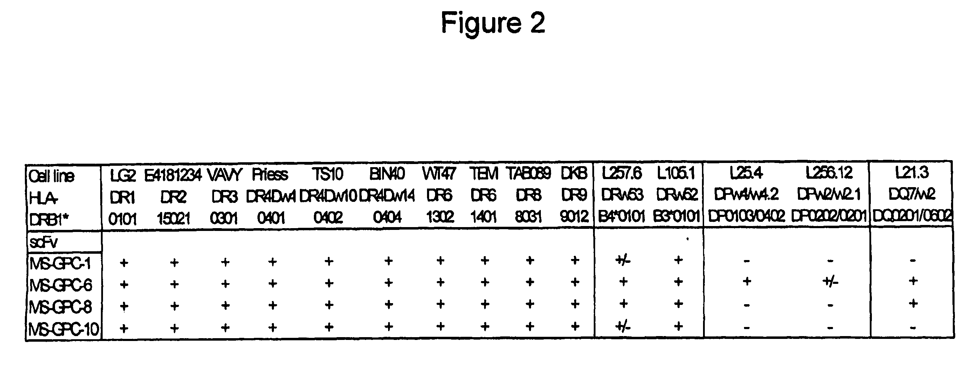

Figure 2 shows that the selected anti-HLA DR antibody fragments react with all DRB1 allotypes tested. This observation taken together with the observation that all anti-HLA DR antibody fragments react with chimeric DR-IE, suggests that all selected anti-HLA DR antibody fragments recognize the first domain of the monomorphic DRα chain or a monomorphic epitope on first domain of the DRβ chain.

Second, to further localize the epitope recognized by each anti-HLA DR antibody fragment, the fragment MS-GPC-8 was used as an example. MS-GPC-8 recognizes all DR molecules, the DQ molecule but not the two DP molecules tested (Figure 2). A sequence comparison of α chains of these molecules and the computer model of the three dimensional structure of DR molecules revealed that the epitope localizes in the C-terminal end of the α-helical region localized from Glu⁵⁵ to Tyr⁷⁹ of the N-terminal domain of the α-chain of HLA-DR.

Third, the PepSpot technique (US 6040423; Heiskanen et al., 1999) is used to create overlapping synthetic peptides corresponding to the sequence of the DRα1 and DRβ1 domains. Cross-reactivity tests between the MS-GPC-8 anti-HLA DR antibody fragment and this set of overlapping set of peptides localizes the epitope for this antibody fragment more precisely within the α-chain or β-chain of the HLA-DR molecule.

### E. Killing activity of mAb fragments

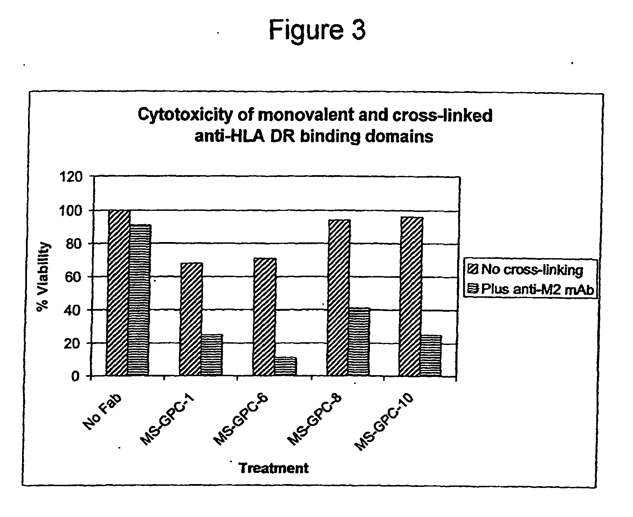

We demonstrated that a multivalent composition comprising of at least two anti-HLA DR antibody fragments caused killing of activated cells expressing the HLA-DR antigen. The killing efficiency of anti-HLA DR antibody fragments selected from the HuCAL library was tested on the HLA-DR positive tumor cell line GRANTA-519 (DSMZ, Germany). 2x10⁵ cells were incubated for 4 h at 37°C under 6% CO₂ with 200nM anti-HLA DR antibody fragments in RPMI 1640 (PAA, Germany) supplemented with 2,5% heat inactivated FBS (Biowhittaker Europe, Belgium), 2mM L-glutamine, 1% non-essential amino acids, 1mM sodium pyruvate and 0,1mg/ml kanamycin. Each anti-HLA DR antibody fragment was tested for its ability to kill activated tumor cells as a monovalent anti-HLA DR antibody fragment or as a bivalent composition by the addition to 100 nM of a bivalent cross-linking anti-FLAG M2 mAb (Sigma). After 4 h incubation at 37°C under 6% CO₂, cell viability was determined by trypan blue staining and subsequent counting of remaining viable cells (Current Protocols in Immunology, 1997).
Anti-HLA DR antibody fragments from the HuCAL library showed much higher cytotoxic activity when cross-linked to form a bivalent composition by co-incubation with anti-FLAG M2 mAb (Figure 3). Incubation of cell lines alone or only in the presence of anti-FLAG M2 mAb without co-incubation of anti-HLA DR antibody fragments did not lead to cytotoxicity as measured by cell viability. We observe that higher order valences of the anti-HLA DR antibody fragments further decrease cell viability significantly. On addition to the incubation mix of Protein G, the multivalent complexes thus formed comprising anti-HLA DR antibody fragments, further decrease cell viability compared to the bivalent composition formed from incubation of the anti-HLA DR antibody fragments with only anti-FLAG M2 mAb.

Similar experiments show that other tumor cell lines that express HLA-DR molecules were also killed using a cross-linked bivalent composition of the anti-HLA DR antibody Fab fragment MS-GPC8. Tumor cell lines that show greater than 50% cell killing after 4h incubation include MHH-CALL4, MN 60, BJAB, BONNA-12 which represent the diseases B cell acute lymphoid leukemia, B cell acute lymphoid leukemia, Burkitt lymphoma and hairy cell leukemia respectively.

Use of the Fab form of the anti-HLA DR antibody fragments MS-GPC-1, 6, 8 and 10 also shows similar cytotoxic activity to the above tumor cells lines when formed as a bivalent composition using the cross-linking anti-FLAG M2 mAb.

The method described above to assay killing activity was used to determine the maximum killing capacity for each of the cross-liked bivalent anti-MHC DR antibody fragments against Priess cells. The maximum killing capacity observed for MS-GPC-1, MS-GPC-6, MS-GPC-8 & MS-GPC-10 was measured as 83%, 88%, 84% and 88% respectively.

### F. Killing selectivity of antigen-binding domains against a human antigen for activated versus non-activated cells

Human peripheral B cells are used to demonstrate that human anti-HLA-DR mAb-mediated cell killing is dependent on cell-activation. Around 50 ml of heparinised venous blood is taken from an HLA-DR typed healthy donor and fresh peripheral blood mononuclear cells (PBMC) are isolated by Ficoll-Hypaque Gradient Centrifugation (Histopaque-1077; Sigma) as described in Current Protocols in Immunology (John Wiley & Sons, Inc.; 1999). Purified B cells (∼5% of peripheral blood leukocytes) are obtained from around 5x10⁷ PBMCs using the B cell isolation kit and MACS LS⁺/VS⁺ columns (Miltenyi Biotec, Germany) according to manufacturers guidelines. Successful depletion of non-B cells is verified by FACS analysis of an aliquot of isolated B cells (HLA-DR positive and CD45 positive). Double staining and analysis is done with commercially available antibodies (Beckton Dickinson) using standard procedures as for example described in Current Protocols in Immunology (John Wiley & Sons, Inc.; 1999). An aliquot of the isolated B cells is tested for the ability of the cells to be activated by stimulation with Pokeweed mitogen (PWM) (Sigma) at a concentration of 2,5µg/ml in RPMI 1640 (PAA, Germany) supplemented with 5% human AB serum (Sigma, Germany), 2mM L-glutamine, 1% non-essential amino acids, 1mM sodium pyruvate and 0,1 mg/ml kanamycin by incubation at 37°C under 6% CO₂ for four days. Successful activation is verified by standard procedures, for example morphology, by measuring ³H-thymidine uptake into the growing cells, or by FACS analysis of HLA-DR expression on the cell surface (Current Protocols in Immunology, John Wiley & Sons, Inc.; 1999).

The selectivity for killing of activated cells versus non-activated cells is demonstrated by incubating 1x10⁶/ml B cells activated as above compared to non-activated cells, respectively with 200nM of one the anti-HLA DR antibody fragments MS-GPC-1, 6, 8 & 10 together with 100nM of the cross-linking anti-FLAG M2 mAb in the medium described above but supplemented with 2,5% heat inactivated FBS (Biowhittaker Europe, Belgium) instead of human serum. After incubation at 37°C under 6% CO₂ for 1, 2, 4, and 6 h, cell viability is determined by fluorescein diacetate staining (FDA, Sigma) and subsequent counting of the green fluorescent cells using a fluorescence microscope (Leica, Germany) using standard procedures (Current Protocols in Immunology, 1997).

B cell activation is shown to be necessary for cell killing. After only 4 h following incubation with the cross-linked anti-HLA DR antibody fragments, over 50% of PWM activated B cells are killed. In contrast, non-activated B cells (not PWM stimulated) are not as susceptible to killing by the cross-linked anti-HLA DR antibody fragments - less than 15% dead cells can be seen after 4h.

We were surprised to see that our cross-linked anti-HLA-DR antibody fragments do not readily kill a particular tumor cell line. We hypothesized that although established as a stable cell line, cells in this culture were not sufficiently activated. Therefore, we conduct an experiment to stimulate activity of the MHH preB1 cell line, using increased cell-surface expression of HLA-DR molecule as a marker of activation as follows.

Non-adherently growing MHH preB1 cells are cultivated in RPMI medium containing the following additives (all from Gibco BRL and PAA): 10% fetal calf serum (FCS), 2 mM L-glutamine, 1% non-essential amino acids, 1 mM sodium pyruvate and 1x Gentamycin. Cells are activated to increase expression of HLA-DR molecule by incubation for two days with Lipopolysaccharide (LPS, Sigma, 10 µg/ml) and Interferon-gamma (IFN-γ, Roche, 40 ng/ml). The cell surface expression of HLA-DR molecules is monitored by flow cytometry with the FITC-conjugated mAb L243 (Becton Dickinson). Following activation, cell killing is performed for 4 h in the above medium but containing a reduced FCS concentration (2.5%) with a final antibody concentration of 200 nM.

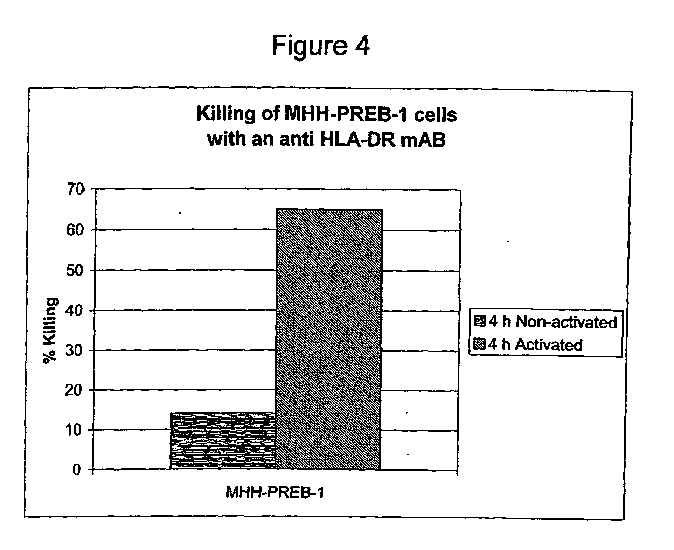

Incubation of MHH preB1 for two days in the presence of LPS and IFN- γ results in a 2-fold increase in HLA-DR surface density (mean fluorescence shift from 123 to 260). Accordingly, the percentage of dead cells after 4 h incubation with 100 nM concentration of the IgG form of MS-GPC-8 (produced as described below) is greater than 60% compared to a non-activated culture for which less than 15% dead cells are observed (Figure 4). Viable cells are identified microscopically by exclusion of Trypan blue. Therefore, activation of MHH preB1 cells by LPS and IFN-γ enhances the cell killing by binding of the IgG form of MS-GPC-8 to HLA DR molecules.

### G. Determination of IC50 for anti-HLA DR antibody fragments

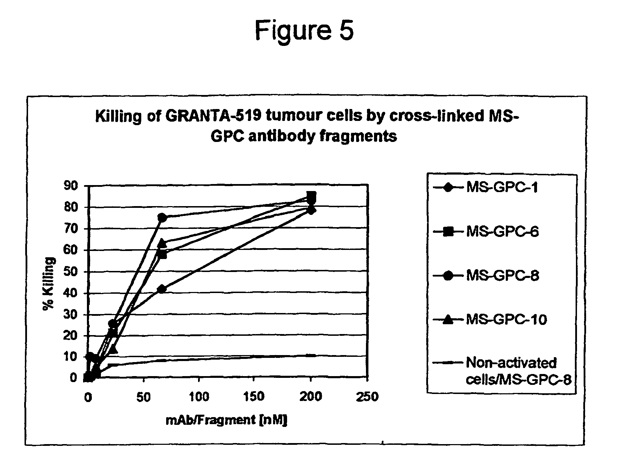

The IC50 for anti-HLA DR antibody fragments selected from the HuCAL library are estimated using the HLA-DR positive tumor cell line GRANTA-519 (DSMZ, Germany). 2x10⁵ cells are incubated for 4 h at 37°C under 6% CO₂ with a dilution series of anti-HLA DR antibody fragments and appropriate concentration of anti-FLAG M2 mAb to cause bivalent cross-linking in RPMI 1640 (PAA, Germany) supplemented with 2,5% heat inactivated FBS (Biowhittaker Europe, Belgium), 2mM L-glutamine, 1% non-essential amino acids, 1mM sodium pyruvate and 0,1mg/ml kanamycin. Appropriate final concentrations of the bivalent composition range from 2 to 200 nM. As a control, non-activated B cells are purified from around 50 ml of heparinised venous blood taken from an HLA-DR typed healthy donor as described above, and similarly treated using a dilution series of the bivalent mAb composition MS-GPC-8/anti-FLAG M2 mAb. After 4 h incubation at 37°C under 6% CO₂, cell viability was determined by fluorescein diacetate staining and subsequent counting of remaining viable cells (Current Protocols in Immunology, 1997). The concentration of bivalent mAb composition that causes 50% cell killing for the activated tumor cells is less than 100 nM. In contrast, 50% killing for the non-activated cells is not seen after 4 h incubation using this range of concentrations for the bivalent mAb composition, which was maximally 200 nM (Figure 5).

The method described above used to determine the IC50 for each of the cross-linked scFv anti-MHC DR antibody fragments. The IC50 estimates made for MS-GPC-1, MS-GPC-6, MS-GPC-8 & MS-GPC-10 was measured as 100, 55, 20-40 and 55 nM respectively - where each IC50 concentration represents the concentration of the resulting bivalent composition.

### H. Mechanism of cell-killing

The examples described above show that cell death occurs - needing only certain multivalent anti-HLA DR antibody fragments to cause killing of activated cells. No further cytotoxic entities or immunological mechanisms were needed to cause cell death, therefore demonstrating that cell death is mediated through an innate pre-programmed mechanism of the activated cell. The mechanism of apoptosis is a widely understood process of pre-programmed cell death. We were surprised by certain characteristics of the cell killing we observed that suggested the mechanism of killing for activated cells when exposed to our human anti-HLA DR antibody fragments was not apoptosis. For example, the speed at which we observed cells were killed appeared to be significantly faster than that reported for apoptosis. Two experiments are conducted to demonstrate that the mechanism of cell killing proceeds by a non-apoptotic mechanism.

First, we use Annexin-V-FITC and propidium iodide (PI) staining techniques to distinguish between apoptotic and non-apoptotic cell death - apoptotic cells (Annexin-V positive/PI negative) can be distinguished from dead (Annexin-V positive/PI positive) and fully functional cells (Annexin-V negative/PI negative) (using the manufactures recommended procedure). 1x10⁶/ml Priess cells are incubated at 37°C under 6%CO₂ with or without 200nM anti-HLA DR antibody fragment MS-GPC-8 together with 100 nM of the cross-linking anti-FLAG M2 mAb in RPMI 1640 (PAA, Germany) supplemented with 2,5% heat inactivated FBS (Biowhittaker Europe, Belgium), 2mM L-glutamine, 1% non-essential amino acids, 1 mM sodium pyruvate and 0,1mg/ml kanamycin. To provide an apoptotic cell culture as control, 1x10⁶/ml Priess cells were induced to enter apoptosis by incubation in the above medium at 37°C under 6%CO₂ with 50µg/ml of the apoptosis-inducing anti-CD95 mAb DX2 (Pharmingen, California) cross-linked with 10µg/ml Protein-G (Sigma, Germany). At various incubation times (1, 15 and 60 min, 3 and 5 h) 200 µl samples are taken, washed twice and stained with Annexin-V-FITC (Pharmingen, California) and PI (Sigma, Germany) using Annexin-V binding buffer following the manufacturer's protocol. The amount of staining with Annexin-V-FITC and PI for each group of cells is analysed with a FACS Calibur (Beckton Dickinson, Germany).

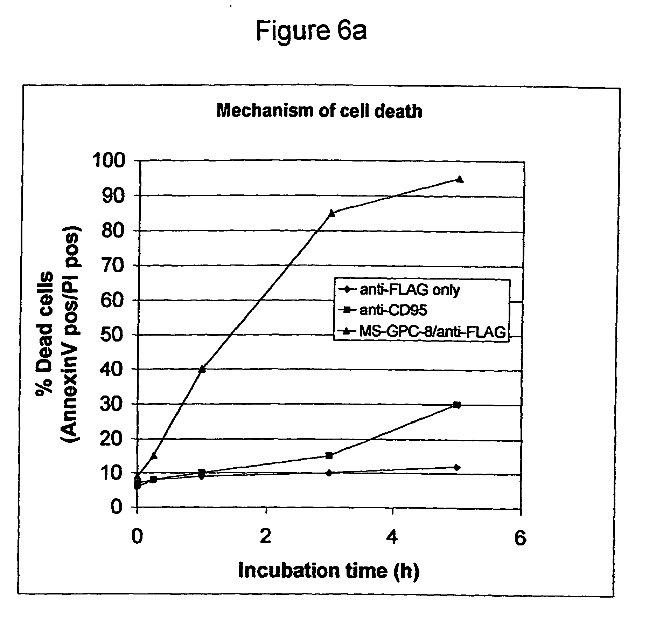

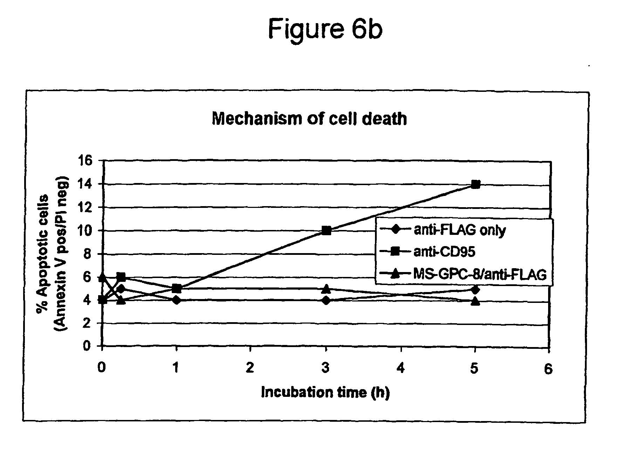

Cell death induced through the cross-linked anti-HLA DR antibody fragments shows a significantly different pattern of cell death than that of the anti-CD95 apoptosis inducing antibody or the cell culture incubated with anti-FLAG M2 mAb alone. The percentage of dead cells (as measured by Annexin-V positive/PI positive staining) for the anti-HLA DR antibody fragment/anti-FLAG M2 mAb treated cells increases far more rapidly than that of the anti-CD95 or the control cells (Figure 6a). In contrast, the percentage of apoptotic cells (as measured by Annexin-V positive/PI negative staining) increases more rapidly for the anti-CD95 treated cells compared to the aoss-linked anti-HLA DR antibody fragments or the control cells (Figure 6b).

Second, we inhibit Caspase activity using zDEVD-fmk, an irreversible Caspase-3 inhibitor, and zVAD-fmk a broad spectrum Caspase inhibitor (both obtained from Bio-Rad). The mechanism of apoptosis is characterized by Caspase activity, and we hypothesized that if Caspases were not necessary for anti HLA-DR mediated cell death, we would observe no change in the viability of cells undergoing cell death in the presence of these Caspase inhibitors compared to those without. 2x10⁵ Priess cells are preincubated for 3 h at 37°C under 6% CO₂ with serial dilutions of the two caspase inhibitors ranging from 180 µM to 10 mM in RPMI 1640 (PAA, Germany) supplemented with 2,5% heat inactivated FBS (Biowhittaker Europe, Belgium), 2mM L-glutamine, 1% non-essential amino acids, 1mM sodium pyruvate and 0,1mg/ml kanamycin. HLA-DR mediated cell death is induced by adding 200nM of the human anti-HLA DR antibody fragment MS-GPC-8 and 100nM of the cross-linking anti-M2 mAb. An anti-CD95 induced apoptotic cell culture serves as a control for the activity of inhibitors (Drenou et al., 1999). After further incubation at 37°C and 6% CO₂, cell viability after 4 and 24 h is determined by trypan blue staining and subsequent counting of non-stained cells. As we hypothesized, cell viability of the anti-HLA DR treated cell culture is not significantly modified by the presence of the Caspase inhibitors, while cell death induced through anti-CD95 treatment is significantly decreased for the cell culture pre-incubated with the Caspase inhibitors. This observation supports our hypothesis that HLA-DR mediated cell death proceeds through a non-apoptotic mechanism that is independent of Caspase proteases.

### I. Antibody optimization

In order to optimize certain biological characteristics of the HLA-DR binding antibody fragments, one of the Fab fragments, MS-GPC8-Fab, was used to construct a library of Fab antibody fragments by replacing the parental VL λ1 chain by the pool of all lambda chains λ 1-3 randomized in CDR3 from the HuCAL library (Knappik et al., 2000).

The Fab fragment MS-GPC-8-Fab (see C.1) was cloned via XbaI/EcoRI from pMx9_Fab_GPC8 into pMORPH18_Fab, a phagemid-based vector for phage display of Fab fragments, to generate pMORPH18_Fab_GPC8 (see Fig. 10). The lambda chain pool was amplified from HuCAL-scFv in pMORPH13_scFv (see B.2 above and Figure 7) with PCR-primers CRT5 and CRT6 (which introduces a unique DraIII restriction endonuclease site) and cloned into pMORPH18_Fab_GPC8 cut with NsiI and DraIII (see vector map of pMORPH18_Fab_GPC8 in Fig. 10).

The resulting Fab optimization library was screened by two rounds of panning against MHC- class II DRA*0101/DRB1*0401 (prepared as above) as described in B.3 with the exception that in the second round the antigen concentration for coating was decreased to 12 ng/well). FACS identified optimized clones as described above in B.5. Two of these clones, MS-GPC-8-6 and MS-GPC-8-17, were further characterized and showed cell killing activity as found for the starting fragment MS-GPC-8. Table 1 contains the sequence characteristics of MS-GPC-8-6 and MS-GPC-8-17. The VH and VL families and the CDR3s listed refer to the HuCAL consensus-based antibody genes as described (Knappik et al., 2000), the full sequences of the VH and VL domains are shown in Figure 11.

The optimized Fab forms of the anti-HLA DR antibody fragments MS-GPC-8-6 and MS-GPC-8-17 showed improved characteristics over the starting MS-GPC-8. For example, the IC50 of the optimized antibodies was 15-20 and 5-15 nM (compared to 20-40nM for MS-GPC-8), and the maximum killing capacity of MHH-Call 4 cells determined as 76 and 78% for MS-GPC-8-6 and MS-GPC-8-17 (compared to 65% for MS-GPC-8) respectively.

### J. Generation of IgG

### J.1. Construction of HuCAL-immunoglobulin expression vectors

### a) Heavy chain cloning

The multiple cloning site of pcDNA3.1+ (Invitrogen) was removed (NheI / ApaI), and a stuffer compatible with the restriction sites used for HuCAL-design was inserted for the ligation of the leader sequences (NheI / EcoRI), VH-domains (EcoRI / BlpI) and the immunoglobulin constant regions (BlpI / ApaI). The leader sequence (EMBL M83133) was equipped with a Kozak sequence (Kozak, 1987). The constant regions of human IgG₁ (PIR J00228), IgG₄ (EMBL K01316) and serum IgA₁ (EMBL J00220) were dissected into overlapping oligonucleotides with lengths of about 70 bases. Silent mutations were introduced to remove restriction sites non-compatible with the HuCAL-design. The oligonucleotides were spliced by overlap extension-PCR.

### b) Light chain cloning

The multiple cloning site of pcDNA3.1/Zeo+ (Invitrogen) was replaced by two different stuffers. The κ-stuffer provided restriction sites for insertion of a κ-leader (NheI / EcoRV), HuCAL-scFv Vκ-domains (EcoRV / BsiWI) and the κ-chain constant region (BsiWI / ApaI). The corresponding restriction sites in the λ-stuffer were NheI / EcoRV (λ-leader), EcoRV / HpaI (Vλ- domains) and HpaI / ApaI (λ-chain constant region). The κ-leader (EMBL Z00022) as well as the λ-leader (EMBL L27692) were both equipped with Kozak sequences. The constant regions of the human x- (EMBL J00241) and A -chain (EMBL M18645) were assembled by overlap extension-PCR as described above.

### J.2. Generation of IgG-expressing CHO-cells

All cells were maintained at 37°C in a humidified atmosphere with 5% CO₂ in media recommended by the supplier. CHO-K1 (CRL-9618) were from ATCC and were co-transfected with an equimolar mixture of IgG heavy and light chain expression vectors. Double-resistant transfectants were selected with 600 µg/ml G418 and 300 µg/ml Zeocin (Invitrogen) followed by limiting dilution. The supernatant of single clones was assessed for IgG expression by capture-ELISA (see below). Positive clones were expanded in RPMI-1640 medium supplemented with 10% ultra-low IgG-FCS (Life Technologies). After adjusting the pH of the supernatant to 8.0 and sterile filtration, the solution was subjected to standard protein A column chromatography (Poros 20A, PE Biosystems).

The IgG forms of anti-HLA DR antigen binding domains show improved characteristics over the antibody fragments. For example, the IC50 value of the IgG form of MS-GPC-8 was 10 nM and the maximum killing capacity determined as 84% for MHH-Call4 and 89% for GRANTA 519. Note that all IC50 concentrations given (also for Fab and scFv fragments) are shown as the molar equivalent of the bivalent IgG to simplify comparison.

### K. In vivo therapy for cancer using an HLA-DR specific antibody

We demonstrate that antigen-binding domains of human composition can successfully be used as a therapeutic for the treatment of cancer. Immunocompromised mice - such as scid, nude or Rag-1 knockout - are inoculated with a DR+ human lymphoma or leukemia cell line(s) of interest The tumor cell dose, usually 1x10⁴ to 1x10⁶/mouse, is established for each tumor tested and administered subcutaniously (s.c.) or intraveniously (i.v.). A period of 2 to 7 days is then required for the tumor to establish itself in the mice where the exact length of this period is established for each given tumor at its particular dose. Once the tumor is established, mice are treated i.v. or s.c with the IgG form of the anti-HLA DR antibody fragment MS-GPC-8 prepared as described above, using doses of 1 to 5 mg/kg over 1 to 5 days. Survival of anti-HLA DR treated and control untreated mice is monitored for up to 8 weeks after cessation of treatment. Tumor progression in the mice inoculated s.c. is additionally quantified by measuring tumor surface area. Significant prolongation of survival of up to 80% of anti-HLA DR treated mice is observed during the experiment, and up to 50% mice survive at the end of the experiment. In s.c. inoculated and untreated mice, the tumor reaches a surface area of 2-3 cm², while in anti-HLA DR treated animals the tumor surface area is significantly less.

### L Immunosuppression using an HLA-DR specific antibody measured by T cell proliferation

The IgG and monovalent forms of the anti-HLA DR antibody fragment MS-GPC-8 prepared as described above can be assayed for their ability to inhibit the proliferative T cell response of antigen-primed lymph node cells from mice carrying a chimeric mouse-human class II transgene with an RA-associated peptide binding site, and lack murine class II molecules (Muller et al., 1990; Woods et al., 1994; Current Protocols in Immunology, Vol. 2, 7.21; Ito et al., 1996). Here, the immunization takes place *in vivo,* but the inhibition and readout are *ex vivo.* Transgenic mice expressing MHC class II molecules with binding sites of two RA associate molecules, DRB1*0401 and 0404 were previously generated (Ito et al 1996). The mice lack murine MHC class II, and thus, all Th responses are channeled through a single human RA-associated MHC class II molecule (Ito et al. 1996). These transgenic mice represent a model for testing human class II antagonist.

The inhibitory effect of the anti-HLA DR antibody fragment MS-GPC-8 and its IgG form on T-cell proliferation is measured using chimeric T-cells and antigen presenting cells isolated from the lymph nodes of chimeric 0401-IE and 0404-IE transgenic mice previously immunized with hen egg lysozyme and ovalbumin (Ito et al. 1996), respectively. 1.5x10⁵ cells are incubated in 0.2 ml wells of 96-well tissue culture plates in the presence of antigen (half-maximal stimulatory concentration) and either the anti-HLA DR antibody fragment MS-GPC-8 or its IgG form (1 nM - 200 nM) in serum free HL-1 medium containing 2 mM L-glutamine and 0.1 g/l Kanamycin for three days. Antigen specific proliferation is measured by ³H-methyl-thymidin incorporation during the last 16h of culture (Falcioni et al., 1999). Inhibition of T-cell proliferation on treatment with the anti-HLA DR antibody fragment and its IgG form may be observed by comparison to control wells containing antigen.

### M. Selection of useful peptide/protein for the treatment of cancers

In order to select the most appropriate protein/peptide to enter further experiments to assess its suitability for use in a therapeutic composition for the treatment of cancers, additional data is collected. Such data for each IgG form of the anti-HLA antigen antibody fragments can include the *in vitro* killing efficiency as measured by IC50, the maximal percentage cell killing as estimated *in vitro,* and tumor reduction data and mouse survival data from *in vivo* animal models.

The IgG for of the anti-HLA antigen antibody fragments that shows the lowest IC50 for killing, the highest maximal percentage cell killing, the best tumor reduction data and/or the best mouse-survival data is chosen to enter further experiments. Such experiments may include, for example, phase I clinical trials in humans.

**Table 1**

| **VH and VL families and CDR 3s of HLA-DR-specific peptides/proteins** | | | | | | | |
|---|---|---|---|---|---|---|---|
| **Name** | VH | CDR3 Length | **VH-CDR3-Seq,** | VL | CDR3 Length | **VL-CDR3-Seq.** | Families |
| | | | | | | | |
| **MS-GPC1** | H2 | 10 | **QYGHRGGFDH** | λ1 | 8 | **QSYDFNES** | H2 λ 1 |
| **MS-GPC6** | H3 | 9 | **GYGRYSPDL** | K3 | 8 | **QQYSNLPF** | H3 K 3 |
| **MS-GPC8** | H2 | 10 | **SPRYRGAFDY** | λ 1 | 8 | **QSYDMPQA** | H2 λ 1 |
| **MS-GPC10** | H2 | 10 | **QLHYRGGFDL** | λ 1 | 8 | **QSYDLTMG** | H2 λ 1 |
| | | | | | | | |
| **MS-GPC8-6** | H2 | 10 | **SPRYRGAFDY** | λ 1 | 8 | **QSYDYDHY** | H2 A 1 |
| **MS-GPC8-17** | H2 | 10 | **SPRYRGAFDY** | λ 1 | 8 | **QSYDFSVY** | H2 λ 1 |

### References

Adorini L, Mueller S, Cardinaux F, Lehmann PV, Falcioni F, Nagy ZA, (1988), Nature 334: 623.
Ausubel, F.M., Brent, R., Kingston, R.E., Moore, D.D., Seidman, J.G., Smith, J.A. and Struhl, K. (1998) Current protocols in molecular biology. John Wiley & Sons, Inc., New York, U.S.A.
Babbitt B, Allen PM, Matsueda G, Habe E, Unanue ER, (1985), Nature 317:359.
Baxevanis, C.N., Wernet, D., Nagy, Z.A., Maurer, P.H., and Klein, J. (1980). Immunogenetics, 11, 617.
Billing, R., and Chatterjee, S. (1983). Transplant. Proc. 15,649.
Bird, R.E. et al. Single-chain antigen-binding proteins [published erratum appears in Science 1989 Apr 28;244(4903):409]. Science 242, 423-6 (1988).
Bonagura, V.R., Ma, a., McDowell, J., Lewison, A., King, D.W. and Suciu-Foca, N. (1987). Cell. Immunolo., 108(2), 356.
Brinkmann, U., Reiter, Y., Jung, S., Lee, B. & Pastan, I. (1993). A recombinant immunotoxin containing a disulfide-stabilized Fv fragment. Proc. Natl. Acad. Sci. U.S.A. 90, 7538-7542.
Brown JH, Jardetsky TS, Gorga JC, Stem LJ, Urban RG, Strominger JL, Wiley DC., (1993), Nature 364: 33.
Cambier JC, Morrison DC, Chien MM, Lehmann KR: J., (1991), Immunol. 146: 2075.
Current Protocols in Immunology, Vol. 2, 7.21 (1997).
Current Protocols in Immunology (John Wiley & Sons, Inc.; 1999).
Drenou B, Blancheteau V, Burgess DH, Fauchet R, Charron DJ, Mooney NA., (1999), J. Immunol. 163: 4115.
Falcioni et al. (1999). Nat Biotechnol. 17: 562-567.
Glockshuber, R., Malia, M., Pfitzinger, I. & Plückthun, A. (1990). A comparison of strategies to stabilize immunoglobulin Fv-fragments. Biochemistry 29, 1362-1367.
Gorga J.C., Foran, J., Burakoff, S.J., Strominger, J.L., (1984) Meth Emzym., 108,607-613.
Gorga, J.C., Horejsi, V., Johnson, D.R., Raghupathy, R., Strominger, J.L., J.Biol. Chem. 262 (1987)16087-94.
Gorga, J.C., Knudsen, P.J., Foran, J.A., Strominger, J.L., Burakoff, S.J., (1986), Cell. Immunol. 103 160-73.
Heiskanen T, Lundkvist A, Soliymani R, Koivunen E, Vaheri A, Lankinen H (1999) Virology, 262(2), 321.
Hopp, T.P., Prickett, K.S., Price, V.L., Libby, R.T., March, C.J., Cerretti, D.P., Urdal, D.L. & Conlon, P.J. (1988), Bio/Technology 6, 1204-1210.
Huston, J.S. et al. Protein engineering of antibody binding sites: recovery of specific activity in an anti-digoxin single-chain Fv analogue produced in Escherichia coli. Proc Natl Acad Sci U S A 85, 5879-83 (1988).
Ito K, Bian H.-J, Molina M, Han J, Magram J, Saar E, Belunis C, Bolin DR, Arceo R, Campbell R, Falcioni F, Vidovic' D, Nagy ZA., (1996), J. Exp. Med. 183: 2635-2644.
Jones et al., (1986), Nature 321: 522-525.
Jonker, M., Schellekens, P.T., Harpprecht, J., and Slingerland, W. (1991), Transplant. Proc., 23, 264.
Jonker, M., van Lambalgen, R., Mitchell, D.J., Durham, S.K., and Steinman, L. (1988), Autoimmunity, 1, 399.
Kabelitz D, Janssen O., (1989), Cell. Immunol. 120: 21.
King, D.J., Turner, A., Farnsworth, A.P.H., Adair, J.R., Owens, R.J., Pedley, R.B., Baldock, D., Proudfoot, K.A., Lawson, A.D.G., Beeley, N.R.A., Millar, K., Millican, T.A., Boyce, B.A., Antoniw, P., Mountain, A., Begent, R.H.J., Shochat, D. and Yarranton, G.T., (1994), Cancer Res. 54, 6176.
Klohe EP, Watts R, Bahl M, Alber C, Yu W-Y, Anderson R, Silver J, Gregersen PK, Karr RK., (1988), J. Immunol. 141: 2158-2164.
Knappik, A. & Plückthun, A., (1994), Biotechniques 17, 754-761.
Knappik, A., Ge, L., Honegger, A., Pack, P., Fischer, M., Wellnhofer, G., Hoess A., Wölle, J., Plückthun, A. and Virnekäs, B., (2000), J. Mol. Biol. 296, 55.
Kahoury E.L. and Marshall L.A., (1990) Cell. Tissue Res., 262(2):217-24
Kozak, M. (1987) J. Mol. Biol. 196, 947.
Kuby, J. Immunology:1994, 2nd edition.
Mourad W, Geha RS, Chatila TJ., (1990), J. Exp. Med. 172: 1513.
Muller et al., (1990), J. Immunol., 145: 4006.
Nabavi N, Freeman GJ, Gault A, Godfrey D, Nadler LM, Glimcher LH., (1992) Nature 360: 266.
Naquet, P., Marchetto, S., and Pierres, M., (1983), Immunogenetics, 18, 559.
Newell MK, VanderWall J, Beard KS, Freed JH., (1993), Proc. Natl. Acad. Sci. USA 90: 10459.
Otten et al (1997) pp 5.4.1 - 5.4.19 in Current Protocols in Immunology, Eds. Coligan et al. Green & Wiley, New York.
Pack, P. and Plückthun, A., (1992), Biochemistry 31, 1579-1584.
Pack, P., (1994), Ph.D. thesis, Ludwig-Maximilians-Universität München.
Pack, P., Kujau, M., Schroeckh, V., Knüpfer, U., Wenderoth, R., Riesenberg D. and Plückthun, A. (1993), Bio/Technology 11, 1271-1277.
Palacios R, Martinez-Maza O, Guy K., (1983), Proc. Natl. Acad. Sci. USA 80: 3456.
Palacios R., (1985), Proc. Natl. Acad. Sci. USA 82: 6652.
Presta, (1992), Curr. Op. Struct. Biol. 2: 593-596.
Riechmann et al., (1988), Nature 332: 323-329.
Rheinnecker, M., Hardt, C., IIag, L.L., Kufer, P., Gruber, R., Hoess, A., Lupas, A., Rottenberger, C., Plückthun, A. and Pack, P., (1996), J. Immunol. 157, 2989.
Rosenbaum JT, Adelman NE, McDevitt HO., (1981), J. Exp. Med. 154:1694.
Sambrook et al., 1989, Molecular Cloning: a Laboratory Manual, 2nd ed.
Schmidt, T. G. M. & Skerra, A. (1993). Prot. Engineering 6, 109-122.
Schmidt, T. G. M. & Skerra, A. (1994). J. Chromatogr. A 676, 337-345.
Schmidt, T. G. M. et al. (1996). J. Mol. Biol. 255, 753-766.
Skerra, A. and Plückthun, A. (1988). Science 240, 1038.
Smith, R.M., Morgan, A., and Wraith, D.C. (1994). Immunology, 83, 1.
Stausbol-Grøn, B., Wind, T., Kjær, S., Kahns, L., Hansen, N.J.V., Kristensen, P. and Clark, B.F.C. (1996) FEBS Lett. 391, 71.
Stem J.L. and Wiley, D.C., (1992), Cell 68 465-477.
Stem, A.S: and Podlaski, F.J, (1993) Techniques in Protein Chemistry IV, Academic Press Inc., San Diego, CA.
Stevens, H.P., Roche, N., Hovius, S.E., and Jonker, M., (1990), Transplant. Proc., 22, 1783.
Truman J-P, Choqueux C, Tschopp J, Vedrenne J, Le Deist F, Charron D, Mooney N., (1997), Blood 89:1996.
Truman J-P, Ericson ML, Choqueux-Seebold JM, Charron DJ, Mooney NA., (1994), Internatl. Immunol. 6: 887.
Vaickus L, Jones VE, Morton CL, Whitford K, Bacon RN., (1989), Cell. Immunol. 119: 445.
Vidovic D, Falcioni F, Bolin DR, Nagy ZA., (1995a), Eur. J. Immunol., 25: 1326.
Vidovic D, Falcioni F, Siklodi B, Belunis CJ, Bolin DR, Ito K, Nagy ZA., (1995b), Eur J. Immunol., 25:3349.
Voss, S. & Skerra, A. (1997). Protein Eng. 10, 975-982.
Waldor, M.K., Sriram, S., McDevitt, H.O., and Steinman, L. (1983). Proc. Natl. Acad. Sci. USA, 80, 2713.
Winter, G., Griffiths, A.D., Hawkins, R.E. and Hoogenboom, H.R. (1994) Making antibodies by phage display technology. Annu. Rev. Immunol. 12, 433.
Woods et al., (1994), J Exp Med. 180: 173-81.

## Claims

1. A peptide/protein comprising at least one antibody-based antigen-binding domain of human composition with binding specificity for an antigen expressed on the surface of a human cell, wherein binding of a multivalent composition of one or more of said peptide/protein to said antigen expressed on the surface of a cell causes or leads to killing of said cell, wherein said antigen-binding domain consists of a combination of a VH domain and a VL domain, wherein said combination is found in one of the clones taken from the list of MS-GPC-1, MS-GPC-6, MS-GPC-8, MS-GPC-10, MS-GPC-8-6, and MS-GPC-8-17 as shown in Table 1 and Figure 11.

2. A peptide/protein comprising at least one antibody-based antigen-binding domain of human composition with binding specificity for an antigen expressed on the surface of a human cell, wherein said antigen comprises epitopes selected from HLA-DR molecules, wherein binding of a multivalent composition of one or more of said peptide/protein to said antigen expressed on the surface of an activated cell causes or leads to killing of said activated cell in the absence of further cytotoxic entities or immunological mechanisms, wherein said antigen-binding domain consists of a combination of HuCAL VH2 and HuCAL Vλ1, wherein the VH CDR3 sequence is taken from the consensus CDR3 sequence
nnnnRGnFDn
wherein each n independently represents any amino acid residue; and wherein the VL CDR3 sequence is taken from the consensus CDR3 sequence
QSYDnnnn
wherein each n independently represents any amino acid residue.

3. The peptide/protein of claim 2, wherein said VH CDR3 is SPRYRGAFDY identified as SEQ ID NO. 10.

4. A method of producing a peptide/protein comprising at least one antibody-based antigen-binding domain of human composition with binding specificity for an antigen expressed on the surface of a human cell, wherein binding of a multivalent composition of one or more of said peptide/protein to said antigen expressed on the surface of a cell causes or leads to killing of said cell, and wherein said peptide/protein is a human IgG antibody, the method comprising the steps of:
a. cloning into at least one immunoglobulin expression vector at least one nucleic acid encoding an antigen binding domain comprised in the peptide/protein of any one of claims 1 to 3;
b. transfecting said at least one vector into a host cell; and
c. expressing said IgG from said at least one nucleic acid.

5. A peptide/protein wherein said peptide/protein is a human IgG antibody comprising an antigen binding domain of the peptide/protein defined in any one of claims 1 to 3 or obtainable by the method of claim 4.

6. The peptide/protein of any one of claims 1 to 3 which is a multivalent composition comprising at least a F(ab')₂ antibody fragment or a mini-antibody fragment.

7. The peptide/protein of any one of claims 1 to 3, which is a multivalent composition comprising at least two monovalent antibody fragments selected from Fv, scFv, dsFv and Fab fragments, and further comprises a cross-linking moiety or moieties.

8. The peptide/protein of any one of claims 1 to 3, which is a multivalent composition comprising at least one full antibody selected from the antibodies of classes IgG1, IgG2a, IgG2b, IgG3, IgG4, IgA, and IgM.

9. The peptide/protein of any one of claims 1 to 3 and 5 to 8, where said antigen comprises epitopes selected from HLA-DR molecules.

10. The peptide/protein of any one of claims 1 to 3 and 5 to 9, wherein said killing occurs with an IC50 value for said multivalent composition lower than 100 nM for killing of GRANTA-519 cells under assay conditions described below:
(a) 2x10⁵ GRANTA-519 cells are incubated in media (RPMI 1640; supplemented with 2.5% heat inactivated FBS, 2mM L-glutamine, 1% non-essential amino acids, 1mM sodium pyruvate and 0.1 mg/ml kanamycin) with a dilution series of said multivalent composition (2 to 200 nM).
(b) After 4 h incubation at 37°C under 6% CO₂, cell viability is determined by fluorescein diacetate staining and subsequent counting of remaining viable cells.
(c) The concentration of multivalent composition that causes 50% cell killing (IC50) is determined.

11. A nucleic acid comprising one or more nucleic acid sequences encoding a peptide/protein of any one of claims 1 to 3 and 5 to 10.

12. A variant of the nucleic acid of claim 11 which hybridizes under stringent conditions with the nucleic acid sequence of claim 11, wherein said variant encodes a peptide/protein according to any one of claims 1 to 3 and 5 to 10.

13. A vector comprising at least one nucleic acid of claim 11 and/or at least one variant thereof according to claim 12.

14. A host cell harboring at least one nucleic acid of claim 11 and/or at least one variant thereof according to claim 12 and/or at least one vector of claim 13.

15. A method for the production of a peptide/protein according to any one of claims 1 to 3 and 5 to 10, comprising the step of expressing at least one nucleic acid of claim 11 or at least one variant thereof according to claim 12 in a suitable expression system, preferably in a host cell.

16. A pharmaceutical composition comprising at least one peptide/protein according to any one of claims 1 to 3 and 5 to 10, and, optionally, a pharmaceutically acceptable carrier and/or diluent.

17. The use of a peptide/protein according to any one of claims I to 3 and 5 to 10, for the preparation of a pharmaceutical composition for the treatment of animals.

18. The use according to claim 17, wherein said animal is a human.

19. The use according to claim 17 or 18, wherein said treatment is the treatment of cancer.

20. The use according to claim 19, wherein said treatment is the treatment of cancers comprising cells expressing MHC class II antigens.

21. The use according to claim 20, wherein said cells are lymphoid tumor cells.

22. The use according to claim 19 or 20, wherein said cancer is selected from Hodgkin, non-Hodgkin and Burkitt lymphoma, B cell lymphoma, B cell precursor leukemia, B cell acute lymphoid leukemia, acute myeloid leukemias, and hairy cell leukemia.

23. The use according to claim 20, wherein said cells are non-lymphoid cells.

24. The use according to claim 17 or 18, wherein said treatment is the treatment of diseases involving the immune system.

25. The use according to claim 24, wherein said treatment is the treatment of a condition selected from rheumatoid arthritis, juvenile arthritis, multiple sclerosis, Grave's disease, insulin-dependent diabetes, narcolepsy, psoriasis, systemic lupus erythematosus, ankylosing spondylitis, transplant rejection, graft vs. host disease, Hashimoto's disease, myasthenia gravis, pemphigus vulgaris, glomerulonephritis, thyroiditis, pancreatitis, insulitis, primary biliary cirrhosis, irritable bowel disease and Sjogren syndrome.

26. The use according to claim 25, wherein said disease is selected from myasthenia gravis, rheumatoid arthritis, multiple sclerosis, transplant rejection and graft vs. host disease.

27. A diagnostic composition containing at least one peptide/protein of any one of claims 1 to 3 and 5 to 10.

28. The diagnostic composition of claim 27 further comprising a cross-linking moiety or moieties.

29. Use of a multivalent composition comprising at least one peptide/protein according to any one of claims 1 to 3 and 5 to 10 for the preparation of a pharmaceutical composition for killing a cell.

30. The use of claim 29, wherein said cell is a lymphoid cell.

31. The use of claim 30, wherein said lymphoid cell is a lymphoid tumor cell.

32. The use of claim 29, wherein said cell is a non-lymphoid cell and expresses MHC class II molecules.

33. The use of claim 29, wherein said pharmaceutical composition is useful for the treatment of a disease selected from B cell non-Hodgkin lymphoma, B cell lymphoma, B cell acute lymphoid leukemia, Burkitt lymphoma, Hodgkin lymphoma, hairy cell leukemia, acute myeloid leukemia and B cell precursor leukemia.

34. A kit comprising
(i) a peptide/protein according to any one of claims 1 to 3 and 5 to 10, and
(ii) a cross-linking moiety.

35. A kit comprising
(i) a peptide/protein according to any one of claims 1 to 3 and 5 to 10, and
(ii) a detectable moiety or moieties, and
(iii) reagents and/or solutions to effect and/or detect binding of (i) to an antigen.

36. A multivalent composition comprising at least one peptide/protein according to any one of claims 1 to 3 and 5 to 10 and comprising at least two of said antigen binding domains.

37. The multivalent composition of claim 36, wherein the multivalent composition is an IgG antibody.

## Patentansprüche

1. Peptid/Protein, das zumindest eine auf einem Antikörper basierende Antigen-Bindungsdomäne von menschlicher Zusammensetzung mit Bindungsspezifität für ein auf der Oberfläche einer menschlichen Zelle exprimiertes Antigen umfasst, wobei die Bindung einer multivalenten Zusammensetzung von einem oder mehreren des Peptids/Proteins an das Antigen, das auf der Oberfläche einer Zelle exprimiert wird, den Tod der Zelle verursacht oder zum Tod der Zelle führt, wobei die Antigen-Bindungsdomäne aus einer Kombination einer VH-Domäne und einer VL-Domäne besteht, wobei die Kombination in einem der Clone aus der Liste MS-GPC-1, MS-GPC-6, MS-GPC-8, MS-GPC-10, MS-GPC-8-6 und MS-GPC-8-17, wie in Tabelle 1 und Figur 11 gezeigt, gefunden wird.

2. Peptid/Protein, das zumindest eine auf einem Antikörper basierende Antigen-Bindungsdomäne von menschlicher Zusammensetzung mit Bindungsspezifität für ein auf der Oberfläche einer menschlichen Zelle exprimiertes Antigen umfasst, wobei das Antigen Epitope umfasst, die aus HLA-DR-Molekülen ausgewählt sind, wobei die Bindung einer multivalenten Zusammensetzung von einem oder mehreren des Peptids/Proteins an das Antigen, das auf der Oberfläche einer aktivierten Zelle exprimiert wird, in Abwesenheit von weiteren cytotoxischen Einheiten oder immunologischen Mechanismen den Tod der aktivierten Zelle verursacht oder zum Tod der aktivierten Zelle führt, wobei die Antigen-Bindungsdomäne aus einer Kombination von HuCAL-VH2 und HuCAL-Vλ1 besteht, wobei die VH-CDR3-Sequenz von der Konsensus-CDR3-Sequenz
nnnnRGnFDn genommen wird, wobei jedes n unabhängig einen Aminosäurerest darstellt; und wobei die VL-CDR3-Sequenz von der Konsensus-CDR3-Sequenz
QSYDnnnn genommen wird, wobei jedes n unabhängig einen Aminosäurerest darstellt.

3. Peptid/Protein nach Anspruch 2, wobei der VH-CDR3 das als SEQ ID NO: 10 identifizierte SPRYRGAFDY ist.

4. Verfahren zur Produktion eines Peptids/Proteins, das zumindest eine auf einem Antikörper basierende Antigen-Bindungsdomäne von menschlicher Zusammensetzung mit Bindungsspezifität für ein auf der Oberfläche einer menschlichen Zelle exprimiertes Antigen umfasst, wobei die Bindung einer multivalenten Zusammensetzung von einem oder mehreren des Peptids/Proteins an das Antigen, das auf der Oberfläche einer Zelle exprimiert wird, den Tod der Zelle verursacht oder zum Tod der Zelle führt, und wobei das Peptid/Protein ein menschlicher IgG-Antikörper ist, wobei das Verfahren die Schritte umfasst:
(a) Clonieren von zumindest einer Nucleinsäure, die eine Antigen-Bindungsdomäne codiert, die von einem Peptid/Protein aus einem der Ansprüche 1 bis 3 umfasst wird, in zumindest einen Immunglobulin-Expressionsvektor;
(b) Transfektion des zumindest einen Vektors in eine Wirtszelle; und
(c) Expression des IgGs von der zumindest einen Nucleinsäure.

5. Peptid/Protein, wobei das Peptid/Protein ein menschlicher IgG-Antiköper ist, der eine Antigen-Bindungsdomäne des Peptids/Proteins umfasst, das in einem der Ansprüche 1 bis 3 definiert oder durch das Verfahren nach Anspruch 4 erhältlich ist.

6. Peptid/Protein nach einem der Ansprüche 1 bis 3, das eine multivalente Zusammensetzung ist, die zumindest ein F(ab')₂-Antikörperfragment oder ein Miniantikörperfragment umfasst.

7. Peptid/Protein nach einem der Ansprüche 1 bis 3, das eine multivalente Zusammensetzung ist, die zumindest zwei monovalente Antikörperfragmente ausgewählt aus Fv-, scFv-, dsFv- und Fab-Fragmenten umfasst, und das außerdem eine Vernetzungseinheit oder Vernetzungseinheiten umfasst.

8. Peptid/Protein nach einem der Ansprüche 1 bis 3, das eine multivalente Zusammensetzung ist, die zumindest einen vollständigen Antikörper ausgewählt aus den Antikörpern der Klassen IgG1, IgG2a, 1gG2b, IgG3, IgG4, IgA und IgM umfasst.

9. Peptid/Protein nach einem der Ansprüche 1 bis 3 und 5 bis 8, wobei das Antigen Epitope ausgewählt aus HLA-DR-Molekülen umfasst.

10. Peptid/Protein nach einem der Ansprüche 1 bis 3 und 5 bis 9, wobei der Tod mit einem IC₅₀-Wert für die multivalente Zusammensetzung eintritt, der niedriger als 100 nM für das Abtöten von GRANTA-519 Zellen unter den nachstehend beschriebenen Assay-Bedingungen ist:
(a) 2x10⁵ GRANTA-519-Zellen werden in Medium (RPMI 1640; ergänzt durch 2.5% hitzeinaktiviertes FBS, 2mM L-Glutamin, 1 % nicht-essentielle Aminosäuren, 1 mM Natriumpyruvat und 0.1 mg/ml Kanamycin) mit einer Verdünnungsreihe der multivalenten Zusammensetzung (2 bis 200 nM) inkubiert;
(b) nach 4 h Inkubation bei 37°C unter 6% CO₂ wird die Zelllebensfähigkeit durch Fluoresceindiacetat-Färbung und anschließende Zählung der verbleibenden lebensfähigen Zellen bestimmt;
(c) die Konzentration der multivalenten Zusammensetzung, die 50% Zelltötung verursacht (IC₅₀), wird bestimmt.

11. Nucleinsäure, die eine oder mehrere Nucleinsäuresequenzen umfasst, die ein Peptid/Protein nach einem der Ansprüche 1 bis 3 und 5 bis 10 codieren.

12. Variante der Nucleinsäure nach Anspruch 11, die unter stringenten Bedingungen mit der Nucleinsäuresequenz nach Anspruch 11 hybridisiert, wobei die Variante ein Peptid/Protein gemäß einem der Ansprüche 1 bis 3 und 5 bis 10 codiert.

13. Vektor, der zumindest eine Nucleinsäure nach Anspruch 11 und/oder zumindest eine Variante davon gemäß Anspruch 12 umfasst.

14. Wirtszelle, die zumindest eine Nucleinsäure nach Anspruch 11 und/oder zumindest eine Variante davon gemäß Anspruch 12 und/oder einen Vektor nach Anspruch 13 beherbergt.

15. Verfahren zur Produktion eines Peptids/Proteins gemäß einem der Ansprüche 1 bis 3 und 5 bis 10, das den Schritt umfasst, zumindest eine Nucleinsäure nach Anspruch 11 oder zumindest eine Variante davon gemäß Anspruch 12 in einem geeigneten Expressionssystem, bevorzugt in einer Wirtszelle, zu exprimieren.

16. Arzneimittel, das zumindest ein Peptid/Protein gemäß einem der Ansprüche 1 bis 3 und 5 bis 10 umfasst, und gegebenenfalls einen pharmazeutisch verträglichen Träger und/oder ein pharmazeutisch verträgliches Verdünnungsmittel.

17. Verwendung eines Peptids/Proteins gemäß einem der Ansprüche 1 bis 3 und 5 bis 10 für die Herstellung eines Arzneimittels für die Behandlung von Lebewesen.

18. Verwendung gemäß Anspruch 17, wobei das Lebewesen ein Mensch ist.

19. Verwendung gemäß Anspruch 17 oder 18, wobei die Behandlung die Behandlung von Krebs ist.

20. Verwendung gemäß Anspruch 19, wobei die Behandlung die Behandlung von Krebsarten ist, die Zellen umfassen, die MHC-Klasse-II-Antigene exprimieren.

21. Verwendung gemäß Anspruch 20, wobei die Zellen lymphoide Tumorzellen sind.

22. Verwendung gemäß Anspruch 19 oder 20, wobei der Krebs ausgewählt ist aus Hodgkin-, Nicht-Hodgkin- und Burkitt-Lymphom, B-Zell-Lymphom, B-Zell-Vorläufer-Leukämie, akute lymphoide Leukämie der B-Zellen, akute myeloische Leukämien und Haarzell-Leukämie.

23. Verwendung gemäß Anspruch 20, wobei die Zellen keine lymphoiden Zellen sind.

24. Verwendung gemäß Anspruch 17 oder 18, wobei die Behandlung die Behandlung von Erkrankungen ist, die das Immunsystem einbeziehen.

25. Verwendung gemäß Anspruch 24, wobei die Behandlung die Behandlung eines Zustandes ist, der ausgewählt ist aus rheumatoider Arthritis, juveniler Arthritis, multipler Sklerose, Morbus Basedow, insulinabhängigem Diabetes, Narkolepsie, Psoriasis, systemischem Lupus erythematodes, Morbus Bechterew, Transplantat-Abstoßung, Graft-versus-Host-Erkrankung, Hashimoto-Thyreoiditis, Myasthenia gravis, Pemphigus vulgaris, Glomerulonephritis, Thyroiditis, Pankreatitis, Insulitis, primär biliärer Zirrhose, irritable Darmerkrankung und Sjögren-Syndrom.

26. Verwendung gemäß Anspruch 25, wobei die Erkrankung ausgewählt ist aus Myasthenia gravis, rheumatoider Arthritis, multipler Sklerose, Transplantat-Abstoßung und Graft-versus-Host-Erkrankung.

27. Diagnostische Zusammensetzung, die zumindest ein Peptid/Protein nach einem der Ansprüche 1 bis 3 und 5 bis 10 enthält.

28. Diagnostische Zusammensetzung nach Anspruch 27, die außerdem eine Vernetzungseinheit oder Vernetzungseinheiten umfasst.

29. Verwendung einer multivalenten Zusammensetzung, die zumindest ein Peptid/Protein gemäß einem der Ansprüche 1 bis 3 und 5 bis 10 umfasst, für die Herstellung eines Arzneimittels für das Abtöten einer Zelle.

30. Verwendung nach Anspruch 29, wobei die Zelle eine lymphoide Zelle ist.

31. Verwendung nach Anspruch 30, wobei die lymphoide Zelle eine lymphoide Tumorzelle ist.

32. Verwendung nach Anspruch 29, wobei die Zelle eine nicht-lymphoide Zelle ist und MHC-Klasse-II-Moleküle exprimiert.

33. Verwendung nach Anspruch 29, wobei das Arzneimittel verwendbar ist für die Behandlung einer Krankheit ausgewählt aus B-Zell-nicht-Hodgkin-Lymphom, B-Zell-Lymphom, akuter lymphoider Leukämie der B-Zellen, Burkitt-Lymphom, Hodgkin-Lymphom, Haarzell-Leukämie, akuter myeloischer Leukämie und B-Zell-Vorläufer-Leukämie.

34. Kit umfassend:
(i) ein Peptid/Protein gemäß einem der Ansprüche 1 bis 3 und 5 bis 10, und
(ii) eine Vernetzungseinheit.

35. Kit umfassend
(i) ein Peptid/Protein gemäß einem der Ansprüche 1 bis 3 und 5 bis 10, und
(ii) eine nachweisbare Einheit oder nachweisbare Einheiten, und
(iii) Reagenzien und/oder Lösungen, um die Bindung von (i) an ein Antigen zu erreichen und/oder nachzuweisen.

36. Multivalente Zusammensetzung, die zumindest ein Peptid/Protein gemäß einem der Ansprüche 1 bis 3 und 5 bis 10 und zumindest zwei der Antigen-Bindungsdomänen umfasst.

37. Multivalente Zusammensetzung nach Anspruch 36, wobei die multivalente Zusammensetzung ein IgG-Antikörper ist.

## Revendications

1. Peptide/protéine comprenant au moins un domaine de liaison à l'antigène de composition humaine basé sur un anticorps avec une spécificité de liaison pour un antigène exprimé à la surface d'une cellule humaine, où la liaison d'une composition multivalente d'un(e) ou plusieurs des dit(e)s peptides/ protéines au dit antigène exprimé à la surface d'une cellule provoque ou mène à la destruction de ladite cellule, où ledit domaine de liaison à l'antigène est constitué d'une combinaison d'un domaine VH et d'un domaine VL, où ladite composition est trouvée dans l'un des clones pris dans la liste de MS-GPC-1, MS-GPC-6, MS-GPC-8, MS-GPC-10, MS-GPC-8-6 et MS-GPC-8-17 tels que présentés dans le Tableau 1 et sur la Figure 11.

2. Peptide/protéine comprenant au moins un domaine de liaison à l'antigène de composition humaine basé sur un anticorps avec une spécificité de liaison pour un antigène exprimé à la surface d'une cellule humaine, où ledit antigène comprend des épitopes choisis parmi des molécules HLA-DR, où la liaison d'une composition multivalente d'un(e) ou plusieurs des dit(e)s peptides/protéines au dit antigène exprimé à la surface d'une cellule activée provoque ou mène à la destruction de ladite cellule activée en l'absence d'autres entités cytotoxiques ou mécanismes immunologiques, où ledit domaine de liaison à l'antigène est constitué d'une combinaison de HuCAL VH2 et HuCAL Vλ1, où la séquence de VH CDR3 est prise à partir de la séquence consensus de CDR3
nnnnRGnFDn
où chaque n représente indépendamment un résidu d'acide aminé quelconque ; et où la séquence de VL CDR3 est prise à partir de la séquence consensus de CDR3
QSYDnnnn
où chaque n représente indépendamment un résidu d'acide aminé quelconque.

3. Peptide/protéine selon la revendication 2, où ledit VH CDR3 est SPRYRGAFDY identifié comme SEQ ID NO : 10.

4. Procédé de production d'un(e) peptide/protéine comprenant au moins un domaine de liaison à l'antigène de composition humaine basé sur un anticorps avec une spécificité de liaison pour un antigène exprimé à la surface d'une cellule humaine, où la liaison d'une composition multivalente d'un(e) ou plusieurs des dit(e)s peptides/protéines au dit antigène exprimé à la surface d'une cellule provoque ou mène à la destruction de ladite cellule, et où ledit (ladite) peptide/protéine est un anticorps IgG humain, le procédé comprenant les étapes consistant à :
(a) cloner dans un moins un vecteur d'expression d'immunoglobuline, au moins un acide nucléique codant pour un domaine de liaison à l'antigène compris dans le (la) peptide/protéine selon l'une quelconque des revendications 1 à 3 ;
(b) transfecter ledit au moins un vecteur dans une cellule hôte ; et
(c) exprimer ladite IgG à partir dudit au moins un acide nucléique.

5. Peptide/protéine où ledit (ladite) peptide/protéine est un anticorps IgG humain comprenant un domaine de liaison à l'antigène du (de la) peptide/protéine défini(e) dans l'une quelconque des revendications 1 à 3 ou pouvant être obtenu(e) par le procédé selon la revendication 4.

6. Peptide/protéine selon l'une quelconque des revendications 1 à 3, qui est une composition multivalente comprenant au moins un fragment d'anticorps F(ab')₂ ou un mini-fragment d'anticorps.

7. Peptide/protéine selon l'une quelconque des revendications 1 à 3, qui est une composition multivalente comprenant au moins deux fragments monovalents d'anticorps choisis parmi les fragments Fv, scFv, dsFv et Fab, et comprend en outre un radical ou des radicaux de réticulation.

8. Peptide/protéine selon l'une quelconque des revendications 1 à 3, qui est une composition multivalente comprenant au moins un anticorps entier choisi parmi les anticorps des classes IgG1, IgG2a, IgG2b, IgG3, IgG4, IgA et IgM.

9. Peptide/protéine selon l'une quelconque des revendications 1 à 3 et 5 à 8, où ledit antigène comprend des épitopes choisis parmi des molécules HLA-DR.

10. Peptide/protéine selon l'une quelconque des revendications 1 à 3 et 5 à 9, où ladite destruction se produit avec une valeur de CI50 pour ladite composition multivalente inférieure à 100 nM pour la destruction de cellules GRANTA-519 dans des conditions de test décrites ci-dessous :
(a) 2 x 10⁵ cellules GRANTA-519 sont incubées dans du milieu (RPMI 1640 ; supplémenté avec 2,5 % de FBS inactivé à la chaleur, 2 mM de L-glutamine, 1 % d'acides aminés non essentiels, 1 mM de pyruvate de sodium et 0,1 mg/ml de kanamycine) avec une série de dilutions de ladite composition multivalente (2 à 200 nM).
(b) Après une incubation de 4 h à 37ºC sous 6 % de CO₂, la viabilité des cellules est déterminée par une coloration avec du diacétate de fluorescéine et ensuite le dénombrement des cellules viables restantes.
(c) La concentration de la composition multivalente qui provoque 50 % de destruction cellulaire (CI50) est déterminée.

11. Acide nucléique comprenant une ou plusieurs séquences d'acide nucléique codant pour un(e) peptide/protéine selon l'une quelconque des revendications 1 à 3 et 5 à 10.

12. Variant de l'acide nucléique selon la revendication 11, qui s'hybride dans des conditions stringentes à la séquence d'acide nucléique selon la revendication 11, où ledit variant code pour un(e) peptide/protéine selon l'une quelconque des revendications 1 à 3 et 5 à 10.

13. Vecteur comprenant au moins un acide nucléique selon la revendication 11 et/ou au moins un variant de celui-ci selon la revendication 12.

14. Cellule hôte contenant au moins un acide nucléique selon la revendication 11 et/ou au moins un variant de celui-ci selon la revendication 12 et/ou au moins un vecteur selon la revendication 13.

15. Procédé de production d'un(e) peptide/protéine selon l'une quelconque des revendications 1 à 3 et 5 à 10, comprenant l'étape consistant à exprimer au moins un acide nucléique selon la revendication 11 ou au moins un variant de celui-ci selon la revendication 12 dans un système d'expression approprié, de préférence dans une cellule hôte.

16. Composition pharmaceutique comprenant au moins un(e) peptide/protéine selon l'une quelconque des revendications 1 à 3 et 5 à 10, et, éventuellement, un support et/ou un diluant pharmaceutiquement acceptable.

17. Utilisation d'un(e) peptide/protéine selon l'une quelconque des revendications 1 à 3 et 5 à 10, pour la préparation d'une composition pharmaceutique destinée au traitement d'animaux.

18. Utilisation selon la revendication 17, où ledit animal est un humain.

19. Utilisation selon la revendication 17 ou 18, où ledit traitement est le traitement du cancer.

20. Utilisation selon la revendication 19, où ledit traitement est le traitement de cancers comprenant des cellules exprimant des antigènes du CMH de classe II.

21. Utilisation selon la revendication 20, où lesdites cellules sont des cellules de tumeurs lymphoïdes.

22. Utilisation selon la revendication 19 ou 20, où ledit cancer est choisi parmi un lymphome de Hodgkin, non hodgkinien ou de Burkitt, un lymphome à cellules B, une leucémie à précurseurs de cellules B, une leucémie lymphoïde aiguë à cellules B, des leucémies myéloïdes aiguës et une leucémie à tricholeucocytes.

23. Utilisation selon la revendication 20, où lesdites cellules sont des cellules non lymphoïdes.

24. Utilisation selon la revendication 17 ou 18, où ledit traitement est le traitement de maladies impliquant le système immunitaire.

25. Utilisation selon la revendication 24, où ledit traitement est le traitement d'une affection choisie parmi la polyarthrite rhumatoïde, l'arthrite juvénile, la sclérose en plaques, la maladie de Basedow, le diabète insulinodépendant, la narcolepsie, le psoriasis, le lupus érythémateux systémique, la spondylarthrite ankylosante, le rejet de transplantation, la maladie du greffon contre l'hôte, la maladie de Hashimoto, la myasthénie grave, le pemphigus vulgaire, la glomérulonéphrite, la thyroïdite, la pancréatite, l'insulite, la cirrhose biliaire primaire, la maladie du côlon irritable et le syndrome de Sjögren.

26. Utilisation selon la revendication 25, où ladite maladie est choisie parmi la myasthénie grave, la polyarthrite rhumatoïde, la sclérose en plaques, le rejet de transplantation et la maladie du greffon contre l'hôte.

27. Composition diagnostique contenant au moins un(e) peptide/protéine selon l'une quelconque des revendications 1 à 3 et 5 à 10.

28. Composition diagnostique selon la revendication 27, comprenant en outre un radical ou des radicaux de réticulation.

29. Utilisation d'une composition multivalente comprenant au moins un(e) peptide/protéine selon l'une quelconque des revendications 1 à 3 et 5 à 10 pour la préparation d'une composition pharmaceutique destinée à la destruction d'une cellule.

30. Utilisation selon la revendication 29, où ladite cellule est une cellule lymphoïde.

31. Utilisation selon la revendication 30, où ladite cellule lymphoïde est une cellule de tumeur lymphoïde.

32. Utilisation selon la revendication 29, où ladite cellule est une cellule non lymphoïde et exprime des molécules du CMH de classe II.

33. Utilisation selon la revendication 29, où ladite composition pharmaceutique est utile pour le traitement d'une maladie choisie parmi le lymphome non hodgkinien à cellules B, le lymphome à cellules B, la leucémie lymphoïde aiguë à cellules B, le lymphome de Burkitt, le lymphome de Hodgkin, la leucémie à tricholeucocytes, la leucémie myéloïde aiguë et la leucémie à précurseurs de cellules B.

34. Kit comprenant
(i) un(e) peptide/protéine selon l'une quelconque des revendications 1 à 3 et 5 à 10, et
(ii) un radical de réticulation.

35. Kit comprenant
(i) un(e) peptide/protéine selon l'une quelconque des revendications 1 à 3 et 5 à 10, et
(ii) un radical ou des radicaux détectables, et
(iii) des réactifs et/ou des solutions pour effectuer et/ou détecter la liaison de
(i) à un antigène.

36. Composition multivalente comprenant au moins un(e) peptide/protéine selon l'une quelconque des revendications 1 à 3 et 5 à 10 et comprenant au moins deux desdits domaines de liaison à l'antigène.

37. Composition multivalente selon la revendication 36, où la composition multivalente est un anticorps IgG.


### SEQUENCE LISTING

<110> GPC Biotech AG
   MorphoSys AG
<120> Human peptides/proteins causing or leading to the killing of cells including lymphoid tumor cells
<130> killing antibody
<140> 00110065.0
   <141> 2000-05-12
<160> 29
<170> PatentIn version 3.0
<210> 1
   <211> 10
   <212> PRT
   <213> artificial sequence
<220>
   <221> VHconCDR3
   <222> (1)..(10)
   <223> "X" represents any amino acid residue
<400> 1
   Xaa Xaa Xaa Xaa Arg Gly Xaa Phe Asp Xaa
   1               5                   10
<210> 2
   <211> 8
   <212> PRT
   <213> artificial sequence
<220>
   <221> VLconCDR3
   <222> (1)..(8)
   <223> "X" represents any amino acid residue
<400> 2
   Gln Ser Tyr Asp Xaa Xaa Xaa Xaa
   1               5
<210> 3
   <211> 8
   <212> PRT
   <213> artificial sequence
<220>
   <221> Streptaq
   <222> (1)..(8)
<400> 3
   Trp Ser His Pro Gln Phe Glu Lys
   1               5
<210> 4
   <211> 17
   <212> DNA
   <213> artificial sequence
<220>
   <221> PrimerCRT5
   <222> (1)..(17)
<400> 4
   gtggtggttc cgatatc 17
<210> 5
   <211> 44
   <212> DNA
   <213> artificial sequence.
<220>
   <221> PrimerCRT6
   <222> (1)..(44)
<400> 5
   agcgtcacac tcggtgcggc tttcggctgg ccaagaacgg gtta 44
<210> 6
   <211> 10
   <212> PRT
   <213> artificial sequence
<220>
   <221> MS-GPC1-VH-CDR3
   <222> (1)..(10)
<400> 6
   Gln Tyr Gly His Arg Gly Gly Phe Asp His
   1 5 10
<210> 7
   <211> 8
   <212> PRT
   <213> artificial sequence
<220>
   <221> MS-GPC1-VL-CDR3
   <222> (1)..(8)
<400> 7
   Gln Ser Tyr Asp Phe Asn Glu Ser
   1 5
<210> 8
   <211> 9
   <212> PRT
   <213> artificial sequence
<220>
   <221> MS-GPC6-VH-CDR3
   <222> (1)..(9)
<400> 8
   Gly Tyr Gly Arg Tyr Ser Pro Asp Leu
   1 5
<210> 9
   <211> 8
   <212> PRT
   <213> artificial sequence
<220>
   <221> MS-GPC6-VL-CDR3
   <222> (1)..(8)
<400> 9
   Gln Gln Tyr Ser Asn Leu Pro Phe
   1 5
<210> 10
   <211> 10
   <212> PRT
   <213> artificial sequence
<220>
   <221> MS-GPC8-VH-CDR3
   <222> (1)..(10)
<400> 10
   Ser Pro Arg Tyr Arg Gly Ala Phe Asp Tyr
   1 5 10
<210> 11
   <211> 8
   <212> PRT
   <213> artificial sequence
<220>
   <221> MS-GPC8-VL-CDR3
   <222> (1)..(8)
<400> 11
   Gln Ser Tyr Asp Met Pro Gln Ala
   1 5
<210> 12
   <211> 10
   <212> PRT
   <213> artificial sequence
<220>
   <221> MS-GPC10-VH-CDR3
   <222> (1)..(10)
<400> 12
   Gln Leu His Tyr Arg Gly Gly Phe Asp Leu
   1               5                   10
<210> 13
   <211> 8
   <212> PRT
   <213> artificial sequence
<220>
   <221> MS-GPC10-VL-CDR3
   <222> (1)..(8)
<400> 13
   Gln Ser Tyr Asp Leu Thr Met Gly
   1               5
<210> 14
   <211> 8
   <212> PRT
   <213> artificial sequence
<220>
   <221> MS-GPCB-6-VL-CDR3
   <222> (1)..(8)
<400> 14
   Gln Ser Tyr Asp Tyr Asp His Tyr
   1               5
<210> 15
   <211> 8
   <212> PRT
   <213> artificial sequence
<220>
   <221> MS-GPC8-17-VL-CDR3
   <222> (1)..(8)
<400> 15
   Gln Ser Tyr Asp Phe Ser Val Tyr
   1               5
<210> 16
   <211> 3548
   <212> DNA
   <213> artificial sequence
<220>
   <221> pMORPH13_scFv
   <222> (1)..(3548)
<400> 16
   agagcatgcg taggagaaaa taaaatgaaa caaagcacta ttgcactggc actcttaccg      60
   ttgctcttca cccctgttac caaagccgac tacaaagatg aagtgcaatt ggttcagtct     120
   ggcgcggaag tgaaaaaacc gggcagcagc gtgaaagtga gctgcaaagc ctccggaggc     180
   acttttagca gctatgcgat tagctgggtg cgccaagccc ctgggcaggg tctcgagtgg     240
   atgggcggca ttattccgat ttttggcacg gcgaactacg cgcagaagtt tcagggccgg     300
   gtgaccatta ccgcggatga aagcaccagc accgcgtata tggaactgag cagcctgcgt     360
   agcgaagata cggccgtgta ttattgcgcg cgttattatg atcgtatgta taatatggat     420
   tattggggcc aaggcaccct ggtgacggtt agctcagcgg gtggcggttc tggcggcggt     480
   gggagcggtg gcggtggttc tggcggtggt ggttccgata tcgaactgac ccagccgcct     540
   tcagtgagcg ttgcaccagg tcagaccgcg cgtatctcgt gtagcggcga tgcgctgggc     600
   gataaatacg cgagctggta ccagcagaaa cccgggcagg cgccagttct ggtgatttat     660
   gatgattctg accgtccctc aggcatcccg gaacgcttta gcggatccaa cagcggcaac     720
   accgcgaccc tgaccattag cggcactcag gcggaagacg aagcggatta ttattgccag     780
   agctatgacg ctcatatgcg tcctgtgttt ggcggcggca cgaagttaac cgttcttggc     840
   caggaattcg agcagaagct gatctctgag gaggatctga actagggtgg tggctctggt     900
   tccggtgatt ttgattatga aaagatggca aacgctaata agggggctat gaccgaaaat     960
   gccgatgaaa acgcgctaca gtctgacgct aaaggcaaac ttgattctgt cgctactgat    1020
   tacggtgctg ctatcgatgg tttcattggt gacgtttccg gccttgctaa tggtaatggt    1080
   gctactggtg attttgctgg ctctaattcc caaatggctc aagtcggtga cggtgataat    1140
   tcacctttaa tgaataattt ccgtcaatat ttaccttccc tccctcaatc ggttgaatgt    1200
   cgcccttttg tctttggcgc tggtaaacca tatgaatttt ctattgattg tgacaaaata    1260
   aacttattcc gtggtgtctt tgcgtttctt ttatatgttg ccacctttat gtatgtattt    1320
   tctacgtttg ctaacatact gcgtaataag gagtcttgat aagcttgacc tgtgaagtga    1380
   aaaatggcgc agattgtgcg acattttttt tgtctgccgt ttaatgaaat tgtaaacgtt    1440
   aatattttgt taaaattcgc gttaaatttt tgttaaatca gctcattttt taaccaatag    1500
   gccgaaatcg gcaaaatccc ttataaatca aaagaataga ccgagatagg gttgagtgtt    1560
   gttccagttt ggaacaagag tccactatta aagaacgtgg actccaacgt caaagggcga    1620
   aaaaccgtct atcagggcga tggcccacta cgagaaccat caccctaatc aagttttttg    1680
   gggtcgaggt gccgtaaagc actaaatcgg aaccctaaag ggagcccccg atttagagct    1740
   tgacggggaa agccggcgaa cgtggcgaga aaggaaggga agaaagcgaa aggagcgggc    1800
   gctagggcgc tggcaagtgt agcggtcacg ctgcgcgtaa ccaccacacc cgccgcgctt    1860
   aatgcgccgc tacagggcgc gtgctagcca tgtgagcaaa aggccagcaa aaggccagga    1920
   accgtaaaaa ggccgcgttg ctggcgtttt tccataggct ccgcccccct gacgagcatc    1980
   acaaaaatcg acgctcaagt cagaggtggc gaaacccgac aggactataa agataccagg    2040
   cgtttccccc tggaagctcc ctcgtgcgct ctcctgttcc gaccctgccg cttaccggat    2100
   acctgtccgc ctttctccct tcgggaagcg tggcgctttc tcatagctca cgctgtaggt    2160
   atctcagttc ggtgtaggtc gttcgctcca agctgggctg tgtgcacgaa ccccccgttc    2220
   agtccgaccg ctgcgcctta tccggtaact atcgtcttga gtccaacccg gtaagacacg    2280
   acttatcgcc actggcagca gccactggta acaggattag cagagcgagg tatgtaggcg 2340
   gtgctacaga gttcttgaag tggtggccta actacggcta cactagaaga acagtatttg 2400
   gtatctgcgc tctgctgtag ccagttacct tcggaaaaag agttggtagc tcttgatccg 2460
   gcaaacaaac caccgctggt agcggtggtt tttttgtttg caagcagcag attacgcgca 2520
   gaaaaaaagg atctcaagaa gatcctttga tcttttctac ggggtctgac gctcagtgga 2580
   acgaaaactc acgttaaggg attttggtca gatctagcac caggcgttta agggcaccaa 2640
   taactgcctt aaaaaaatta cgccccgccc tgccactcat cgcagtactg ttgtaattca 2700
   ttaagcattc tgccgacatg gaagccatca caaacggcat gatgaacctg aatcgccagc 2760
   ggcatcagca ccttgtcgcc ttgcgtataa tatttgccca tagtgaaaac gggggcgaag 2820
   aagttgtcca tattggctac gtttaaatca aaactggtga aactcaccca gggattggct 2880
   gagacgaaaa acatattctc aataaaccct ttagggaaat aggccaggtt ttcaccgtaa 2940
   cacgccacat cttgcgaata tatgtgtaga aactgccgga aatcgtcgtg gtattcactc 3000
   cagagcgatg aaaacgtttc agtttgctca tggaaaacgg tgtaacaagg gtgaacacta 3060
   tcccatatca ccagctcacc gtctttcatt gccatacgga actccgggtg agcattcatc 3120
   aggcgggcaa gaatgtgaat aaaggccgga taaaacttgt gcttattttt ctttacggtc 3180
   tttaaaaagg ccgtaatatc cagctgaacg gtctggttat aggtacattg agcaactgac 3240
   tgaaatgcct caaaatgttc tttacgatgc cattgggata tatcaacggt ggtatatcca 3300
   gtgatttttt tctccatttt agcttcctta gctcctgaaa atctcgataa ctcaaaaaat 3360
   acgcccggta gtgatcttat ttcattatgg tgaaagttgg aacctcaccc gacgtctaat 3420
   gtgagttagc tcactcatta ggcaccccag gctttacact ttatgcttcc ggctcgtatg 3480
   ttgtgtggaa ttgtgagcgg ataacaattt cacacaggaa acagctatga ccatgattac 3540
   gaatttct 3548
<210> 17
   <211> 4410
   <212> DNA
   <213> artificial sequence
<220>
   <221> pMx7_FS_5D2
   <222> (1)..(4410)
<400> 17
   tctagagcat gcgtaggaga aaataaaatg aaacaaagca ctattgcact ggcactctta     60
   ccgttgctct tcacccctgt taccaaagcc gactacaaag atgaagtgca attggtggaa    120
   agcggcggcg gcctggtgca accgggcggc agcctgcgtc tgagctgcgc ggcctccgga    180
   tttaccttta gcagctatgc gatgagctgg gtgcgccaag cccctgggaa gggtctcgag    240
   tgggtgagcg cgattagcgg tagcggcggc agcacctatt atgcggatag cgtgaaaggc 300
   cgttttacca tttcacgtga taattcgaaa aacaccctgt atctgcaaat gaacagcctg 360
   cgtgcggaag atacggccgt gtattattgc gcgcgtgtta agaagcattt ttctcgtaag 420
   aattggtttg attattgggg ccaaggcacc ctggtgacgg ttagctcagc gggtggcggt 480
   tctggcggcg gtgggagcgg tggcggtggt tctggcggtg gtggttccga tatcgtgatg 540
   acccagagcc cactgagcct gccagtgact ccgggcgagc ctgcgagcat tagctgcaga 600
   agcagccaaa gcctgctgca tagcaacggc tataactatc tggattggta ccttcaaaaa 660
   ccaggtcaaa gcccgcagct attaatttat ctgggcagca accgtgccag tggggtcccg 720
   gatcgtttta gcggctctgg atccggcacc gattttaccc tgaaaattag ccgtgtggaa 780
   gctgaagacg tgggcgtgta ttattgccag cagcattata ccaccccgcc gacctttggc 840
   cagggtacga aagttgaaat taaacgtacg gaattcgact ataaagatga cgatgacaaa 900
   ggcgcgccgt ggagccaccc gcagtttgaa aaatgataag cttgacctgt gaagtgaaaa 960
   atggcgcaga ttgtgcgaca ttttttttgt ctgccgttta attaaagggg ggggggggcc 1020
   ggcctggggg ggggtgtaca tgaaattgta aacgttaata ttttgttaaa attcgcgtta 1080
   aatttttgtt aaatcagctc attttttaac caataggccg aaatcggcaa aatcccttat 1140
   aaatcaaaag aatagaccga gatagggttg agtgttgttc cagtttggaa caagagtcca 1200
   ctattaaaga acgtggactc caacgtcaaa gggcgaaaaa ccgtctatca gggcgatggc 1260
   ccactacgag aaccatcacc ctaatcaagt tttttggggt cgaggtgccg taaagcacta 1320
   aatcggaacc ctaaagggag cccccgattt agagcttgac ggggaaagcc ggcgaacgtg 1380
   gcgagaaagg aagggaagaa agcgaaagga gcgggcgcta gggcgctggc aagtgtagcg 1440
   gtcacgctgc gcgtaaccac cacacccgcc gcgcttaatg cgccgctaca gggcgcgtgc 1500
   tagactagtg tttaaaccgg accggggggg ggcttaagtg ggctgcaaaa caaaacggcc 1560
   tcctgtcagg aagccgcttt tatcgggtag cctcactgcc cgctttccag tcgggaaacc 1620
   tgtcgtgcca gctgcatcag tgaatcggcc aacgcgcggg gagaggcggt ttgcgtattg 1680
   ggagccaggg tggtttttct tttcaccagt gagacgggca acagctgatt gcccttcacc 1740
   gcctggccct gagagagttg cagcaagcgg tccacgctgg tttgccccag caggcgaaaa 1800
   tcctgtttga tggtggtcag cggcgggata taacatgagc tgtcctcggt atcgtcgtat 1860
   cccactaccg agatgtccgc accaacgcgc agcccggact cggtaatggc acgcattgcg 1920
   cccagcgcca tctgatcgtt ggcaaccagc atcgcagtgg gaacgatgcc ctcattcagc 1980
   atttgcatgg tttgttgaaa accggacatg gcactccagt cgccttcccg ttccgctatc 2040
   ggctgaattt gattgcgagt gagatattta tgccagccag ccagacgcag acgcgccgag 2100
   acagaactta atgggccagc taacagcgcg atttgctggt ggcccaatgc gaccagatgc 2160
   tccacgccca gtcgcgtacc gtcctcatgg gagaaaataa tactgttgat gggtgtctgg 2220
   tcagagacat caagaaataa cgccggaaca ttagtgcagg cagcttccac agcaatagca 2280
   tcctggtcat ccagcggata gttaataatc agcccactga cacgttgcgc gagaagattg 2340
   tgcaccgccg ctttacaggc ttcgacgccg cttcgttcta ccatcgacac gaccacgctg 2400
   gcacccagtt gatcggcgcg agatttaatc gccgcgacaa tttgcgacgg cgcgtgcagg 2460
   gccagactgg aggtggcaac gccaatcagc aacgactgtt tgcccgccag ttgttgtgcc 2520
   acgcggttag gaatgtaatt cagctccgcc atcgccgctt ccactttttc ccgcgttttc 2580
   gcagaaacgt ggctggcctg gttcaccacg cgggaaacgg tctgataaga gacaccggca 2640
   tactctgcga catcgtataa cgttactggt ttcacattca ccaccctgaa ttgactctct 2700
   tccgggcgct atcatgccat accgcgaaag gttttgcgcc attcgatgct agccatgtga 2760
   gcaaaaggcc agcaaaaggc caggaaccgt aaaaaggccg cgttgctggc gtttttccat 2820
   aggctccgcc cccctgacga gcatcacaaa aatcgacgct caagtcagag gtggcgaaac 2880
   ccgacaggac tataaagata ccaggcgttt ccccctggaa gctccctcgt gcgctctcct 2940
   gttccgaccc tgccgcttac cggatacctg tccgcctttc tcccttcggg aagcgtggcg 3000
   ctttctcata gctcacgctg taggtatctc agttcggtgt aggtcgttcg ctccaagctg 3060
   ggctgtgtgc acgaaccccc cgttcagccc gaccgctgcg ccttatccgg taactatcgt 3120
   cttgagtcca acccggtaag acacgactta tcgccactgg cagcagccac tggtaacagg 3180
   attagcagag cgaggtatgt aggcggtgct acagagttct tgaagtggtg gcctaactac 3240
   ggctacacta gaagaacagt atttggtatc tgcgctctgc tgtagccagt taccttcgga 3300
   aaaagagttg gtagctcttg atccggcaaa caaaccaccg ctggtagcgg tggttttttt 3360
   gtttgcaagc agcagattac gcgcagaaaa aaaggatctc aagaagatcc tttgatcttt 3420
   tctacggggt ctgacgctca gtggaacgaa aactcacgtt aagggatttt ggtcagatct 3480
   agcaccaggc gtttaagggc accaataact gccttaaaaa aattacgccc cgccctgcca 3540
   ctcatcgcag tactgttgta attcattaag cattctgccg acatggaagc catcacaaac 3600
   ggcatgatga acctgaatcg ccagcggcat cagcaccttg tcgccttgcg tataatattt 3660
   gcccatagtg aaaacggggg cgaagaagtt gtccatattg gctacgttta aatcaaaact 3720
   ggtgaaactc acccagggat tggctgagac gaaaaacata ttctcaataa accctttagg 3780
   gaaataggcc aggttttcac cgtaacacgc cacatcttgc gaatatatgt gtagaaactg 3840
   ccggaaatcg tcgtggtatt cactccagag cgatgaaaac gtttcagttt gctcatggaa 3900
   aacggtgtaa caagggtgaa cactatccca tatcaccagc tcaccgtctt tcattgccat 3960
   acggaactcc gggtgagcat tcatcaggcg ggcaagaatg tgaataaagg ccggataaaa 4020
   cttgtgctta tttttcttta cggtctttaa aaaggccgta atatccagct gaacggtctg    4080
   gttataggta cattgagcaa ctgactgaaa tgcctcaaaa tgttctttac gatgccattg    4140
   ggatatatca acggtggtat atccagtgat ttttttctcc attttagctt ccttagctcc    4200
   tgaaaatctc gataactcaa aaaatacgcc cggtagtgat cttatttcat tatggtgaaa    4260
   gttggaacct cacccgacgt ctaatgtgag ttagctcact cattaggcac cccaggcttt    4320
   acactttatg cttccggctc gtatgttgtg tggaattgtg agcggataac aatttcacac    4380
   aggaaacagc tatgaccatg attacgaatt                                    4410
<210> 18
   <211> 5020
   <212> DNA
   <213> artificial sequence
<220>
   <221> pMx9_Fab_GPC8
   <222> (1)..(5020)
<400> 18
   atcgtgctga cccagccgcc ttcagtgagt ggcgcaccag gtcagcgtgt gaccatctcg 60
   tgtagcggca gcagcagcaa cattggcagc aactatgtga gctggtacca gcagttgccc 120
   gggacggcgc cgaaactgct gatttatgat aacaaccagc gtccctcagg cgtgccggat 180
   cgttttagcg gatccaaaag cggcaccagc gcgagccttg cgattacggg cctgcaaagc 240
   gaagacgaag cggattatta ttgccagagc tatgacatgc ctcaggctgt gtttggcggc 300
   ggcacgaagt ttaaccgttc ttggccagcc gaaagccgca ccgagtgtga cgctgtttcc 360
   gccgagcagc gaagaattgc aggcgaacaa agcgaccctg gtgtgcctga ttagcgactt 420
   ttatccggga gccgtgacag tggcctggaa ggcagatagc agccccgtca aggcgggagt 480
   ggagaccacc acaccctcca aacaaagcaa caacaagtac gcggccagca gctatctgag 540
   cctgacgcct gagcagtgga agtcccacag aagctacagc tgccaggtca cgcatgaggg 600
   gagcaccgtg gaaaaaaccg ttgcgccgac tgaggcctga taagcatgcg taggagaaaa 660
   taaaatgaaa caaagcacta ttgcactggc actcttaccg ttgctcttca cccctgttac 720
   caaagcccag gtgcaattga aagaaagcgg cccggccctg gtgaaaccga cccaaaccct 780
   gaccctgacc tgtacctttt ccggatttag cctgtccacg tctggcgttg gcgtgggctg 840
   gattcgccag ccgcctggga aagccctcga gtggctggct ctgattgatt gggatgatga 900
   taagtattat agcaccagcc tgaaaacgcg tctgaccatt agcaaagata cttcgaaaaa 960
   tcaggtggtg ctgactatga ccaacatgga cccggtggat acggccacct attattgcgc 1020
   gcgttctcct cgttatcgtg gtgcttttga ttattggggc caaggcaccc tggtgacggt 1080
   tagctcagcg tcgaccaaag gtccaagcgt gtttccgctg gctccgagca gcaaaagcac 1140
   cagcggcggc acggctgccc tgggctgcct ggttaaagat tatttcccgg aaccagtcac 1200
   cgtgagctgg aacagcgggg cgctgaccag cggcgtgcat acctttccgg cggtgctgca 1260
   aagcagcggc ctgtatagcc tgagcagcgt tgtgaccgtg ccgagcagca gcttaggcac 1320
   tcagacctat atttgcaacg tgaaccataa accgagcaac accaaagtgg ataaaaaagt 1380
   ggaaccgaaa agcgaattcg actataaaga tgacgatgac aaaggcgcgc cgtggagcca 1440
   cccgcagttt gaaaaatgat aagcttgacc tgtgaagtga aaaatggcgc agattgtgcg 1500
   acattttttt tgtctgccgt ttaattaaag gggggggggg gccggcctgg gggggggtgt 1560
   acatgaaatt gtaaacgtta atattttgtt aaaattcgcg ttaaattttt gttaaatcag 1620
   ctcatttttt aaccaatagg ccgaaatcgg caaaatccct tataaatcaa aagaatagac 1680
   cgagataggg ttgagtgttg ttccagtttg gaacaagagt ccactattaa agaacgtgga 1740
   ctccaacgtc aaagggcgaa aaaccgtcta tcagggcgat ggcccactac gagaaccatc 1800
   accctaatca agttttttgg ggtcgaggtg ccgtaaagca ctaaatcgga accctaaagg 1860
   gagcccccga tttagagctt gacggggaaa gccggcgaac gtggcgagaa aggaagggaa 1920
   gaaagcgaaa ggagcgggcg ctagggcgct ggcaagtgta gcggtcacgc tgcgcgtaac 1980
   caccacaccc gccgcgctta atgcgccgct acagggcgcg tgctagacta gtgtttaaac 2040
   cggaccgggg gggggcttaa gtgggctgca aaacaaaacg gcctcctgtc aggaagccgc 2100
   ttttatcggg tagcctcact gcccgctttc cagtcgggaa acctgtcgtg ccagctgcat 2160
   cagtgaatcg gccaacgcgc ggggagaggc ggtttgcgta ttgggagcca gggtggtttt 2220
   tcttttcacc agtgagacgg gcaacagctg attgcccttc accgcctggc cctgagagag 2280
   ttgcagcaag cggtccacgc tggtttgccc cagcaggcga aaatcctgtt tgatggtggt 2340
   cagcggcggg atataacatg agctgtcctc ggtatcgtcg tatcccacta ccgagatgtc 2400
   cgcaccaacg cgcagcccgg actcggtaat ggcacgcatt gcgcccagcg ccatctgatc 2460
   gttggcaacc agcatcgcag tgggaacgat gccctcattc agcatttgca tggtttgttg 2520
   aaaaccggac atggcactcc agtcgccttc ccgttccgct atcggctgaa tttgattgcg 2580
   agtgagatat ttatgccagc cagccagacg cagacgcgcc gagacagaac ttaatgggcc 2640
   agctaacagc gcgatttgct ggtggcccaa tgcgaccaga tgctccacgc ccagtcgcgt 2700
   accgtcctca tgggagaaaa taatactgtt gatgggtgtc tggtcagaga catcaagaaa 2760
   taacgccgga acattagtgc aggcagcttc cacagcaata gcatcctggt catccagcgg 2820
   atagttaata atcagcccac tgacacgttg cgcgagaaga ttgtgcaccg ccgctttaca 2880
   ggcttcgacg ccgcttcgtt ctaccatcga cacgaccacg ctggcaccca gttgatcggc 2940
   gcgagattta atcgccgcga caatttgcga cggcgcgtgc agggccagac tggaggtggc 3000
   aacgccaatc agcaacgact gtttgcccgc cagttgttgt gccacgcggt taggaatgta 3060
   attcagctcc gccatcgccg cttccacttt ttcccgcgtt ttcgcagaaa cgtggctggc 3120
   ctggttcacc acgcgggaaa cggtctgata agagacaccg gcatactctg cgacatcgta 3180
   taacgttact ggtttcacat tcaccaccct gaattgactc tcttccgggc gctatcatgc 3240
   cataccgcga aaggttttgc gccattcgat gctagccatg tgagcaaaag gccagcaaaa 3300
   ggccaggaac cgtaaaaagg ccgcgttgct ggcgtttttc cataggctcc gcccccctga 3360
   cgagcatcac aaaaatcgac gctcaagtca gaggtggcga aacccgacag gactataaag 3420
   ataccaggcg tttccccctg gaagctccct cgtgcgctct cctgttccga ccctgccgct 3480
   taccggatac ctgtccgcct ttctcccttc gggaagcgtg gcgctttctc atagctcacg 3540
   ctgtaggtat ctcagttcgg tgtaggtcgt tcgctccaag ctgggctgtg tgcacgaacc 3600
   ccccgttcag cccgaccgct gcgccttatc cggtaactat cgtcttgagt ccaacccggt 3660
   aagacacgac ttatcgccac tggcagcagc cactggtaac aggattagca gagcgaggta 3720
   tgtaggcggt gctacagagt tcttgaagtg gtggcctaac tacggctaca ctagaagaac 3780
   agtatttggt atctgcgctc tgctgtagcc agttaccttc ggaaaaagag ttggtagctc 3840
   ttgatccggc aaacaaacca ccgctggtag cggtggtttt tttgtttgca agcagcagat 3900
   tacgcgcaga aaaaaaggat ctcaagaaga tcctttgatc ttttctacgg ggtctgacgc 3960
   tcagtggaac gaaaactcac gttaagggat tttggtcaga tctagcacca ggcgtttaag 4020
   ggcaccaata actgccttaa aaaaattacg ccccgccctg ccactcatcg cagtactgtt 4080
   gtaattcatt aagcattctg ccgacatgga agccatcaca aacggcatga tgaacctgaa 4140
   tcgccagcgg catcagcacc ttgtcgcctt gcgtataata tttgcccata gtgaaaacgg 4200
   gggcgaagaa gttgtccata ttggctacgt ttaaatcaaa actggtgaaa ctcacccagg 4260
   gattggctga gacgaaaaac atattctcaa taaacccttt agggaaatag gccaggtttt 4320
   caccgtaaca cgccacatct tgcgaatata tgtgtagaaa ctgccggaaa tcgtcgtggt 4380
   attcactcca gagcgatgaa aacgtttcag tttgctcatg gaaaacggtg taacaagggt 4440
   gaacactatc ccatatcacc agctcaccgt ctttcattgc catacggaac tccgggtgag 4500
   cattcatcag gcgggcaaga atgtgaataa aggccggata aaacttgtgc ttatttttct 4560
   ttacggtctt taaaaaggcc gtaatatcca gctgaacggt ctggttatag gtacattgag 4620
   caactgactg aaatgcctca aaatgttctt tacgatgcca ttgggatata tcaacggtgg 4680
   tatatccagt gatttttttc tccattttag cttccttagc tcctgaaaat ctcgataact 4740
   caaaaaatac gcccggtagt gatcttattt cattatggtg aaagttggaa cctcacccga 4800
   cgtctaatgt gagttagctc actcattagg caccccaggc tttacacttt atgcttccgg 4860
   ctcgtatgtt gtgtggaatt gtgagcggat aacaatttca cacaggaaac agctatgacc 4920
   atgattacga atttctagat aacgagggca aaaaatgaaa aagacagcta tcgcgattgc 4980
   agtggcactg gctggtttcg ctaccgtagc gcaggccgat 5020
<210> 19
   <211> 4145
   <212> DNA
   <213> artificial sequence
<220>
   <221> pMORPH18_Fab_GPC8
   <222> (1)..(4145)
<400> 19
   tcagataacg agggcaaaaa atgaaaaaga cagctatcgc gattgcagtg gcactggctg      60
   gtttcgctac cgtagcgcag gccgatatcg tgctgaccca gccgccttca gtgagtggcg     120
   caccaggtca gcgtgtgacc atctcgtgta gcggcagcag cagcaacatt ggcagcaact     180
   atgtgagctg gtaccagcag ttgcccggga cggcgccgaa actgctgatt tatgataaca     240
   accagcgtcc ctcaggcgtg ccggatcgtt ttagcggatc caaaagcggc accagcgcga     300
   gccttgcgat tacgggcctg caaagcgaag acgaagcgga ttattattgc cagagctatg     360
   acatgcctca ggctgtgttt ggcggcggca cgaagtttaa ccgttcttgg ccagccgaaa     420
   gccgcaccga gtgtgacgct gtttccgccg agcagcgaag aattgcaggc gaacaaagcg     480
   accctggtgt gcctgattag cgacttttat ccgggagccg tgacagtggc ctggaaggca     540
   gatagcagcc ccgtcaaggc gggagtggag accaccacac cctccaaaca aagcaacaac     600
   aagtacgcgg ccagcagcta tctgagcctg acgcctgagc agtggaagtc ccacagaagc     660
   tacagctgcc aggtcacgca tgaggggagc accgtggaaa aaaccgttgc gccgactgag     720
   gcctgataag catgcgtagg agaaaataaa atgaaacaaa gcactattgc actggcactc     780
   ttaccgttgc tcttcacccc tgttaccaaa gcccaggtgc aattgaaaga aagcggcccg     840
   gccctggtga aaccgaccca aaccctgacc ctgacctgta ccttttccgg atttagcctg     900
   tccacgtctg gcgttggcgt gggctggatt cgccagccgc ctgggaaagc cctcgagtgg     960
   ctggctctga ttgattggga tgatgataag tattatagca ccagcctgaa aacgcgtctg    1020
   accattagca aagatacttc gaaaaatcag gtggtgctga ctatgaccaa catggacccg    1080
   gtggatacgg ccacctatta ttgcgcgcgt tctcctcgtt atcgtggtgc ttttgattat    1140
   tggggccaag gcaccctggt gacggttagc tcagcgtcga ccaaaggtcc aagcgtgttt    1200
   ccgctggctc cgagcagcaa aagcaccagc ggcggcacgg ctgccctggg ctgcctggtt    1260
   aaagattatt tcccggaacc agtcaccgtg agctggaaca gcggggcgct gaccagcggc    1320
   gtgcatacct ttccggcggt gctgcaaagc agcggcctgt atagcctgag cagcgttgtg    1380
   accgtgccga gcagcagctt aggcactcag acctatattt gcaacgtgaa ccataaaccg    1440
   agcaacacca aagtggataa aaaagtggaa ccgaaaagcg aattcggggg agggagcggg 1500
   agcggtgatt ttgattatga aaagatggca aacgctaata agggggctat gaccgaaaat 1560
   gccgatgaaa acgcgctaca gtctgacgct aaaggcaaac ttgattctgt cgctactgat 1620
   tacggtgctg ctatcgatgg tttcattggt gacgtttccg gccttgctaa tggtaatggt 1680
   gctactggtg attttgctgg ctctaattcc caaatggctc aagtcggtga cggtgataat 1740
   tcacctttaa tgaataattt ccgtcaatat ttaccttccc tccctcaatc ggttgaatgt 1800
   cgcccttttg tctttggcgc tggtaaacca tatgaatttt ctattgattg tgacaaaata 1860
   aacttattcc gtggtgtctt tgcgtttctt ttatatgttg ccacctttat gtatgtattt 1920
   tctacgtttg ctaacatact gcgtaataag gagtcttgat aagcttgacc tgtgaagtga 1980
   aaaatggcgc agattgtgcg acattttttt tgtctgccgt ttaatgaaat tgtaaacgtt 2040
   aatattttgt taaaattcgc gttaaatttt tgttaaatca gctcattttt taaccaatag 2100
   gccgaaatcg gcaaaatccc ttataaatca aaagaataga ccgagatagg gttgagtgtt 2160
   gttccagttt ggaacaagag tccactatta aagaacgtgg actccaacgt caaagggcga 2220
   aaaaccgtct atcagggcga tggcccacta cgagaaccat caccctaatc aagttttttg 2280
   gggtcgaggt gccgtaaagc actaaatcgg aaccctaaag ggagcccccg atttagagct 2340
   tgacggggaa agccggcgaa cgtggcgaga aaggaaggga agaaagcgaa aggagcgggc 2400
   gctagggcgc tggcaagtgt agcggtcacg ctgcgcgtaa ccaccacacc cgccgcgctt 2460
   aatgcgccgc tacagggcgc gtgctagcca tgtgagcaaa aggccagcaa aaggccagga 2520
   accgtaaaaa ggccgcgttg ctggcgtttt tccataggct ccgcccccct gacgagcatc 2580
   acaaaaatcg acgctcaagt cagaggtggc gaaacccgac aggactataa agataccagg 2640
   cgtttccccc tggaagctcc ctcgtgcgct ctcctgttcc gaccctgccg cttaccggat 2700
   acctgtccgc ctttctccct tcgggaagcg tggcgctttc tcatagctca cgctgtaggt 2760
   atctcagttc ggtgtaggtc gttcgctcca agctgggctg tgtgcacgaa ccccccgttc 2820
   agtccgaccg ctgcgcctta tccggtaact atcgtcttga gtccaacccg gtaagacacg 2880
   acttatcgcc actggcagca gccactggta acaggattag cagagcgagg tatgtaggcg 2940
   gtgctacaga gttcttgaag tggtggccta actacggcta cactagaaga acagtatttg 3000
   gtatctgcgc tctgctgtag ccagttacct tcggaaaaag agttggtagc tcttgatccg 3060
   gcaaacaaac caccgctggt agcggtggtt tttttgtttg caagcagcag attacgcgca 3120
   gaaaaaaagg atctcaagaa gatcctttga tcttttctac ggggtctgac gctcagtgga 3180
   acgaaaactc acgttaaggg attttggtca gatctagcac caggcgttta agggcaccaa 3240
   taactgcctt aaaaaaatta cgccccgccc tgccactcat cgcagtactg ttgtaattca 3300
   ttaagcattc tgccgacatg gaagccatca caaacggcat gatgaacctg aatcgccagc 3360
   ggcatcagca ccttgtcgcc ttgcgtataa tatttgccca tagtgaaaac gggggcgaag 3420
   aagttgtcca tattggctac gtttaaatca aaactggtga aactcaccca gggattggct 3480
   gagacgaaaa acatattctc aataaaccct ttagggaaat aggccaggtt ttcaccgtaa 3540
   cacgccacat cttgcgaata tatgtgtaga aactgccgga aatcgtcgtg gtattcactc 3600
   cagagcgatg aaaacgtttc agtttgctca tggaaaacgg tgtaacaagg gtgaacacta 3660
   tcccatatca ccagctcacc gtctttcatt gccatacgga actccgggtg agcattcatc 3720
   aggcgggcaa gaatgtgaat aaaggccgga taaaacttgt gcttattttt ctttacggtc 3780
   tttaaaaagg ccgtaatatc cagctgaacg gtctggttat aggtacattg agcaactgac 3840
   tgaaatgcct caaaatgttc tttacgatgc cattgggata tatcaacggt ggtatatcca 3900
   gtgatttttt tctccatttt agcttcctta gctcctgaaa atctcgataa ctcaaaaaat 3960
   acgcccggta gtgatcttat ttcattatgg tgaaagttgg aacctcaccc gacgtctaat 4020
   gtgagttagc tcactcatta ggcaccccag gctttacact ttatgcttcc ggctcgtatg 4080
   ttgtgtggaa ttgtgagcgg ataacaattt cacacaggaa acagctatga ccatgattac 4140
   gaatt 4145
<210> 20
   <211> 120
   <212> PRT
   <213> artificial sequence
<220>
   <221> MS-GPC1-VH
   <222> (1)..(120)
<400> 20
   Gln Val Gln Leu Lys Glu Ser Gly Pro Ala Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Asp Trp Asp Asp Asp Lys Tyr Tyr Ser Thr Ser
       50                  55                  60
   Leu Lys Thr Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Gln Tyr Gly His Arg Gly Gly Phe Asp His Trp Gly Gln
               100                 105                 110
   Gly Thr Leu Val Thr Val Ser Ser
           115                 120
<210> 21
   <211> 109
   <212> PRT
   <213> artificial sequence
<220>
   <221> MS-GPC1-VL
   <222> (1)..(109)
<400> 21
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Tyr Val Ser Trp Tyr Gln Gln Leu Pro Gly Thr Ala Pro Lys Leu Leu
           35                  40                  45
   Ile Tyr Asp Asn Asn Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ser Tyr Asp Phe Asn Glu
                   85                  90                  95
   Ser Val Phe Gly Gly Gly Thr Lys Leu Thr Val Leu Gly
               100                 105
<210> 22
   <211> 118
   <212> PRT
   <213> artificial sequence
<220>
   <221> MS-GPC6-VH
   <222> (1) .. (118)
<400> 22
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Tyr Gly Arg Tyr Ser Pro Asp Leu Trp Gly Gln Gly Thr
               100                 105                 110
   Leu Val Thr Val Ser Ser
           115
<210> 23
   <211> 110
   <212> PRT
   <213> artificial sequence
<220>
   <221> MS-GPC6-VL
   <222> (1)..(110)
<400> 23
   Asp Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Tyr Ser Asn Leu Pro
                   85                  90                  95
   Phe Thr Phe Gly Gln Gly Thr Lys Val Glu Ile Lys Arg Thr
               100                 105                 110
<210> 24
   <211> 120
   <212> PRT
   <213> artificial sequence
<220>
   <221> MS-GPC8-VH
   <222> (1)..(120)
<400> 24
   Gln Val Gln Leu Lys Glu Ser Gly Pro Ala Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Asp Trp Asp Asp Asp Lys Tyr Tyr Ser Thr Ser
       50                  55                  60
   Leu Lys Thr Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Ser Pro Arg Tyr Arg Gly Ala Phe Asp Tyr Trp Gly Gln
               100                 105                 110
   Gly Thr Leu Val Thr Val Ser Ser
           115                 120
<210> 25
   <211> 109
   <212> PRT
   <213> artificial sequence
<220>
   <221> MS-GPC8-VL
   <222> (1)..(109)
<400> 25
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Tyr Val Ser Trp Tyr Gln Gln Leu Pro Gly Thr Ala Pro Lys Leu Leu
           35                  40                  45
   Ile Tyr Asp Asn Asn Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ser Tyr Asp Met Pro Gln
                   85                  90                  95
   Ala Val Phe Gly Gly Gly Thr Lys Leu Thr Val Leu Gly
               100                 105
<210> 26
   <211> 120
   <212> PRT
   <213> artificial sequence
<220>
   <221> MS-GPC10-VH
   <222> (1)..(120)
<400> 26
   Gln Val Gln Leu Lys Glu Ser Gly Pro Ala Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Asp Trp Asp Asp Asp Lys Tyr Tyr Ser Thr Ser
   50 55 60
   Leu Lys Thr Arg Leu Thr Ile Ser Lys Asp Thr Ser Lys Asn Gln Val
   65 70 75 80
   Val Leu Thr Met Thr Asn Met Asp Pro Val Asp Thr Ala Thr Tyr Tyr
   85 90 95
   Cys Ala Arg Gln Leu His Tyr Arg Gly Gly Phe Asp Leu Trp Gly Gln
   100 105 110
   Gly Thr Leu Val Thr Val Ser Ser
   115 120
<210> 27
   <211> 109
   <212> PRT
   <213> artificial sequence
<220>
   <221> MS-GPC10-VL
   <222> (1)..(109)
<400> 27
   Asp Ile Val Leu Thr Gln Pro Pro Ser Val Ser Gly Ala Pro Gly Gln
   1 5 10 15
   Arg Val Thr Ile Ser Cys Ser Gly Ser Ser Ser Asn Ile Gly Ser Asn
   20 25 30
   Tyr Val Ser Trp Tyr Gln Gln Leu Pro Gly Thr Ala Pro Lys Leu Leu
   35 40 45
   Ile Tyr Asp Asn Asn Gln Arg Pro Ser Gly Val Pro Asp Arg Phe Ser
   50 55 60
   Gly Ser Lys Ser Gly Thr Ser Ala Ser Leu Ala Ile Thr Gly Leu Gln
   65 70 75 80
   Ser Glu Asp Glu Ala Asp Tyr Tyr Cys Gln Ser Tyr Asp Leu Thr Met
   85 90 95
   Gly Val Phe Gly Gly Gly Thr Lys Leu Thr Val Leu Gly
   100 105
<210> 28
   <211> 109
   <212> PRT
   <213> artificial sequence
<220>
   <221> MS-GPC8-6-VL
   <222> (1)..(109)
<400> 28
   Asp Ile Val Leu Thr Gln Pro Pro Ser Val Ser Gly Ala Pro Gly Gln
   1 5 10 15
   Arg Val Thr Ile Ser Cys Ser Gly Ser Ser Ser Asn Ile Gly Ser Asn
   20 25 30
   Tyr Val Ser Trp Tyr Gln Gln Leu Pro Gly Thr Ala Pro Lys Leu Leu
   35 40 45
   Ile Tyr Asp Asn Asn Gln Arg Pro Ser Gly Val Pro Asp Arg Phe Ser
   50 55 60
   Gly Ser Lys Ser Gly Thr Ser Ala Ser Leu Ala Ile Thr Gly Leu Gln
   65 70 75 80
   Ser Glu Asp Glu Ala Asp Tyr Tyr Cys Gln Ser Tyr Asp Tyr Asp His
   85 90 95
   Tyr Val Phe Gly Gly Gly Thr Lys Leu Thr Val Leu Gly
   100 105
<210> 29
   <211> 109
   <212> PRT
   <213> artificial sequence
<220>
   <221> MS-GPC8-17-VL
   <222> (1)..(109)
<400> 29
   Asp Ile Val Leu Thr Gln Pro Pro Ser Val Ser Gly Ala Pro Gly Gln
   1 5 10 15
   Arg Val Thr Ile Ser Cys Ser Gly Ser Ser Ser Asn Ile Gly Ser Asn
   20 25 30
   Tyr Val Ser Trp Tyr Gln Gln Leu Pro Gly Thr Ala Pro Lys Leu Leu
   35 40 45
   Ile Tyr Asp Asn Asn Gln Arg Pro Ser Gly Val Pro Asp Arg Phe Ser
   50 55 60
   Gly Ser Lys Ser Gly Thr Ser Ala Ser Leu Ala Ile Thr Gly Leu Gln
   65 70 75 80
   Ser Glu Asp Glu Ala Asp Tyr Tyr Cys Gln Ser Tyr Asp Phe Ser Val
   85 90 95
   Tyr Val Phe Gly Gly Gly Thr Lys Leu Thr Val Leu Gly
   100 105

### SEQUENCE LISTING

<110> GPC Biotech AG
   MorphoSys AG
<120> Human peptides/proteins causing or leading to the killing of cells including lymphoid tumor cells
<130> killing antibody
<140> 00110065.0
   <141> 2000-05-12
<160> 29
<170> PatentIn version 3.0
<210> 1
   <211> 10
   <212> PRT
   <213> artificial sequence
<220>
   <221> VHconCDR3
   <222> (1)..(10)
   <223> "X" represents any amino acid residue
<400> 1
   Xaa Xaa Xaa Xaa Arg Gly Xaa Phe Asp Xaa
   1               5                   10
<210> 2
   <211> 8
   <212> PRT
   <213> artificial sequence
<220>
   <221> VLconCDR3
   <222> (1)..(8)
   <223> "X" represents any amino acid residue
<400> 2
   Gln Ser Tyr Asp Xaa Xaa Xaa Xaa
   1               5
<210> 3
   <211> 8
   <212> PRT
   <213> artificial sequence
<220>
   <221> Streptaq
   <222> (1)..(8)
<400> 3
   Trp Ser His Pro Gln Phe Glu Lys
   1 5
<210> 4
   <211> 17
   <212> DNA
   <213> artificial sequence
<220>
   <221> PrimerCRT5
   <222> (1)..(17)
<400> 4
   gtggtggttc cgatatc 17
<210> 5
   <211> 44
   <212> DNA
   <213> artificial sequence
<220>
   <221> PrimerCRT6
   <222> (1)..(44)
<400> 5
   agcgtcacac tcggtgcggc tttcggctgg ccaagaacgg gtta 44
<210> 6
   <211> 10
   <212> PRT
   <213> artificial sequence
<220>
   <221> MS-GPC1-VH-CDR3
   <222> (1)..(10)
<400> 6
   Gln Tyr Gly His Arg Gly Gly Phe Asp His
   1 5 10
<210> 7
   <211> 8
   <212> PRT
   <213> artificial sequence
<220>
   <221> MS-GPC1-VL-CDR3
   <222> (1)..(8)
<400> 7
   Gln Ser Tyr Asp Phe Asn Glu Ser
   1               5
<210> 8
   <211> 9
   <212> PRT
   <213> artificial sequence
<220>
   <221> MS-GPC6-VH-CDR3
   <222> (1)..(9)
<400> 8
   Gly Tyr Gly Arg Tyr Ser Pro Asp Leu
   1               5
<210> 9
   <211> 8
   <212> PRT
   <213> artificial sequence
<220>
   <221> MS-GPC6-VL-CDR3
   <222> (1)..(8)
<400> 9
   Gln Gln Tyr Ser Asn Leu Pro Phe
   1               5
<210> 10
   <211> 10
   <212> PRT
   <213> artificial sequence
<220>
   <221> MS-GPC8-VH-CDR3
   <222> (1)..(10)
<400> 10
   Ser Pro Arg Tyr Arg Gly Ala Phe Asp Tyr
   1               5                   10
<210> 11
   <211> 8
   <212> PRT
   <213> artificial sequence
<220>
   <221> MS-GPC8-VL-CDR3
   <222> (1)..(8)
<400> 11
   Gln Ser Tyr Asp Met Pro Gln Ala
   1               5
<210> 12
   <211> 10
   <212> PRT
   <213> artificial sequence
<220>
   <221> MS-GPC10-VH-CDR3
   <222> (1)..(10)
<400> 12
   Gln Leu His Tyr Arg Gly Gly Phe Asp Leu
   1               5                   10
<210> 13
   <211> 8
   <212> PRT
   <213> artificial sequence
<220>
   <221> MS-GPC10-VL-CDR3
   <222> (1)..(8)
<400> 13
   Gln Ser Tyr Asp Leu Thr Met Gly
   1               5
<210> 14
   <211> 8
   <212> PRT
   <213> artificial sequence
<220>
   <221> MS-GPC8-6-VL-CDR3
   <222> (1)..(8)
<400> 14
   Gln Ser Tyr Asp Tyr Asp His Tyr
   1 5
<210> 15
   <211> 8
   <212> PRT
   <213> artificial sequence
<220>
   <221> MS-GPC8-17-VL-CDR3
   <222> (1)..(8)
<400> 15
   Gln Ser Tyr Asp Phe Ser Val Tyr
   1 5
<210> 16
   <211> 3548
   <212> DNA
   <213> artificial sequence
<220>
   <221> pMORPH13_scFv
   <222> (1)..(3548)
<400> 16
   agagcatgcg taggagaaaa taaaatgaaa caaagcacta ttgcactggc actcttaccg 60
   ttgctcttca cccctgttac caaagccgac tacaaagatg aagtgcaatt ggttcagtct 120
   ggcgcggaag tgaaaaaacc gggcagcagc gtgaaagtga gctgcaaagc ctccggaggc 180
   acttttagca gctatgcgat tagctgggtg cgccaagccc ctgggcaggg tctcgagtgg 240
   atgggcggca ttattccgat ttttggcacg gcgaactacg cgcagaagtt tcagggccgg 300
   gtgaccatta ccgcggatga aagcaccagc accgcgtata tggaactgag cagcctgcgt 360
   agcgaagata cggccgtgta ttattgcgcg cgttattatg atcgtatgta taatatggat 420
   tattggggcc aaggcaccct ggtgacggtt agctcagcgg gtggcggttc tggcggcggt    480
   gggagcggtg gcggtggttc tggcggtggt ggttccgata tcgaactgac ccagccgcct    540
   tcagtgagcg ttgcaccagg tcagaccgcg cgtatctcgt gtagcggcga tgcgctgggc    600
   gataaatacg cgagctggta ccagcagaaa cccgggcagg cgccagttct ggtgatttat    660
   gatgattctg accgtccctc aggcatcccg gaacgcttta gcggatccaa cagcggcaac    720
   accgcgaccc tgaccattag cggcactcag gcggaagacg aagcggatta ttattgccag    780
   agctatgacg ctcatatgcg tcctgtgttt ggcggcggca cgaagttaac cgttcttggc    840
   caggaattcg agcagaagct gatctctgag gaggatctga actagggtgg tggctctggt    900
   tccggtgatt ttgattatga aaagatggca aacgctaata agggggctat gaccgaaaat    960
   gccgatgaaa acgcgctaca gtctgacgct aaaggcaaac ttgattctgt cgctactgat   1020
   tacggtgctg ctatcgatgg tttcattggt gacgtttccg gccttgctaa tggtaatggt   1080
   gctactggtg attttgctgg ctctaattcc caaatggctc aagtcggtga cggtgataat   1140
   tcacctttaa tgaataattt ccgtcaatat ttaccttccc tccctcaatc ggttgaatgt   1200
   cgcccttttg tctttggcgc tggtaaacca tatgaatttt ctattgattg tgacaaaata   1260
   aacttattcc gtggtgtctt tgcgtttctt ttatatgttg ccacctttat gtatgtattt   1320
   tctacgtttg ctaacatact gcgtaataag gagtcttgat aagcttgacc tgtgaagtga   1380
   aaaatggcgc agattgtgcg acattttttt tgtctgccgt ttaatgaaat tgtaaacgtt   1440
   aatattttgt taaaattcgc gttaaatttt tgttaaatca gctcattttt taaccaatag   1500
   gccgaaatcg gcaaaatccc ttataaatca aaagaataga ccgagatagg gttgagtgtt   1560
   gttccagttt ggaacaagag tccactatta aagaacgtgg actccaacgt caaagggcga   1620
   aaaaccgtct atcagggcga tggcccacta cgagaaccat caccctaatc aagttttttg   1680
   gggtcgaggt gccgtaaagc actaaatcgg aaccctaaag ggagcccccg atttagagct   1740
   tgacggggaa agccggcgaa cgtggcgaga aaggaaggga agaaagcgaa aggagcgggc   1800
   gctagggcgc tggcaagtgt agcggtcacg ctgcgcgtaa ccaccacacc cgccgcgctt   1860
   aatgcgccgc tacagggcgc gtgctagcca tgtgagcaaa aggccagcaa aaggccagga   1920
   accgtaaaaa ggccgcgttg ctggcgtttt tccataggct ccgcccccct gacgagcatc   1980
   acaaaaatcg acgctcaagt cagaggtggc gaaacccgac aggactataa agataccagg   2040
   cgtttccccc tggaagctcc ctcgtgcgct ctcctgttcc gaccctgccg cttaccggat   2100
   acctgtccgc ctttctccct tcgggaagcg tggcgctttc tcatagctca cgctgtaggt   2160
   atctcagttc ggtgtaggtc gttcgctcca agctgggctg tgtgcacgaa ccccccgttc   2220
   agtccgaccg ctgcgcctta tccggtaact atcgtcttga gtccaacccg gtaagacacg   2280
   acttatcgcc actggcagca gccactggta acaggattag cagagcgagg tatgtaggcg   2340
   gtgctacaga gttcttgaag tggtggccta actacggcta cactagaaga acagtatttg   2400
   gtatctgcgc tctgctgtag ccagttacct tcggaaaaag agttggtagc tcttgatccg   2460
   gcaaacaaac caccgctggt agcggtggtt tttttgtttg caagcagcag attacgcgca   2520
   gaaaaaaagg atctcaagaa gatcctttga tcttttctac ggggtctgac gctcagtgga   2580
   acgaaaactc acgttaaggg attttggtca gatctagcac caggcgttta agggcaccaa   2640
   taactgcctt aaaaaaatta cgccccgccc tgccactcat cgcagtactg ttgtaattca   2700
   ttaagcattc tgccgacatg gaagccatca caaacggcat gatgaacctg aatcgccagc   2760
   ggcatcagca ccttgtcgcc ttgcgtataa tatttgccca tagtgaaaac gggggcgaag   2820
   aagttgtcca tattggctac gtttaaatca aaactggtga aactcaccca gggattggct   2880
   gagacgaaaa acatattctc aataaaccct ttagggaaat aggccaggtt ttcaccgtaa   2940
   cacgccacat cttgcgaata tatgtgtaga aactgccgga aatcgtcgtg gtattcactc   3000
   cagagcgatg aaaacgtttc agtttgctca tggaaaacgg tgtaacaagg gtgaacacta   3060
   tcccatatca ccagctcacc gtctttcatt gccatacgga actccgggtg agcattcatc   3120
   aggcgggcaa gaatgtgaat aaaggccgga taaaacttgt gcttattttt ctttacggtc   3180
   tttaaaaagg ccgtaatatc cagctgaacg gtctggttat aggtacattg agcaactgac   3240
   tgaaatgcct caaaatgttc tttacgatgc cattgggata tatcaacggt ggtatatcca   3300
   gtgatttttt tctccatttt agcttcctta gctcctgaaa atctcgataa ctcaaaaaat   3360
   acgcccggta gtgatcttat ttcattatgg tgaaagttgg aacctcaccc gacgtctaat   3420
   gtgagttagc tcactcatta ggcaccccag gctttacact ttatgcttcc ggctcgtatg   3480
   ttgtgtggaa ttgtgagcgg ataacaattt cacacaggaa acagctatga ccatgattac   3540
   gaatttct                                                            3548
<210> 17
   <211> 4410
   <212> DNA
   <213> artificial sequence
<220>
   <221> pMx7_FS_5D2
   <222> (1)..(4410)
<400> 17
   tctagagcat gcgtaggaga aaataaaatg aaacaaagca ctattgcact ggcactctta    60
   ccgttgctct tcacccctgt taccaaagcc gactacaaag atgaagtgca attggtggaa   120
   agcggcggcg gcctggtgca accgggcggc agcctgcgtc tgagctgcgc ggcctccgga   180
   tttaccttta gcagctatgc gatgagctgg gtgcgccaag cccctgggaa gggtctcgag   240
   tgggtgagcg cgattagcgg tagcggcggc agcacctatt atgcggatag cgtgaaaggc 300
   cgttttacca tttcacgtga taattcgaaa aacaccctgt atctgcaaat gaacagcctg 360
   cgtgcggaag atacggccgt gtattattgc gcgcgtgtta agaagcattt ttctcgtaag 420
   aattggtttg attattgggg ccaaggcacc ctggtgacgg ttagctcagc gggtggcggt 480
   tctggcggcg gtgggagcgg tggcggtggt tctggcggtg gtggttccga tatcgtgatg 540
   acccagagcc cactgagcct gccagtgact ccgggcgagc ctgcgagcat tagctgcaga 600
   agcagccaaa gcctgctgca tagcaacggc tataactatc tggattggta ccttcaaaaa 660
   ccaggtcaaa gcccgcagct attaatttat ctgggcagca accgtgccag tggggtcccg 720
   gatcgtttta gcggctctgg atccggcacc gattttaccc tgaaaattag ccgtgtggaa 780
   gctgaagacg tgggcgtgta ttattgccag cagcattata ccaccccgcc gacctttggc 840
   cagggtacga aagttgaaat taaacgtacg gaattcgact ataaagatga cgatgacaaa 900
   ggcgcgccgt ggagccaccc gcagtttgaa aaatgataag cttgacctgt gaagtgaaaa 960
   atggcgcaga ttgtgcgaca ttttttttgt ctgccgttta attaaagggg ggggggggcc 1020
   ggcctggggg ggggtgtaca tgaaattgta aacgttaata ttttgttaaa attcgcgtta 1080
   aatttttgtt aaatcagctc attttttaac caataggccg aaatcggcaa aatcccttat 1140
   aaatcaaaag aatagaccga gatagggttg agtgttgttc cagtttggaa caagagtcca 1200
   ctattaaaga acgtggactc caacgtcaaa gggcgaaaaa ccgtctatca gggcgatggc 1260
   ccactacgag aaccatcacc ctaatcaagt tttttggggt cgaggtgccg taaagcacta 1320
   aatcggaacc ctaaagggag cccccgattt agagcttgac ggggaaagcc ggcgaacgtg 1380
   gcgagaaagg aagggaagaa agcgaaagga gcgggcgcta gggcgctggc aagtgtagcg 1440
   gtcacgctgc gcgtaaccac cacacccgcc gcgcttaatg cgccgctaca gggcgcgtgc 1500
   tagactagtg tttaaaccgg accggggggg ggcttaagtg ggctgcaaaa caaaacggcc 1560
   tcctgtcagg aagccgcttt tatcgggtag cctcactgcc cgctttccag tcgggaaacc 1620
   tgtcgtgcca gctgcatcag tgaatcggcc aacgcgcggg gagaggcggt ttgcgtattg 1680
   ggagccaggg tggtttttct tttcaccagt gagacgggca acagctgatt gcccttcacc 1740
   gcctggccct gagagagttg cagcaagcgg tccacgctgg tttgccccag caggcgaaaa 1800
   tcctgtttga tggtggtcag cggcgggata taacatgagc tgtcctcggt atcgtcgtat 1860
   cccactaccg agatgtccgc accaacgcgc agcccggact cggtaatggc acgcattgcg 1920
   cccagcgcca tctgatcgtt ggcaaccagc atcgcagtgg gaacgatgcc ctcattcagc 1980
   atttgcatgg tttgttgaaa accggacatg gcactccagt cgccttcccg ttccgctatc 2040
   ggctgaattt gattgcgagt gagatattta tgccagccag ccagacgcag acgcgccgag 2100
   acagaactta atgggccagc taacagcgcg atttgctggt ggcccaatgc gaccagatgc 2160
   tccacgccca gtcgcgtacc gtcctcatgg gagaaaataa tactgttgat gggtgtctgg 2220
   tcagagacat caagaaataa cgccggaaca ttagtgcagg cagcttccac agcaatagca 2280
   tcctggtcat ccagcggata gttaataatc agcccactga cacgttgcgc gagaagattg 2340
   tgcaccgccg ctttacaggc ttcgacgccg cttcgttcta ccatcgacac gaccacgctg 2400
   gcacccagtt gatcggcgcg agatttaatc gccgcgacaa tttgcgacgg cgcgtgcagg 2460
   gccagactgg aggtggcaac gccaatcagc aacgactgtt tgcccgccag ttgttgtgcc 2520
   acgcggttag gaatgtaatt cagctccgcc atcgccgctt ccactttttc ccgcgttttc 2580
   gcagaaacgt ggctggcctg gttcaccacg cgggaaacgg tctgataaga gacaccggca 2640
   tactctgcga catcgtataa cgttactggt ttcacattca ccaccctgaa ttgactctct 2700
   tccgggcgct atcatgccat accgcgaaag gttttgcgcc attcgatgct agccatgtga 2760
   gcaaaaggcc agcaaaaggc caggaaccgt aaaaaggccg cgttgctggc gtttttccat 2820
   aggctccgcc cccctgacga gcatcacaaa aatcgacgct caagtcagag gtggcgaaac 2880
   ccgacaggac tataaagata ccaggcgttt ccccctggaa gctccctcgt gcgctctcct 2940
   gttccgaccc tgccgcttac cggatacctg tccgcctttc tcccttcggg aagcgtggcg 3000
   ctttctcata gctcacgctg taggtatctc agttcggtgt aggtcgttcg ctccaagctg 3060
   ggctgtgtgc acgaaccccc cgttcagccc gaccgctgcg ccttatccgg taactatcgt 3120
   cttgagtcca acccggtaag acacgactta tcgccactgg cagcagccac tggtaacagg 3180
   attagcagag cgaggtatgt aggcggtgct acagagttct tgaagtggtg gcctaactac 3240
   ggctacacta gaagaacagt atttggtatc tgcgctctgc tgtagccagt taccttcgga 3300
   aaaagagttg gtagctcttg atccggcaaa caaaccaccg ctggtagcgg tggttttttt 3360
   gtttgcaagc agcagattac gcgcagaaaa aaaggatctc aagaagatcc tttgatcttt 3420
   tctacggggt ctgacgctca gtggaacgaa aactcacgtt aagggatttt ggtcagatct 3480
   agcaccaggc gtttaagggc accaataact gccttaaaaa aattacgccc cgccctgcca 3540
   ctcatcgcag tactgttgta attcattaag cattctgccg acatggaagc catcacaaac 3600
   ggcatgatga acctgaatcg ccagcggcat cagcaccttg tcgccttgcg tataatattt 3660
   gcccatagtg aaaacggggg cgaagaagtt gtccatattg gctacgttta aatcaaaact 3720
   ggtgaaactc acccagggat tggctgagac gaaaaacata ttctcaataa accctttagg 3780
   gaaataggcc aggttttcac cgtaacacgc cacatcttgc gaatatatgt gtagaaactg 3840
   ccggaaatcg tcgtggtatt cactccagag cgatgaaaac gtttcagttt gctcatggaa 3900
   aacggtgtaa caagggtgaa cactatccca tatcaccagc tcaccgtctt tcattgccat 3960
   acggaactcc gggtgagcat tcatcaggcg ggcaagaatg tgaataaagg ccggataaaa 4020
   cttgtgctta tttttcttta cggtctttaa aaaggccgta atatccagct gaacggtctg 4080
   gttataggta cattgagcaa ctgactgaaa tgcctcaaaa tgttctttac gatgccattg 4140
   ggatatatca acggtggtat atccagtgat ttttttctcc attttagctt ccttagctcc 4200
   tgaaaatctc gataactcaa aaaatacgcc cggtagtgat cttatttcat tatggtgaaa 4260
   gttggaacct cacccgacgt ctaatgtgag ttagctcact cattaggcac cccaggcttt 4320
   acactttatg cttccggctc gtatgttgtg tggaattgtg agcggataac aatttcacac 4380
   aggaaacagc tatgaccatg attacgaatt 4410
<210> 18
   <211> 5020
   <212> DNA
   <213> artificial sequence
<220>
   <221> pMx9_Fab_GPC8
   <222> (1)..(5020)
<400> 18
   atcgtgctga cccagccgcc ttcagtgagt ggcgcaccag gtcagcgtgt gaccatctcg      60
   tgtagcggca gcagcagcaa cattggcagc aactatgtga gctggtacca gcagttgccc     120
   gggacggcgc cgaaactgct gatttatgat aacaaccagc gtccctcagg cgtgccggat     180
   cgttttagcg gatccaaaag cggcaccagc gcgagccttg cgattacggg cctgcaaagc     240
   gaagacgaag cggattatta ttgccagagc tatgacatgc ctcaggctgt gtttggcggc     300
   ggcacgaagt ttaaccgttc ttggccagcc gaaagccgca ccgagtgtga cgctgtttcc     360
   gccgagcagc gaagaattgc aggcgaacaa agcgaccctg gtgtgcctga ttagcgactt     420
   ttatccggga gccgtgacag tggcctggaa ggcagatagc agccccgtca aggcgggagt     480
   ggagaccacc acaccctcca aacaaagcaa caacaagtac gcggccagca gctatctgag     540
   cctgacgcct gagcagtgga agtcccacag aagctacagc tgccaggtca cgcatgaggg     600
   gagcaccgtg gaaaaaaccg ttgcgccgac tgaggcctga taagcatgcg taggagaaaa     660
   taaaatgaaa caaagcacta ttgcactggc actcttaccg ttgctcttca cccctgttac     720
   caaagcccag gtgcaattga aagaaagcgg cccggccctg gtgaaaccga cccaaaccct     780
   gaccctgacc tgtacctttt ccggatttag cctgtccacg tctggcgttg gcgtgggctg     840
   gattcgccag ccgcctggga aagccctcga gtggctggct ctgattgatt gggatgatga     900
   taagtattat agcaccagcc tgaaaacgcg tctgaccatt agcaaagata cttcgaaaaa     960
   tcaggtggtg ctgactatga ccaacatgga cccggtggat acggccacct attattgcgc    1020
   gcgttctcct cgttatcgtg gtgcttttga ttattggggc caaggcaccc tggtgacggt    1080
   tagctcagcg tcgaccaaag gtccaagcgt gtttccgctg gctccgagca gcaaaagcac    1140
   cagcggcggc acggctgccc tgggctgcct ggttaaagat tatttcccgg aaccagtcac 1200
   cgtgagctgg aacagcgggg cgctgaccag cggcgtgcat acctttccgg cggtgctgca 1260
   aagcagcggc ctgtatagcc tgagcagcgt tgtgaccgtg ccgagcagca gcttaggcac 1320
   tcagacctat atttgcaacg tgaaccataa accgagcaac accaaagtgg ataaaaaagt 1380
   ggaaccgaaa agcgaattcg actataaaga tgacgatgac aaaggcgcgc cgtggagcca 1440
   cccgcagttt gaaaaatgat aagcttgacc tgtgaagtga aaaatggcgc agattgtgcg 1500
   acattttttt tgtctgccgt ttaattaaag gggggggggg gccggcctgg gggggggtgt 1560
   acatgaaatt gtaaacgtta atattttgtt aaaattcgcg ttaaattttt gttaaatcag 1620
   ctcatttttt aaccaatagg ccgaaatcgg caaaatccct tataaatcaa aagaatagac 1680
   cgagataggg ttgagtgttg ttccagtttg gaacaagagt ccactattaa agaacgtgga 1740
   ctccaacgtc aaagggcgaa aaaccgtcta tcagggcgat ggcccactac gagaaccatc 1800
   accctaatca agttttttgg ggtcgaggtg ccgtaaagca ctaaatcgga accctaaagg 1860
   gagcccccga tttagagctt gacggggaaa gccggcgaac gtggcgagaa aggaagggaa 1920
   gaaagcgaaa ggagcgggcg ctagggcgct ggcaagtgta gcggtcacgc tgcgcgtaac 1980
   caccacaccc gccgcgctta atgcgccgct acagggcgcg tgctagacta gtgtttaaac 2040
   cggaccgggg gggggcttaa gtgggctgca aaacaaaacg gcctcctgtc aggaagccgc 2100
   ttttatcggg tagcctcact gcccgctttc cagtcgggaa acctgtcgtg ccagctgcat 2160
   cagtgaatcg gccaacgcgc ggggagaggc ggtttgcgta ttgggagcca gggtggtttt 2220
   tcttttcacc agtgagacgg gcaacagctg attgcccttc accgcctggc cctgagagag 2280
   ttgcagcaag cggtccacgc tggtttgccc cagcaggcga aaatcctgtt tgatggtggt 2340
   cagcggcggg atataacatg agctgtcctc ggtatcgtcg tatcccacta ccgagatgtc 2400
   cgcaccaacg cgcagcccgg actcggtaat ggcacgcatt gcgcccagcg ccatctgatc 2460
   gttggcaacc agcatcgcag tgggaacgat gccctcattc agcatttgca tggtttgttg 2520
   aaaaccggac atggcactcc agtcgccttc ccgttccgct atcggctgaa tttgattgcg 2580
   agtgagatat ttatgccagc cagccagacg cagacgcgcc gagacagaac ttaatgggcc 2640
   agctaacagc gcgatttgct ggtggcccaa tgcgaccaga tgctccacgc ccagtcgcgt 2700
   accgtcctca tgggagaaaa taatactgtt gatgggtgtc tggtcagaga catcaagaaa 2760
   taacgccgga acattagtgc aggcagcttc cacagcaata gcatcctggt catccagcgg 2820
   atagttaata atcagcccac tgacacgttg cgcgagaaga ttgtgcaccg ccgctttaca 2880
   ggcttcgacg ccgcttcgtt ctaccatcga cacgaccacg ctggcaccca gttgatcggc 2940
   gcgagattta atcgccgcga caatttgcga cggcgcgtgc agggccagac tggaggtggc 3000
   aacgccaatc agcaacgact gtttgcccgc cagttgttgt gccacgcggt taggaatgta 3060
   attcagctcc gccatcgccg cttccacttt ttcccgcgtt ttcgcagaaa cgtggctggc 3120
   ctggttcacc acgcgggaaa cggtctgata agagacaccg gcatactctg cgacatcgta 3180
   taacgttact ggtttcacat tcaccaccct gaattgactc tcttccgggc gctatcatgc 3240
   cataccgcga aaggttttgc gccattcgat gctagccatg tgagcaaaag gccagcaaaa 3300
   ggccaggaac cgtaaaaagg ccgcgttgct ggcgtttttc cataggctcc gcccccctga 3360
   cgagcatcac aaaaatcgac gctcaagtca gaggtggcga aacccgacag gactataaag 3420
   ataccaggcg tttccccctg gaagctccct cgtgcgctct cctgttccga ccctgccgct 3480
   taccggatac ctgtccgcct ttctcccttc gggaagcgtg gcgctttctc atagctcacg 3540
   ctgtaggtat ctcagttcgg tgtaggtcgt tcgctccaag ctgggctgtg tgcacgaacc 3600
   ccccgttcag cccgaccgct gcgccttatc cggtaactat cgtcttgagt ccaacccggt 3660
   aagacacgac ttatcgccac tggcagcagc cactggtaac aggattagca gagcgaggta 3720
   tgtaggcggt gctacagagt tcttgaagtg gtggcctaac tacggctaca ctagaagaac 3780
   agtatttggt atctgcgctc tgctgtagcc agttaccttc ggaaaaagag ttggtagctc 3840
   ttgatccggc aaacaaacca ccgctggtag cggtggtttt tttgtttgca agcagcagat 3900
   tacgcgcaga aaaaaaggat ctcaagaaga tcctttgatc ttttctacgg ggtctgacgc 3960
   tcagtggaac gaaaactcac gttaagggat tttggtcaga tctagcacca ggcgtttaag 4020
   ggcaccaata actgccttaa aaaaattacg ccccgccctg ccactcatcg cagtactgtt 4080
   gtaattcatt aagcattctg ccgacatgga agccatcaca aacggcatga tgaacctgaa 4140
   tcgccagcgg catcagcacc ttgtcgcctt gcgtataata tttgcccata gtgaaaacgg 4200
   gggcgaagaa gttgtccata ttggctacgt ttaaatcaaa actggtgaaa ctcacccagg 4260
   gattggctga gacgaaaaac atattctcaa taaacccttt agggaaatag gccaggtttt 4320
   caccgtaaca cgccacatct tgcgaatata tgtgtagaaa ctgccggaaa tcgtcgtggt 4380
   attcactcca gagcgatgaa aacgtttcag tttgctcatg gaaaacggtg taacaagggt 4440
   gaacactatc ccatatcacc agctcaccgt ctttcattgc catacggaac tccgggtgag 4500
   cattcatcag gcgggcaaga atgtgaataa aggccggata aaacttgtgc ttatttttct 4560
   ttacggtctt taaaaaggcc gtaatatcca gctgaacggt ctggttatag gtacattgag 4620
   caactgactg aaatgcctca aaatgttctt tacgatgcca ttgggatata tcaacggtgg 4680
   tatatccagt gatttttttc tccattttag cttccttagc tcctgaaaat ctcgataact 4740
   caaaaaatac gcccggtagt gatcttattt cattatggtg aaagttggaa cctcacccga 4800
   cgtctaatgt gagttagctc actcattagg caccccaggc tttacacttt atgcttccgg 4860
   ctcgtatgtt gtgtggaatt gtgagcggat aacaatttca cacaggaaac agctatgacc 4920
   atgattacga atttctagat aacgagggca aaaaatgaaa aagacagcta tcgcgattgc 4980
   agtggcactg gctggtttcg ctaccgtagc gcaggccgat 5020
<210> 19
   <211> 4145
   <212> DNA
   <213> artificial sequence
<220>
   <221> pMORPH18_Fab_GPC8
   <222> (1)..(4145)
<400> 19
   tcagataacg agggcaaaaa atgaaaaaga cagctatcgc gattgcagtg gcactggctg      60
   gtttcgctac cgtagcgcag gccgatatcg tgctgaccca gccgccttca gtgagtggcg     120
   caccaggtca gcgtgtgacc atctcgtgta gcggcagcag cagcaacatt ggcagcaact     180
   atgtgagctg gtaccagcag ttgcccggga cggcgccgaa actgctgatt tatgataaca     240
   accagcgtcc ctcaggcgtg ccggatcgtt ttagcggatc caaaagcggc accagcgcga     300
   gccttgcgat tacgggcctg caaagcgaag acgaagcgga ttattattgc cagagctatg     360
   acatgcctca ggctgtgttt ggcggcggca cgaagtttaa ccgttcttgg ccagccgaaa     420
   gccgcaccga gtgtgacgct gtttccgccg agcagcgaag aattgcaggc gaacaaagcg     480
   accctggtgt gcctgattag cgacttttat ccgggagccg tgacagtggc ctggaaggca     540
   gatagcagcc ccgtcaaggc gggagtggag accaccacac cctccaaaca aagcaacaac     600
   aagtacgcgg ccagcagcta tctgagcctg acgcctgagc agtggaagtc ccacagaagc     660
   tacagctgcc aggtcacgca tgaggggagc accgtggaaa aaaccgttgc gccgactgag     720
   gcctgataag catgcgtagg agaaaataaa atgaaacaaa gcactattgc actggcactc     780
   ttaccgttgc tcttcacccc tgttaccaaa gcccaggtgc aattgaaaga aagcggcccg     840
   gccctggtga aaccgaccca aaccctgacc ctgacctgta ccttttccgg atttagcctg      900
   tccacgtctg gcgttggcgt gggctggatt cgccagccgc ctgggaaagc cctcgagtgg     960
   ctggctctga ttgattggga tgatgataag tattatagca ccagcctgaa aacgcgtctg    1020
   accattagca aagatacttc gaaaaatcag gtggtgctga ctatgaccaa catggacccg    1080
   gtggatacgg ccacctatta ttgcgcgcgt tctcctcgtt atcgtggtgc ttttgattat    1140
   tggggccaag gcaccctggt gacggttagc tcagcgtcga ccaaaggtcc aagcgtgttt    1200
   ccgctggctc cgagcagcaa aagcaccagc ggcggcacgg ctgccctggg ctgcctggtt    1260
   aaagattatt tcccggaacc agtcaccgtg agctggaaca gcggggcgct gaccagcggc    1320
   gtgcatacct ttccggcggt gctgcaaagc agcggcctgt atagcctgag cagcgttgtg    1380
   accgtgccga gcagcagctt aggcactcag acctatattt gcaacgtgaa ccataaaccg    1440
   agcaacacca aagtggataa aaaagtggaa ccgaaaagcg aattcggggg agggagcggg 1500
   agcggtgatt ttgattatga aaagatggca aacgctaata agggggctat gaccgaaaat 1560
   gccgatgaaa acgcgctaca gtctgacgct aaaggcaaac ttgattctgt cgctactgat 1620
   tacggtgctg ctatcgatgg tttcattggt gacgtttccg gccttgctaa tggtaatggt 1680
   gctactggtg attttgctgg ctctaattcc caaatggctc aagtcggtga cggtgataat 1740
   tcacctttaa tgaataattt ccgtcaatat ttaccttccc tccctcaatc ggttgaatgt 1800
   cgcccttttg tctttggcgc tggtaaacca tatgaatttt ctattgattg tgacaaaata 1860
   aacttattcc gtggtgtctt tgcgtttctt ttatatgttg ccacctttat gtatgtattt 1920
   tctacgtttg ctaacatact gcgtaataag gagtcttgat aagcttgacc tgtgaagtga 1980
   aaaatggcgc agattgtgcg acattttttt tgtctgccgt ttaatgaaat tgtaaacgtt 2040
   aatattttgt taaaattcgc gttaaatttt tgttaaatca gctcattttt taaccaatag 2100
   gccgaaatcg gcaaaatccc ttataaatca aaagaataga ccgagatagg gttgagtgtt 2160
   gttccagttt ggaacaagag tccactatta aagaacgtgg actccaacgt caaagggcga 2220
   aaaaccgtct atcagggcga tggcccacta cgagaaccat caccctaatc aagttttttg 2280
   gggtcgaggt gccgtaaagc actaaatcgg aaccctaaag ggagcccccg atttagagct 2340
   tgacggggaa agccggcgaa cgtggcgaga aaggaaggga agaaagcgaa aggagcgggc 2400
   gctagggcgc tggcaagtgt agcggtcacg ctgcgcgtaa ccaccacacc cgccgcgctt 2460
   aatgcgccgc tacagggcgc gtgctagcca tgtgagcaaa aggccagcaa aaggccagga 2520
   accgtaaaaa ggccgcgttg ctggcgtttt tccataggct ccgcccccct gacgagcatc 2580
   acaaaaatcg acgctcaagt cagaggtggc gaaacccgac aggactataa agataccagg 2640
   cgtttccccc tggaagctcc ctcgtgcgct ctcctgttcc gaccctgccg cttaccggat 2700
   acctgtccgc ctttctccct tcgggaagcg tggcgctttc tcatagctca cgctgtaggt 2760
   atctcagttc ggtgtaggtc gttcgctcca agctgggctg tgtgcacgaa ccccccgttc 2820
   agtccgaccg ctgcgcctta tccggtaact atcgtcttga gtccaacccg gtaagacacg 2880
   acttatcgcc actggcagca gccactggta acaggattag cagagcgagg tatgtaggcg 2940
   gtgctacaga gttcttgaag tggtggccta actacggcta cactagaaga acagtatttg 3000
   gtatctgcgc tctgctgtag ccagttacct tcggaaaaag agttggtagc tcttgatccg 3060
   gcaaacaaac caccgctggt agcggtggtt tttttgtttg caagcagcag attacgcgca 3120
   gaaaaaaagg atctcaagaa gatcctttga tcttttctac ggggtctgac gctcagtgga 3180
   acgaaaactc acgttaaggg attttggtca gatctagcac caggcgttta agggcaccaa 3240
   taactgcctt aaaaaaatta cgccccgccc tgccactcat cgcagtactg ttgtaattca 3300
   ttaagcattc tgccgacatg gaagccatca caaacggcat gatgaacctg aatcgccagc 3360
   ggcatcagca ccttgtcgcc ttgcgtataa tatttgccca tagtgaaaac gggggcgaag   3420
   aagttgtcca tattggctac gtttaaatca aaactggtga aactcaccca gggattggct   3480
   gagacgaaaa acatattctc aataaaccct ttagggaaat aggccaggtt ttcaccgtaa   3540
   cacgccacat cttgcgaata tatgtgtaga aactgccgga aatcgtcgtg gtattcactc   3600
   cagagcgatg aaaacgtttc agtttgctca tggaaaacgg tgtaacaagg gtgaacacta   3660
   tcccatatca ccagctcacc gtctttcatt gccatacgga actccgggtg agcattcatc   3720
   aggcgggcaa gaatgtgaat aaaggccgga taaaacttgt gcttattttt ctttacggtc   3780
   tttaaaaagg ccgtaatatc cagctgaacg gtctggttat aggtacattg agcaactgac   3840
   tgaaatgcct caaaatgttc tttacgatgc cattgggata tatcaacggt ggtatatcca   3900
   gtgatttttt tctccatttt agcttcctta gctcctgaaa atctcgataa ctcaaaaaat   3960
   acgcccggta gtgatcttat ttcattatgg tgaaagttgg aacctcaccc gacgtctaat   4020
   gtgagttagc tcactcatta ggcaccccag gctttacact ttatgcttcc ggctcgtatg   4080
   ttgtgtggaa ttgtgagcgg ataacaattt cacacaggaa acagctatga ccatgattac   4140
   gaatt                                                               4145
<210> 20
   <211> 120
   <212> PRT
   <213> artificial sequence
<220>
   <221> MS-GPC1-VH
   <222> (1)..(120)
<400> 20
   Gln Val Gln Leu Lys Glu Ser Gly Pro Ala Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Asp Trp Asp Asp Asp Lys Tyr Tyr Ser Thr Ser
       50                  55                  60
   Leu Lys Thr Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Gln Tyr Gly His Arg Gly Gly Phe Asp His Trp Gly Gln
               100                 105                 110
   Gly Thr Leu Val Thr Val Ser Ser
           115                 120
<210> 21
   <211> 109
   <212> PRT
   <213> artificial sequence
<220>
   <221> MS-GPC1-VL
   <222> (1)..(109)
<400> 21
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Tyr Val Ser Trp Tyr Gln Gln Leu Pro Gly Thr Ala Pro Lys Leu Leu
           35                  40                  45
   Ile Tyr Asp Asn Asn Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ser Tyr Asp Phe Asn Glu
                   85                  90                  95
   Ser Val Phe Gly Gly Gly Thr Lys Leu Thr Val Leu Gly
               100                 105
<210> 22
   <211> 118
   <212> PRT
   <213> artificial sequence
<220>
   <221> MS-GPC6-VH
   <222> (1)..(118)
<400> 22
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Tyr Gly Arg Tyr Ser Pro Asp Leu Trp Gly Gln Gly Thr
               100                 105                 110
   Leu Val Thr Val Ser Ser
           115
<210> 23
   <211> 110
   <212> PRT
   <213> artificial sequence
<220>
   <221> MS-GPC6-VL
   <222> (1)..(110)
<400> 23
   Asp Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Tyr Ser Asn Leu Pro
                   85                  90                  95
   Phe Thr Phe Gly Gln Gly Thr Lys Val Glu Ile Lys Arg Thr
               100                 105                 110
<210> 24
   <211> 120
   <212> PRT
   <213> artificial sequence
<220>
   <221> MS-GPC8-VH
   <222> (1)..(120)
<400> 24
   Gln Val Gln Leu Lys Glu Ser Gly Pro Ala Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Asp Trp Asp Asp Asp Lys Tyr Tyr Ser Thr Ser
       50                  55                  60
   Leu Lys Thr Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Ser Pro Arg Tyr Arg Gly Ala Phe Asp Tyr Trp Gly Gln
               100                 105                 110
   Gly Thr Leu Val Thr Val Ser Ser
           115                 120
<210> 25
   <211> 109
   <212> PRT
   <213> artificial sequence
<220>
   <221> MS-GPC8-VL
   <222> (1)..(109)
<400> 25
   Asp Ile Val Leu Thr Gln Pro Pro Ser Val Ser Gly Ala Pro Gly Gln
   1 5 10 15
   Arg Val Thr Ile Ser Cys Ser Gly Ser Ser Ser Asn Ile Gly Ser Asn
   20 25 30
   Tyr Val Ser Trp Tyr Gln Gln Leu Pro Gly Thr Ala Pro Lys Leu Leu
   35 40 45
   Ile Tyr Asp Asn Asn Gln Arg Pro Ser Gly Val Pro Asp Arg Phe Ser
   50 55 60
   Gly Ser Lys Ser Gly Thr Ser Ala Ser Leu Ala Ile Thr Gly Leu Gln
   65 70 75 80
   Ser Glu Asp Glu Ala Asp Tyr Tyr Cys Gln Ser Tyr Asp Met Pro Gln
   85 90 95
   Ala Val Phe Gly Gly Gly Thr Lys Leu Thr Val Leu Gly
   100 105
<210> 26
   <211> 120
   <212> PRT
   <213> artificial sequence
<220>
   <221> MS-GPC10-VH
   <222> (1)..(120)
<400> 26
   Gln Val Gln Leu Lys Glu Ser Gly Pro Ala Leu Val Lys Pro Thr Gln
   1 5 10 15
   Thr Leu Thr Leu Thr Cys Thr Phe Ser Gly Phe Ser Leu Ser Thr Ser
   20 25 30
   Gly Val Gly Val Gly Trp Ile Arg Gln Pro Pro Gly Lys Ala Leu Glu
   35 40 45
   Trp Leu Ala Leu Ile Asp Trp Asp Asp Asp Lys Tyr Tyr Ser Thr Ser
   50 55 60
   Leu Lys Thr Arg Leu Thr Ile Ser Lys Asp Thr Ser Lys Asn Gln Val
   65 70 75 80
   Val Leu Thr Met Thr Asn Met Asp Pro Val Asp Thr Ala Thr Tyr Tyr
   85 90 95
   Cys Ala Arg Gln Leu His Tyr Arg Gly Gly Phe Asp Leu Trp Gly Gln
   100 105 110
   Gly Thr Leu Val Thr Val Ser Ser
   115 120
<210> 27
   <211> 109
   <212> PRT
   <213> artificial sequence
<220>
   <221> MS-GPC10-VL
   <222> (1)..(109)
<400> 27
   Asp Ile Val Leu Thr Gln Pro Pro Ser Val Ser Gly Ala Pro Gly Gln
   1 5 10 15
   Arg Val Thr Ile Ser Cys Ser Gly Ser Ser Ser Asn Ile Gly Ser Asn
   20 25 30
   Tyr Val Ser Trp Tyr Gln Gln Leu Pro Gly Thr Ala Pro Lys Leu Leu
   35 40 45
   Ile Tyr Asp Asn Asn Gln Arg Pro Ser Gly Val Pro Asp Arg Phe Ser
   50 55 60
   Gly Ser Lys Ser Gly Thr Ser Ala Ser Leu Ala Ile Thr Gly Leu Gln
   65 70 75 80
   Ser Glu Asp Glu Ala Asp Tyr Tyr Cys Gln Ser Tyr Asp Leu Thr Met
   85 90 95
   Gly Val Phe Gly Gly Gly Thr Lys Leu Thr Val Leu Gly
   100 105
<210> 28
   <211> 109
   <212> PRT
   <213> artificial sequence
<220>
   <221> MS-GPC8-6-VL
   <222> (1)..(109)
<400> 28
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Tyr Val Ser Trp Tyr Gln Gln Leu Pro Gly Thr Ala Pro Lys Leu Leu
           35                  40                  45
   Ile Tyr Asp Asn Asn Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ser Tyr Asp Tyr Asp His
                   85                  90                  95
   Tyr Val Phe Gly Gly Gly Thr Lys Leu Thr Val Leu Gly
               100                 105
<210> 29
   <211> 109
   <212> PRT
   <213> artificial sequence
<220>
   <221> MS-GPC8-17-VL
   <222> (1)..(109)
<400> 29
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Tyr Val Ser Trp Tyr Gln Gln Leu Pro Gly Thr Ala Pro Lys Leu Leu
           35                  40                  45
   Ile Tyr Asp Asn Asn Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ser Tyr Asp Phe Ser Val
                   85                  90                  95
   Tyr Val Phe Gly Gly Gly Thr Lys Leu Thr Val Leu Gly
               100                 105